(19) Europäisches Patentamt / European Patent Office / Office européen des brevets

(11) **EP 4 484 455 A1**

(12) **EUROPEAN PATENT APPLICATION**
published in accordance with Art. 153(4) EPC

(43) Date of publication:
**01.01.2025 Bulletin 2025/01**

(21) Application number: **23780599.9**

(22) Date of filing: **28.03.2023**

(51) International Patent Classification (IPC):
***C08F 12/26*** *(2006.01)* ***C08L 25/18*** *(2006.01)*
***C08J 9/40*** *(2006.01)* ***C08J 5/22*** *(2006.01)*
***B01J 41/05*** *(2017.01)* ***B01J 41/14*** *(2006.01)*
***B01J 47/12*** *(2017.01)* ***C08K 3/01*** *(2018.01)*

(52) Cooperative Patent Classification (CPC):
**B01J 41/05; B01J 41/14; B01J 47/12; C08F 12/26; C08J 5/22; C08J 9/40; C08K 3/01; C08L 25/18;** Y02E 60/50

(86) International application number:
**PCT/JP2023/012655**

(87) International publication number:
**WO 2023/190590 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.03.2022 JP 2022051521**

(71) Applicants:
- **Kake Educational Institution**
  **Okayama-shi, Okayama 700-0005 (JP)**
- **TOKUYAMA CORPORATION**
  **Yamaguchi 745-8648 (JP)**

(72) Inventors:
- **HIGASHIMURA Hideyuki**
  **Okayama-shi, Okayama 700-0005 (JP)**
- **Sheikh Md Chanmiya**
  **Okayama-shi, Okayama 700-0005 (JP)**
- **ISOMURA Takenori**
  **Shunan-shi, Yamaguchi 745-8648 (JP)**
- **UWAMORI Masahiro**
  **Shunan-shi, Yamaguchi 745-8648 (JP)**

(74) Representative: **Canzler & Bergmeier Patentanwälte Partnerschaft mbB**
**Despag-Straße 6**
**85055 Ingolstadt (DE)**

# (54) ANION EXCHANGE RESIN, ANION EXCHANGE MEMBRANE, ANION-EXCHANGE-GROUP-CONTAINING MONOMER, AND QUATERNARY-IMIDAZOLE-GROUP-CONTAINING MONOMER

(57) To provide an anion exchange resin which has an anion exchange group having high alkali resistance and in which a main chain structure is not affected even if the anion exchange group decomposes. The anion exchange resin according to the present invention is characterized by having an imidazolium group as an anion exchange group in a side chain.

FIG.1

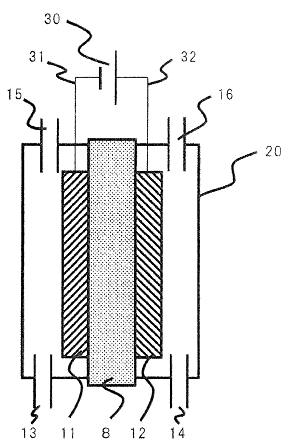


EP 4 484 455 A1

## Description

TECHNICAL FIELD

The present invention relates to an anion exchange resin, and particularly relates to an anion exchange resin preferably used as a material for an anion exchange membrane which is installed to a hydrogen production device using water electrolysis. Also, the present invention relates to an anion exchange membrane including the anion exchange resin. Further, the present invention relates to an anion exchange group-containing monomer and a quaternary imidazole group-containing monomer which are precursors for the production of the anion exchange resin.

BACKGROUND

As an alternative to petroleum energy, the use of hydrogen is seen as a desirable alternative. As a method for producing hydrogen, water electrolysis technologies are being developed. Among the water electrolysis technologies, alkaline water electrolysis has advantages as the reaction field is alkaline, and inexpensive non-precious metal catalysts can be used. Thus, the study of an alkaline water electrolysis using an anion exchange membrane is in progress.

A water electrolysis device using an anion exchange membrane has a structure that the anion exchange membrane is directly bonded with a catalytic electrode (catalytic electrode layer) which is coated with a binder resin made of an anion exchange resin. Thus, a distance between the electrodes is shortened. As a result, ionic resistance between the electrodes is reduced, and it is possible to operate at high current density. Also, another advantage is that pure water can be used as a hydrogen source. Further, hydrogen can be taken out while only supplying water to an anode side and performing water electrolysis at a cathode side, thereby hydrogen with low humidity can be obtained. As a result, a dehydration process can be simplified, and costs of hydrogen storage, transfer, etc., can be reduced.

Reaction in the water electrolysis device is, should it be simplified, a reverse reaction of a reaction taking place in a solid fuel battery. Therefore, in the water electrolysis device, an anion exchange membrane, an anion exchange resin, etc., which are used in the solid fuel battery can be used (for example, see Patent Document 1: WO2017/022775). Further, as an anion exchange group contained in the anion exchange membrane, etc., which are used for these solid fuel battery and water electrolysis device, a group made of a quaternary ammonium salt is known (for example, see Patent Document 1, Patent Document 2: WO2009/081931, and Patent Document 3: WO2011/125717).

An anion exchange membrane containing a quaternary ammonium salt disclosed in the above patent documents has an excellent heat resistance. However, according to the keen study by the present inventors, in the case of using the resin containing the quaternary ammonium salt as the anion exchange group to the water electrolysis device, there are still rooms for improvements in regards with the below mentioned points.

According to the study by the present inventors, when the quaternary ammonium group is held under alkaline environment at relatively high temperature, it may turn into a tertiary ammonium group due to dealkylation, or it may turn into a hydroxyl group in response to substitution reaction. As a result, problems such as a decrease in a remaining ratio of the anion exchange group were found. Regarding the water electrolysis, the system is alkaline, hence in order to perform the water electrolysis for long period of time with higher efficiency, even more enhanced alkali resistance is demanded. Particularly, as for the binder resin forming the catalytic electrode layer, an anion exchange resin which does not deteriorate even after long period of use is demanded.

On the other hand, the ion exchange resin shown in Patent Document 1 as an example contains the quaternary ammonium group in a crosslinking structure, hence it has an enhanced heat resistance. However, Patent Document 1 also discloses an embodiment which uses the quaternary ammonium group not included in the crosslinking structure as an anion exchange group. The quaternary ammonium group which is not included in the crosslinking structure is easily degraded under alkaline environment, and the remaining ratio of the anion exchange group decreases. Thus, it is necessary to enhance the alkali resistance. Further, the method disclosed in Patent Document 1 only shows a method of forming the quaternary ammonium salt containing the crosslinking structure by contacting diamine and the resin which does not contain the anion exchange group. In order to form the crosslinking structure, strict control of reaction steps was needed.

Non-patent Documents 1 to 3 disclose an ion exchange resin containing an imidazolium group in a main chain.

PRIORITY DOCUMENTS

PATENT DOCUMENTS

Patent Document 1: WO2017/022775

Patent Document 2: WO2009/081931
Patent Document 3: WO2011/125717

NON-PATENT DOCUMENT

Non-Patent Document 1: J. American Chemical Society, 2012, 134(26), 10753-10756
Non-Patent Document 2: Energy & Environmental Science, 2016, 9, 2130-2142
Non-Patent Document 3: ACS Macro Letters, 2017, 6(10), 1089-1093

SUMMARY

A quaternary ammonium group has a low remaining ratio under alkaline environment, thus when it is used for a water electrolysis device, the quaternary ammonium group had a problem in its durability. The present inventors have studied for an anion exchange group with high alkali resistance, and have found that an imidazolium group is promising.

However, the imidazolium group also degrades when it is used under harsh condition or by using for long period time. The ion exchange resins disclosed in Non-Patent Documents 1 to 3 contain an imidazolium group in the main chain. Therefore, when the imidazolium group degrades, the main chain itself is cleaved, and the durability of the ion exchange resin declines drastically.

The object of the present invention is to provide the ion exchange resin containing an anion exchange group which has a high alkali resistance and has no influence on the main chain structure even when the anion exchange group is degraded.

The present inventors have carried out keen study to achieve the above-mentioned object. The present inventors have found that an ion exchange resin containing an imidazolium group as an anion exchange group in a side chain can achieve a high alkali resistance, and the durability can be maintained because the main chain structure is not influenced even when the anion exchange group is degraded.

That is, the present invention includes the following described gist.

[1] An anion exchange resin containing a side chain shown in the below formula (1).

[Chemical formula 1]

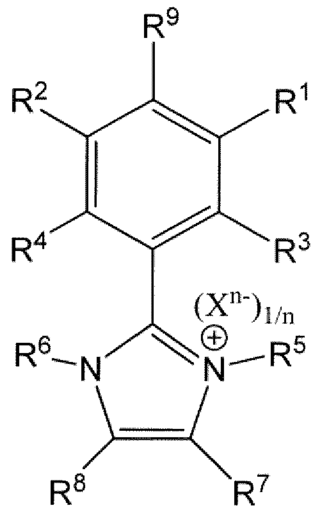

(1)

In the formula (1), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with a main chain forming the anion exchange resin;
$R^5$ and $R^6$ each represents an alkyl group which may contain a substituent group, a cycloalkyl group which may contain a substituent group, an aryl group which may contain a substituent group, or a group which bonds with the main chain forming the anion exchange resin;
at least one of $R^1$, $R^2$, $R^3$, $R^4$, $R^5$, $R^6$, $R^7$, $R^8$, and $R^9$ is a group which bonds with the main chain of the anion

exchange resin; and

$X^{n-}$ represents an anion and n represents an anion valance.

[2] An anion exchange membrane including the anion exchange resin according to the above-mentioned [1].

[3] An anion exchange membrane including a porous membrane as a base material, and voids of the porous membrane are filled with the anion exchange resin according to the above-mentioned [1].

[4] A catalytic electrode forming composition including the anion exchange resin according to the above-mentioned [1] and an electrocatalyst metal.

[5] An anion exchange group-containing monomer shown by a below formula (2)

[Chemical formula 2]

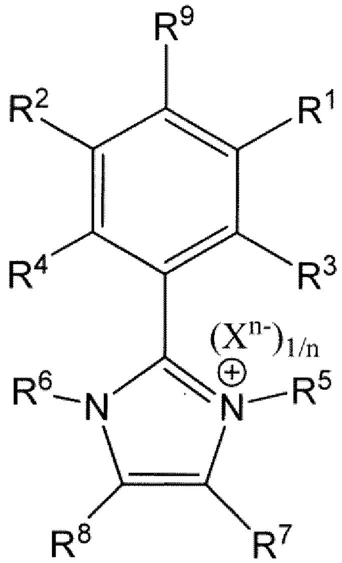

(2)

In the formula (2), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a polymerizable group shown by a below formula (P);

$R^5$ and $R^6$ each represents an alkyl group which may contain a substituent group, a cycloalkyl group which may contain a substituent group, an aryl group which may contain a substituent group, or a polymerizable group shown by a below formula (P);

at least one of $R^1$, $R^2$, $R^3$, $R^4$, $R^5$, $R^6$, $R^7$, $R^8$, and $R^9$ is the polymerizable group shown by the below formula (P); and

$X^{n-}$ represents an anion and n represents an anion valance.

[Chemical formula 3]

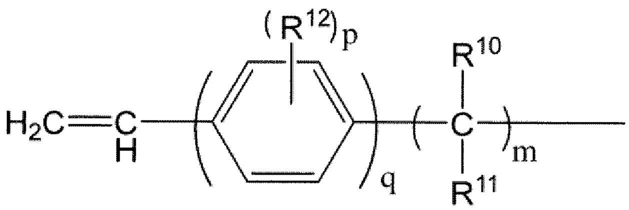

(P)

In the formula (P), $R^{10}$ and $R^{11}$ each represents a hydrogen atom, an alkyl group which may contain a substituent group, a cycloalkyl group which may contain a substituent group, or an aryl group which may contain a substituent group;

m is an integer of 0 to 10;

$R^{12}$ represents a hydrogen atom, an alkyl group which may contain a substituent group, a cycloalkyl group which may contain a substituent group, or an aryl group which may contain a substituent group;

p is an integer of 0 to 4; and
q is an integer of 0 to 10.

[6] A polymerization-curable composition including the anion exchange group-containing monomer according to the above-mentioned [5].
[7] An anion exchange resin obtained by polymerizing the polymerization-curable composition according to the above-mentioned [6].
[8] An anion exchange membrane obtained by coating and polymerizing the polymerization-curable composition according to the above-mentioned [6].
[9] A method for producing an anion exchange membrane, including:

filling at least voids of a porous membrane with the polymerization-curable composition according to the above-mentioned [6] by contacting the porous membrane and the polymerization-curable composition; and
polymerizing the polymerization-curable composition to thereby produce an anion exchange membrane in which an anion exchange resin is filled in the voids.

[10] A monomer containing an imidazole group shown by a below formula (3).

[Chemical formula 4]

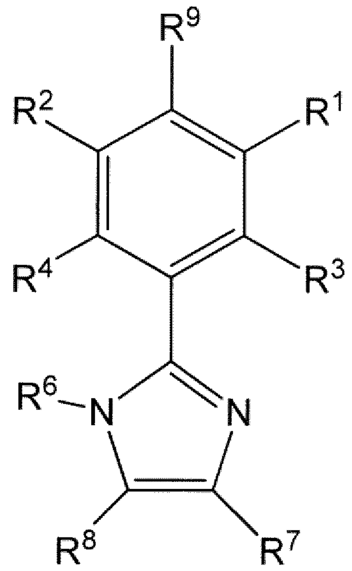

(3)

In the formula (3), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a polymerizable group shown by a below formula (P);
$R^6$ represents an alkyl group which may contain a substituent group, a cycloalkyl group which may contain a substituent group, an aryl group which may contain a substituent group, or a polymerizable group shown by a below formula (P); and
at least one of $R^1$, $R^2$, $R^3$, $R^4$, $R^6$, $R^7$, $R^8$, and $R^9$ is a polymerizable group shown by below formula (P).

[Chemical formula 5]

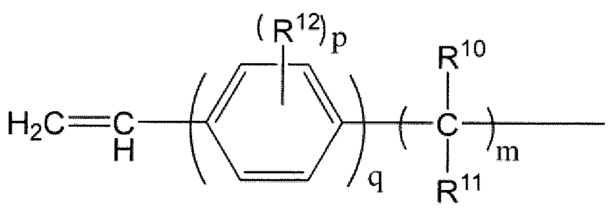

(P)

In the formula (P), $R^{10}$ and $R^{11}$ each represents a hydrogen atom, an alkyl group which may contain a substituent group, a cycloalkyl group which may contain a substituent group, or an aryl group which may contain a substituent group;
m is an integer of 0 to 10;
$R^{12}$ represents a hydrogen atom, an alkyl group which may contain a substituent group, a cycloalkyl group which may contain a substituent group, or an aryl group which may contain a substituent group;
p is an integer of 0 to 4; and
q is an integer of 0 to 10.

[11] A polymerization-curable composition including a monomer containing the imidazole group according to the above-mentioned [10].
[12] A method for producing an anion exchange resin including a side chain shown by a below formula (1'), including:

polymerizing the polymerization-curable composition according to the above-mentioned [11] to obtain a resin containing an imidazole group; and
contacting the obtained resin containing the imidazole group with $R^{5'}$-X1 which is a halogen-containing compound containing at least one selected from an alkyl group which may containing a substituent group, a cycloalkyl group which may contain a substituent group, or an aryl group which may contain a substituent group.

[Chemical formula 6]

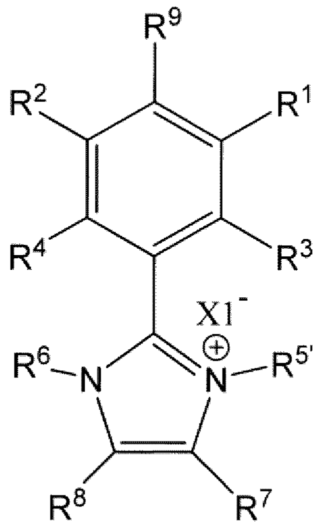

(1')

In the formula (1'), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with a main chain forming the anion exchange resin by polymerizing the polymerizable group shown by the above-formula (P);
$R^{5'}$ represents an alkyl group which may contain a substituent group, a cycloalkyl group which may contain a substituent group, or an aryl group which may contain a substituent group;
$R^6$ represents an alkyl group which may contain a substituent group, a cycloalkyl group which may contain a substituent group, an aryl group which may contain a substituent group, or the group which bonds with the main chain forming the anion exchange resin by polymerizing the polymerizable group shown by the above-formula (P);
at least one of $R^1$, $R^2$, $R^3$, $R^4$, $R^6$, $R^7$, $R^8$, and $R^9$ is the group which bonds with the main chain forming the anion exchange resin by polymerizing the polymerizable group shown by the above-formula (P); and
X1- represents a halogen ion.

[13] A method for producing an anion exchange membrane including a quaternary imidazole group which contains a halogen ion as a counter ion, including:

coating a polymerization-curable composition according to the above-mentioned [11] to form a membrane;

contacting a resin membrane, containing an imidazole group obtained by polymerizing the polymerization-curable composition, and halogenated alkyl which contains an alkyl group.

[14] A method for producing an anion exchange membrane including a quaternary imidazole group which contains a halogen ion as a counter ion, including:

filling at least voids of a porous membrane with the polymerization-curable composition according to the above-mentioned [11] by contacting the porous membrane and the polymerization-curable composition;
polymerizing the polymerization-curable composition to thereby produce an anion exchange membrane precursor in which a resin containing an imidazole group is filled in the voids; and
contacting the obtained anion exchange membrane precursor and halogenated alkyl which contains an alkyl group.

[15] A method for producing an anion exchange resin shown by a below formula (1"), including:

forming the anion exchange resin containing a halogen ion as a counter ion using the method according to the above-mentioned [12]; and
contacting the anion exchange resin and substances containing an anion other than the halogen ion.

[Chemical formula 7]

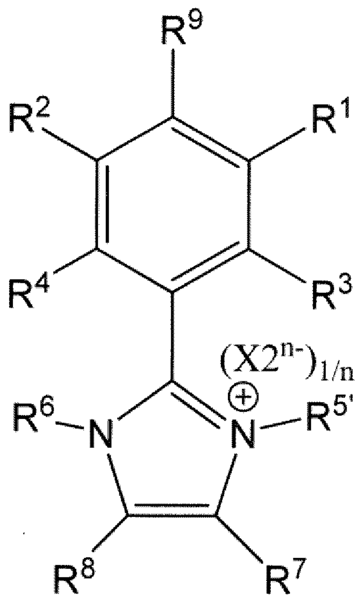

(1")

In the above-formula (1"), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with the main chain forming the anion exchange resin by polymerizing the polymerizable group shown by the above-formula (P);
$R^{5'}$ represents an alkyl group which may contain a substituent group, a cycloalkyl group which may contain a substituent group, or an aryl group which may contain a substituent group;
$R^6$ represents an alkyl group which may contain a substituent group, a cycloalkyl group which may contain a substituent group, an aryl group which may contain a substituent group, or the group which bonds with the main chain forming the anion exchange resin by polymerizing the polymerizable group shown by the above-formula (P);
at least one of $R^1$, $R^2$, $R^3$, $R^4$, $R^6$, $R^7$, $R^8$, and $R^9$ is the group which bonds with the main chain forming the anion exchange resin by polymerizing the polymerizable group shown by the above-formula (P); and
$X2^{n-}$ represents an anion other than a halogen ion and n represents an anion valence.

[16] A method for producing an anion exchange membrane, including:

forming an anion exchange membrane containing a quaternary imidazole group including a halogen ion as a counter ion using the method according to the above-mentioned [13]; and
contacting the obtained anion exchange membrane and a substance containing an anion other than the halogen ion.

[17] A method for producing an anion exchange membrane, including:

forming an anion exchange membrane containing a quaternary imidazole group including a halogen ion as a counter ion using the method according to the above-mentioned [14]; and
contacting the obtained anion exchange membrane and a substance containing an anion other than the halogen ion

EFFECTS OF THE INVENTION

The present invention provides an anion exchange resin which has a high alkali resistance, and has a main chain structure not affected even when an anion exchange group is degraded, thus enables to maintain a durability. Such anion exchange resin has a high durability even when it is used for a water electrolysis device, thus it can contribute in reduction of a hydrogen production cost. Also, the present invention also provides a raw material monomer suitably used for a production of the anion exchange resin.

BRIEF DESCRIPTION OF THE DRAWINGS

FIG.1 is a schematic view showing an example of a structure of a water electrolysis device.
FIG.2 is a preliminary test result of an alkali resistance of an anion exchange group.
FIG.3 is a $^1$H-NMR chart of an imidazole group-containing monomer shown by a formula 3 prepared using a production example 1.
FIG.4 is a $^1$H-NMR chart of an anion exchange group-containing monomer shown by a formula 4 prepared using a production example 2.
FIG.5 is a $^1$H-NMR chart of an imidazole group-containing resin shown by a formula 5 prepared using a production example 3.
FIG.6 is a $^1$H-NMR chart of an imidazole group-containing monomer shown by a formula 6 prepared using a production example 4.
FIG.7 is a $^1$H-NMR chart of a monomer shown by a formula 7 prepared using a production example 5.
FIG.8 is a $^1$H-NMR chart of a monomer shown by a formula 9 prepared using a production example 6.
FIG.9 is a $^1$H-NMR chart of an anion exchange resin shown by a formula 10 prepared using a production example 7.
FIG.10 is a $^1$H-NMR chart of a monomer shown by a formula 12 prepared using a production example 9.
FIG.11 is a $^1$H-NMR chart of a monomer shown by a formula 13 prepared using a production example 10.
FIG.12 is a $^1$H-NMR chart of an imidazole group-containing monomer shown by a formula 17 prepared using a production example 11.
FIG.13 is a $^1$H-NMR chart of a monomer shown by a formula 18 prepared using a production example 12.
FIG.14 is a $^1$H-NMR chart of a resin shown by a formula 19 prepared using a production example 13.
FIG.15 is a $^1$H-NMR chart of a resin shown by a formula 20 prepared using a production example 14.
FIG.16 is a $^1$H-NMR chart of a monomer shown by a formula 23 prepared using a production example 15.
FIG.17 is a $^1$H-NMR chart of a resin shown by a formula 24 prepared using a production example 16.
FIG.18 is a $^1$H-NMR chart of a resin shown by a formula 25 prepared using a production example 17.
FIG.19 is a $^1$H-NMR chart of a monomer mixture shown by a formula 29 and a formula 30 prepared using a production example 18.
FIG.20 is a $^1$H-NMR chart of a resin shown by a formula 31 prepared using a production example 19.
FIG.21 is a $^1$H-NMR chart of a resin shown by a formula 32 prepared using a production example 20.

DETAILED DESCRIPTION

First, main terms used in the present specification are explained.
In the present specification, "a main chain" refers to the longest chain of atoms bonded covalently in a polymer configuring a resin. The main chain is generally made of carbon backbone, however, atoms other than carbon may be included (such as oxygen, nitrogen, sulfur, metals, etc.). Among these, from the point of durability, preferably it is made of

carbon backbone.

A side chain is a chemical group (substituent group) bonded to a macromolecule which is called a main chain. In the present specification, an imidazolium group is included in the side chain.

Hereinbelow, embodiments of the present invention are described. First, an anion exchange resin is explained, and then, a raw material monomer used for the production of the resin is explained, or a method for producing the monomer and the resin is explained.

<Anion Exchange Resin>

The anion exchange resin of the present invention can be used as a separator of a water electrolysis device, or used as an ionic conductivity-imparting agent (binder resin) for forming a catalytic electrode layer. Here, a catalytic electrode layer refers to both an anode generating hydrogen and a cathode generating oxygen; and it is not limited to be used to either one of the electrodes, and the anion exchange resin can be suitably used for the production of the catalytic electrode layers of both anode and cathode.

The anion exchange resin of the present invention contains a side chain shown by a below formula (1).

[Chemical formula 8]

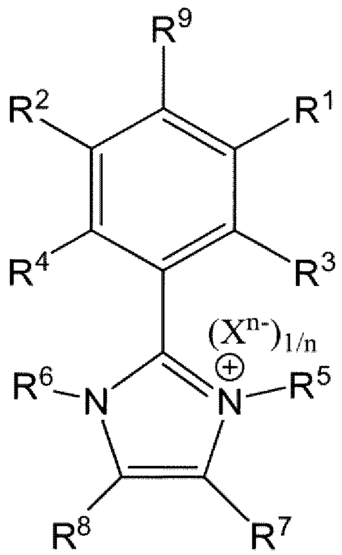

(1)

$R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with the main chain forming the anion exchange resin.

$R^5$ and $R^6$ each represents an alkyl group which may contain a substituent group, a cycloalkyl group which may contain a substituent group, an aryl group which may contain a substituent group, or a group which bonds with the main chain forming the anion exchange resin.

Examples of substituent groups which may be contained in the alkyl group, the cycloalkyl group, and the aryl group include an alkyl group having 1 to 10 carbon atoms, an alkoxy group having 1 to 10 carbon atoms, a cycloalkyl group having 3 to 10 carbon atoms, an aryl group having 6 to 10 carbon atoms, an aralkyl group having 7 to 14 carbon atoms (an aralkyl group which may be substituted with a vinyl group), a haloalkyl group having 1 to 10 carbon atoms (an alkyl group having 1 to 10 carbon atoms which may be substituted with a halogen atom), and a halogen atom. Among these, considering the productivity of the anion exchange resin, the alkyl group having 1 to 10 carbon atoms is preferable, and particularly preferably it is a methyl group, an ethyl group, or a trimethyl group. Note that, obviously, an alkyl group substituted with another alkyl group corresponds to a branched alkyl group.

The alkyl group which may contain a substituent group is preferably an alkyl group having 1 to 20 carbon atoms, and more preferably 3 to 20 carbon atoms in which the number of carbon atoms of the substituent group are excluded. Among these, specifically, a methyl group, an ethyl group, an i-propyl group, an n-propyl group, an i-butyl group, an n-butyl group, an n-hexyl group, an n-heptyl group, an n-octyl group, a nonyl group, or a decanyl group is preferable. A particularly preferable group may be a methyl group, an ethyl group, an i-propyl group, i-butyl group, an n-butyl group, or an n-hexyl group, as these can easily achieve good balance between a hydrophilic property and hydrophobic property, and also has excellent productivity of the anion exchange resin.

The cycloalkyl group which may contain a substituent group is preferably an alkyl group having 3 to 20 carbon atoms, and more preferably 6 to 10 carbon atoms in which the number of carbon atoms of the substituent group is excluded. Among these, a cyclohexyl group, 4-methyl cyclohexyl group, or 4-butyl cyclohexyl group is preferable as these can easily achieve good balance between a hydrophilic property and hydrophobic property, and also has excellent productivity of the anion exchange resin.

The aryl group which may contain a substituent group is preferably an aryl group having 6 to 18 carbon atoms, and more preferably 6 to 14 carbon atoms in which the number of carbon atoms of the substituent group is excluded. Among these, a phenyl group, a naphthyl group, a tolyl group, a xylyl group, 4-alkyl (an alkyl group having 1 to 3 carbon atoms)-phenyl group, or a biphenyl group is preferable as these can easily achieve good balance between a hydrophilic property and hydrophobic property, and also has excellent productivity of the anion exchange resin.

Note that, at least one of $R^1$, $R^2$, $R^3$, $R^4$, $R^5$, $R^6$, $R^7$, $R^8$, and $R^9$ is a group which bonds with the main chain forming the anion exchange resin (hereinafter, this group is referred as "bonding group"). The number of bonding groups is preferably 1 to 2, and particularly preferably it is one.

The bonding group may be a single bond, or it may be a group which is shown by the below formula (P').

[Chemical formula 9]

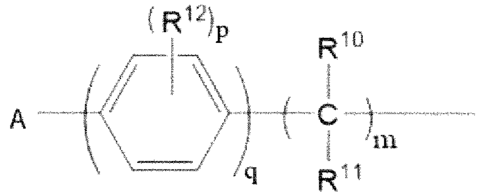

(P')

In the above-formula (P'), "A" is a bonding portion.

$R^{10}$, $R^{11}$, and $R^{12}$ each represents a hydrogen atom, an alkyl group which may contain a substituent group, a cycloalkyl group which may contain a substituent group, or an aryl group which may contain a substituent group. Here, preferable examples of the alkyl group which may contain a substituent group, a cycloalkyl group which may contain a substituent group, and an aryl group which may contain a substituent group are the same as mentioned in above.

In the above-formula (P'), m is an integer of 0 to 10, and preferably m is an integer of 1 to 6.

Also, p is a sum of hydrogens and substituent groups on the benzene ring (that is, p is a number of $R^{12}$), and a total is four. However, conventionally, hydrogen is not counted. That is, when all of $R^{12}$ are hydrogen atoms, p is shown as zero. Therefore, p is an integer of 0 to 4, preferably an integer of 0 to 3, and further preferably it may be an integer of 0 to 2. Particularly preferably, it may be 0 (all of $R^{12}$ are hydrogen). Also, q is an integer of 0 to 10, preferably an integer of 1 to 5, and more preferably an integer of 1 to 3.

In the case that m and q are both 0 (zero), this means that the above-mentioned bonding group is a single bond.

The anion exchange resin of the present invention contains an imidazolium group as shown in the formula (1), and one nitrogen of the imidazolium group is cationized. $X^{n-}$ represents an anion which becomes a counter ion of a cation, and n represents an anion valance. $X^{n-}$ is one or more selected from the group consisting of $OH^-$, $HCO_3^-$, $CO_3^{2-}$, $Cl^-$, Br, and $I^-$; and two or more anions may be included. In the case of using the anion exchange resin in the water electrolysis device, OH-type is generally used, however, $HCO_3^-$, $CO_3^{2-}$, $Cl^-$, $Br^-$, or $I^-$ may be included.

In order to protect cationized nitrogen (quaternary nitrogen) in the imidazolium group, $R^3$, $R^4$, $R^5$, and $R^6$ positioned around the nitrogen are preferably bulky groups. In the case that any one of $R^3$, $R^4$, $R^5$, and $R^6$ is a bonding group, $R^3$, $R^4$, $R^5$, and $R^6$ other than the one which is the bonding group preferably has a total of 1 to 30 carbon atoms, and more preferably has a total of 5 to 20 carbon atoms. For example, when $R^6$ is a bonding group, a total number of carbon atoms of $R^3$, $R^4$, and $R^5$ is within the above-mentioned range. By having the relatively bulky groups around the quaternary nitrogen, dealkylation from the nitrogen and a substitution reaction of the nitrogen are suppressed, hence the durability of the ion exchange resin is enhanced.

Since the anion exchange resin is used in the water electrolysis device, the anion exchange resin is preferably water-insoluble. Also, in the case of forming a membrane, in some cases, the membrane is made by dissolving in an organic solvent, thus preferably the anion exchange resin is soluble in an organic solvent. Examples of organic solvents are listed in below.

Alcohols with 1 to 4 carbon atoms; methanol, ethanol, 1-propanol, 2-propanol, 1-butanol, and 2-butanol
Ether-based solvents; tetrahydrofuran (THF) and 1,4-dioxane Halogen-based solvents; chloroform and dichloromethane

Ketone-based solvents; acetone and methyl ethyl ketone
Halogen-based solvents; toluene and xylene
Other polar solvents besides those mentioned in above; dimethylformamide (DMF), dimethyl sulfoxide (DMSO), etc.

Note that, in the present invention, being soluble in the organic solvent means that 0.5 g of the anion exchange resin (the target resin) dissolves in 100 ml of the organic solvent at 23°C.

The solubility of the anion exchange resin can be adjusted by appropriately selecting $R^1$, $R^2$, $R^3$, $R^4$, $R^5$, $R^6$, $R^7$, $R^8$, $R^9$, $R^{10}$, $R^{11}$, $R^{12}$, m, p, and q. For example, when the bulky hydrocarbon group is included, the anion exchange resin becomes hydrophobic and insoluble in water, and easily dissolves in the organic solvent. Also, as it is described later in detail, when forming the anion exchange resin, by copolymerizing with a monomer other than the hydrophobic anion exchange monomer, a hydrophilic property and a hydrophobic property can be balanced.

From the point of stability of the anion exchange resin, $R^3$, $R^4$, $R^5$, $R^6$, $R^7$, and $R^8$ are preferably other than hydrogen.

Below table shows preferable examples of $R^1$, $R^2$, $R^3$, $R^4$, $R^5$, $R^6$, $R^7$, $R^8$, and $R^9$.

[Table 1-1]

| $R^1$ | $R^2$ | $R^3$ | $R^4$ | $R^5$ | $R^6$ | $R^7$ | $R^8$ | $R^9$ |
|---|---|---|---|---|---|---|---|---|
| H | H | Me | Me | Et | Et | Me | Me | S B |
| H | H | Me | Me | i-Pr | i-Pr | Me | Me | S B |
| H | H | Me | Me | n-Pr | n-Pr | Me | Me | S B |
| H | H | Me | Me | 2,2-$Me_2$Pr | 2,2-$Me_2$Pr | Me | Me | S B |
| H | H | Me | Me | 3-Mebu | 3-Mebu | Me | Me | S B |
| H | H | Me | Me | n-Hex | n-Hex | Me | Me | S B |
| H | H | Me | Me | 3,3-$Me_2$bu | 3,3-$Me_2$bu | Me | Me | S B |
| H | H | Me | Me | CyHex | CyHex | Me | Me | S B |
| H | H | Me | Me | Et | i-Pr | Me | Me | S B |
| H | H | Me | Me | Et | n-Pr | Me | Me | S B |
| H | H | Me | Me | Et | 2,2-$Me_2$Pr | Me | Me | S B |
| H | H | Me | Me | Et | 3-Mebu | Me | Me | S B |
| H | H | Me | Me | Et | n-Hex | Me | Me | S B |
| H | H | Me | Me | Et | 3,3-$Me_2$bu | Me | Me | S B |
| H | H | Me | Me | Et | CyHex | Me | Me | S B |
| H | H | Me | Me | i-Pr | Et | Me | Me | S B |
| H | H | Me | Me | i-Pr | n-Pr | Me | Me | S B |
| H | H | Me | Me | i-Pr | n-Hex | Me | Me | S B |
| H | H | Me | Me | i-Pr | CyHex | Me | Me | S B |
| H | H | Me | Me | n-Pr | Et | Me | Me | S B |
| H | H | Me | Me | n-Pr | i-Pr | Me | Me | S B |
| H | H | Me | Me | n-Pr | n-Hex | Me | Me | S B |
| H | H | Me | Me | n-Pr | CyHex | Me | Me | S B |
| H | H | Me | Me | n-Hex | Et | Me | Me | S B |
| H | H | Me | Me | n-Hex | i-Pr | Me | Me | S B |
| H | H | Me | Me | n-Hex | n-Pr | Me | Me | S B |
| H | H | Me | Me | n-Hex | CyHex | Me | Me | S B |
| H | H | Me | Me | CyHex | Et | Me | Me | S B |
| H | H | Me | Me | CyHex | i-Pr | Me | Me | S B |

(continued)

| $R^1$ | $R^2$ | $R^3$ | $R^4$ | $R^5$ | $R^6$ | $R^7$ | $R^8$ | $R^9$ |
|---|---|---|---|---|---|---|---|---|
| H | H | Me | Me | CyHex | n-Pr | Me | Me | S B |
| H | H | Me | Me | CyHex | n-Hex | Me | Me | S B |
| H | H | Et | Et | Et | Et | Me | Me | S B |
| H | H | i-Pr | i-Pr | Et | Et | Me | Me | S B |
| H | H | CyHex | CyHex | Et | Et | Me | Me | S B |
| H | H | Me | Me | Me | Me | Me | Et | S B |
| H | H | Me | Me | Me | Me | Et | Et | S B |

[Table 1-2]

| $R^1$ | $R^2$ | $R^3$ | $R^4$ | $R^5$ | $R^6$ | $R^7$ | $R^8$ | $R^9$ |
|---|---|---|---|---|---|---|---|---|
| H | H | Me | Me | Et | Me | Et | Et | S B |
| H | H | Me | Me | Et | Et | Et | Et | S B |
| H | H | Me | Me | Me | Me | n-Pr | n-Pr | S B |
| H | H | Me | Me | Et | Me | n-Pr | n-Pr | S B |
| H | H | Me | Me | Et | Et | n-Pr | n-Pr | S B |
| H | H | Me | Me | Me | Me | n-Bu | n-Bu | S B |
| H | H | Me | Me | Et | Me | n-Bu | n-Bu | S B |
| H | H | Me | Me | Et | Et | n-Bu | n-Bu | S B |
| H | H | Me | Me | Me | Me | n-Pen | n-Pen | S B |
| H | H | Me | Me | Et | Me | n-Pen | n-Pen | S B |
| H | H | Me | Me | Et | Et | n-Pen | n-Pen | S B |
| H | H | Me | Me | Me | Me | n-Hex | n-Hex | S B |
| H | H | Me | Me | Et | Me | n-Hex | n-Hex | S B |
| H | H | Me | Me | Et | Et | n-Hex | n-Hex | S B |
| H | H | Me | Me | Me | Me | Me | n-Pr | S B |
| H | H | Me | Me | Et | Me | Me | n-Pr | S B |
| H | H | Me | Me | Et | Et | Me | n-Pr | S B |
| H | H | Me | Me | Me | Me | Me | n-Bu | S B |
| H | H | Me | Me | Et | Me | Me | n-Bu | S B |
| H | H | Me | Me | Et | Et | Me | n-Bu | S B |
| H | H | Me | Me | Me | Me | Me | n-Pen | S B |
| H | H | Me | Me | Et | Me | Me | n-Pen | S B |
| H | H | Me | Me | Et | Et | Me | n-Pen | S B |
| H | H | Me | Me | Me | Me | Me | n-Hex | S B |
| H | H | Me | Me | Et | Me | Me | n-Hex | S B |
| H | H | Me | Me | Et | Et | Me | n-Hex | S B |
| H | H | Me | Me | Me | Me | Me | Me | S B |
| SB | H | Me | Me | Et | Et | Me | Me | H |
| H | SB | Me | Me | Et | Et | Me | Me | H |

(continued)

| $R^1$ | $R^2$ | $R^3$ | $R^4$ | $R^5$ | $R^6$ | $R^7$ | $R^8$ | $R^9$ |
|---|---|---|---|---|---|---|---|---|
| H | H | Me | Me | SB | Me | Me | Me | H |
| H | H | Me | Me | SB | Et | Me | Me | H |
| H | H | Me | Me | SB | n-Pr | Me | Me | H |
| H | H | Me | Me | SB | n-Bu | Me | Me | H |
| H | H | Me | Me | SB | n-Pen | Me | Me | H |
| H | H | Me | Me | SB | n-Hex | Me | Me | H |
| H | H | Me | Me | n-Hex | SB | Me | Me | H |

The abbreviations shown in the above-tables are as described in below.

SB: single bond (bonding portion to the main chain)
Me: methyl
Et: ethyl
i-Pr: i-propyl
n-Pr: n-propyl
n-Bu: n-butyl
n-Pen: n-pentyl
2,2-$Me_2$Pr: 2,2-dimethylpropyl
3,3-$Me_2$bu: 3,3-dimethylbutyl
3-Mebu: 3-methylbutyl
CyHex: cyclohexyl
n-Hex: n-hexyl

Also, preferable examples of $R^{10}$, $R^{11}$, $R^{12}$, m, p, and q configuring the bonding groups are shown in below table.

[Table 2]

| $R^{10}$ | $R^{11}$ | $R^{12}$ | m | p(Note) | q |
|---|---|---|---|---|---|
| - | - | - | 0 | | 0 |
| H | H | H | 1 | 0 | 1 |
| H | H | H | 4 | 0 | 1 |
| Note: p is a sum of hydrogens and substituent groups on an aromatic ring (that is, p is a number of $R^{12}$), and a total is four. However, conventionally, hydrogen is not counted. That is, when all of $R^{12}$ are hydrogen atoms, p is zero. | | | | | |

Other preferable embodiments of the anion exchange resin according to the present invention are shown by below (1A), (1B), and (1C). Note that, anion is not shown. Also, A is a bonding portion to the main chain.

[Chemical formula 10]

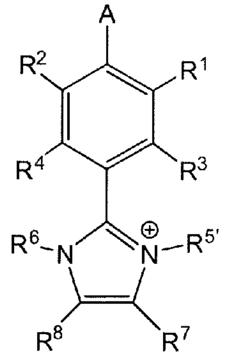

(1A)

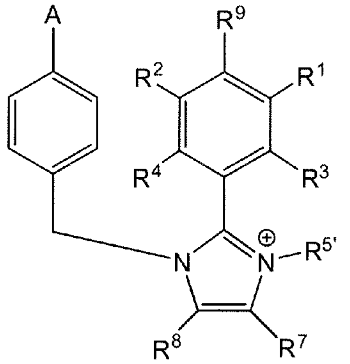

(1B)

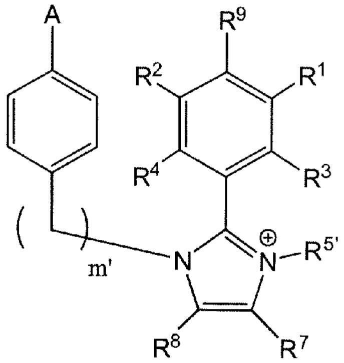

(1C)

In the above formulae (1A) to (1C), each substituent group is as mentioned in above, and particularly preferable substituent groups are as described in below.

A is a bonding portion between the main chain.

$R^1$ and $R^2$ are each preferably a hydrogen atom, or an alkyl group having 1 to 10 carbon atoms, and particularly preferably, it is hydrogen atom.

$R^3$, $R^4$, and $R^5$, and $R^6$ of the formula (1A) are each preferably an alkyl group having 1 to 10 carbon atoms, or a cycloalkyl group having 3 to 10 carbon atoms. Particularly preferably, these are each independently, a methyl group, an ethyl group, an i-propyl group, a n-propyl group, n-hexyl group, or a cyclohexyl group.

$R^7$ and $R^8$ are each preferably an alkyl group having 1 to 10 carbon atoms, or an aryl group having 6 to 14 carbon atoms. Among these, for the formular (1A), $R^7$ and $R^8$ are each preferably an alkyl group having 1 to 3 carbon atoms; and a methyl group, an ethyl group, an i-propyl group, or a n-propyl group is preferable. Also, for the formula (1B) and the formula (1C), $R^7$ and $R^8$ are each preferably an alkyl group having 1 to 6 carbon atoms, or an aryl group having 6 to 10 carbon atoms; and a methyl group, an ethyl group, an i-propyl group, an n-propyl group, an n-pentyl group, an n-hexyl group, or a phenyl group is preferable.

$R^7$ and $R^8$ may be an alkyl group having 1 to 4 carbon atoms. $R^7$ and $R^8$ may have the same structures, or may have different structures. The number of carbon atoms of $R^7$ may be more or less than the number of carbon atoms of $R^8$. Either one of $R^7$ and $R^8$ is an n-propyl group or an n-butyl group, and the other may be a methyl group or an ethyl group.

$R^9$ of the above-formulae (1B) and (1C) is preferably a hydrogen atom or an alkyl group having 1 to 3 carbon atoms, and more preferably it is a hydrogen atom, a methyl group, an ethyl group, an i-propyl group, or n-propyl group.

In the above formula (1C), m' is preferably 2 to 6, and more preferably 3 to 5.

The higher the content ratio of the side chain shown by the formula (1) in the anion exchange resin according to the present invention, the more preferable it is. Therefore, the content ratio is preferably 10 mass% or more, and more preferably 20 mass% or more. As mentioned in above, depending on the number of carbon atoms of the substituent group, the hydrophilic property and the hydrophobic property can be balanced. Therefore, the upper limit of the content ratio is 100 mass%, that is the case which is made only of the side chain shown by the formula (1). The higher the content ratio of the side chain is, the higher the physical properties such as an anion exchange capacity, etc. Thereby, a preferable anion exchange resin is formed.

Note that, the formulae (1A), (1B), and (1C) each have a substituent group with the different number of carbon atoms, hence, the content ratio is preferably as described in below. For example, in the case of the formula (1A), the content ratio is preferably within a range between 10 mass% or more and 98 mass% or less, more preferably within a range between 20 mass% or more and 96 mass% or less, and even more preferably within a range between 30 mass% or more and 94 mass% or less.

Also, in the case of the formula (1B), the content ratio is preferably 40 mass% or more, more preferably 80 mass% or more, and even more preferably 100 mass%.

Also, in the case of the formula (1C), the content ratio is preferably 40 mass% or more, more preferably 80 mass% or more, and even more preferably 100 mass%.

Note that, the content ratio of the side chain refers to a weight ratio of the side chain in the anion exchange resin with respect to the resin which excludes a mass of the main chain portion.

Regarding the anion exchange resin according to the present invention, in order to adjust a reactivity and physical properties of the anion exchange body, if needed, other components may be copolymerized within the range which does not interfere the object of the present invention. Examples of such arbitrary components include aromatic vinyl compounds

such as styrene, α-methyl styrene, vinyl naphthalene, and acenaphtylene.

The content ratio of configurational unit derived from said other components is not particularly limited, and it may be determined based on the balance between the hydrophilic property and the hydrophobic property of the side chain. As mentioned in above, the higher the content ratio of the side chain is, the more preferable it is; and the lower the content ratio of the configurational unit of said other components is, the more preferable it is. For example, when the side chain shown by the formula (1) is introduced, if the hydrophobic property can be maintained, said other components are not needed. On the other hand, in the case that the side chain with high hydrophilic property such as the side chain shown by the formula (1A) is introduced, said other components are preferably used. Therefore, the configurational unit derived from said other components is preferably within a range between 0.1 and 90 mass%, more preferably within a range between 5 and 75 mass%, and even more preferably within a range between 10 and 60 mass%, with respect to a weight of the anion exchange resin.

As mentioned in above, depending on the balance between the hydrophilic property and the hydrophobic property of the side chain, the used amount of said other components differs, thus the used amount is preferably as described in below.

For example, in the case of the formula (1A), the configurational unit derived from said other components is preferably within a range between 0.1 mass% or more and 90 mass% or less, more preferably within a range between 5 mass% or more and 75 mass% or less, and even more preferably 10 mass% or more and 60 mass% or less.

Also, in the case of the formula (1B), the configurational unit derived from said other components is preferably within a range between 0.1 mass% or more and 95 mass% or less, more preferably within a range between 0.2 mass% or more and 80 mass% or less, and even more preferably 0.3 mass% or more and 70 mass% or less.

Also, in the case of the formula (1C), the configurational unit derived from said other components is preferably within a range between 0.1 mass% or more and 98 mass% or less, more preferably within a range between 0.2 mass% or more and 85 mass% or less, and even more preferably within a range between 0.5 mass% or more and 75 mass% or less.

In addition to the side chain shown by the formula (1), the anion exchange resin can include a configurational unit containing a quaternary ammonium group as the anion exchange group.

The structure of the main chain of the anion exchange resin is not particularly limited. However, from the point of easy production, it is a chain of carbon-carbon bonds by a vinyl polymerization. Note that, atoms other than carbon may be included (such as oxygen, nitrogen, sulfur, and metals).

A weight average molecular weight (Mw) of the anion exchange resin is preferably within a range between 5,000 to 5,000,000, and more preferably within a range between 10,000 to 1,000,000. By having Mw within a range between 5,000 to 5,000,000, solubility in water is lowered, and at the same time, solubility in an organic solvent can be maintained. As a result, it becomes easy to be used while being dissolved in the organic solvent. Mw can be measured using a gel permeation chromatography (GPC) method.

Note that, a polymer containing an imidazole group is known to have a possibility of absorbing a gel which has been placed in a column due to the presence of non-covalent electron pairs corresponding to nitrogen atom in the imidazole group; and for the calculation of Mw using a GPC method, there is a chance of error. However, the current technology cannot eliminate the effect of absorbing the non-covalent electron pairs. Thus, in the present invention, a Mw value which is measured by a method using a GPC method described in detail in later section of examples is used.

In the present invention, the ion exchange capacity of the anion exchange resin affects the solubility and ionic conductivity in water and in an organic solvent.

Regarding a resin which the anion exchange capacity is too low, the hydrophobic property tends to become high, and the solubility in the organic solvent tends to increase. On the other hand, the ionic conductivity tends to become significantly low, and in a water electrolysis, an internal resistance tends to increase, and the water electrolysis efficiency tends to be lower.

Also, in the case that the anion exchange capacity is too high, the hydrophilic property tends to become high, and causes to dissolve in water easily. At the same time, the solubility in the organic solvent tends to be lowered. Further, regarding the water electrolysis device, while in use, there is a tendency to be swollen significantly, or to dissolve in water. Thus, there is a possibility that it may be difficult for the electrolysis device to exhibit its ability.

Besides the reasons described in above, the anion exchange capacity affects the alkali resistance of the anion exchange group included in the anion exchange resin. The alkali resistance of the anion exchange group can be easily evaluated from a change rate of anion exchange capacity of before and after the immersion of the anion exchange resin in an alkaline solution. The alkaline solution is a solution that alkali metal hydroxides such as sodium hydroxide, potassium hydroxide, etc., are dissolved in water or a mixed solution of water and an organic solvent. A change rate of the anion exchange capacity is a value shown by a percentage of an anion exchange capacity of after immersion with respect to an anion exchange capacity before the immersion. In the case that the anion exchange capacity has declined after being immersed in the alkaline solution, this means that a degradation reaction against the anion exchange group has proceeded. In general, when the alkali resistance of the resins which contain the anion exchange group having the same structures but with different anion exchange capacities are compared, the resin with lower anion exchange capacity

has a lower change rate of the anion exchange capacity (that is, degradation of the anion exchange group is known). A mechanism of this phenomena is not necessarily clear, and it is speculated as described in below. The lower the anion exchange capacity is, the greater the hydrophobic property is; hence, the number of water molecules in the resin and near the anion exchange group is smaller. As a result, the hydration numbers of hydroxide ions which are counter ions also decline, and a nucleophilic reaction of the hydroxide ions tends to enhance, a degradation reaction such as Hoffman degradation tends to be facilitated. On the other hand, when the resin has a high anion exchange capacity, a hydrophilic property in the resin and of the anion exchange group part is high. Thus, the hydration numbers of hydroxide ions which are counter ions of the anion exchange groups tend to increase, and a nucleophilic reaction tends to decline, thus it is difficult to cause a degradation reaction.

As discussed hereinabove, the anion exchange capacity of the anion exchange resin is preferably within a range between 0.5 to 4.0 mmol/g, and more preferably within a range of 1.2 to 3.5 mmol/g. By selecting such anion exchange capacity, it is possible to obtain the anion exchange resin which is soluble in an organic solvent but not in water, and also which achieves a high alkali resistance.

The anion exchange resin of the present invention exhibits excellent properties, thus by mixing the anion exchange resin and known metals for forming catalytic electrodes, the catalytic electrode forming composition can be formed.

<Anion Exchange Membrane>

The anion exchange membrane of the present invention includes the anion exchange resin. Specifically, the anion exchange membrane of the present invention can be obtained by forming the above-mentioned anion exchange resin into a membrane. As a method for forming the anion exchange resin into a membrane, any known methods can be used. Non-limiting specific examples of the membrane forming method are described in below.

(1) A method which heats the anion exchange resin so that the anion exchange resin exhibits a flexibility, and then applying pressure to flatten, thereby forming a membrane-like object.

(2) A method which pours the liquified anion exchange resin on a flat plane, then evaporating the solvent, thereby forming a membrane-like object.

(3) A method which impregnates the liquified anion exchange resin into a porous membrane, and then evaporating the solvent to fill the voids of the porous membrane with the anion exchange resin, thereby obtaining the membrane-like object.

(4) A method which impregnates monomers as the raw materials of the anion exchange resin into a porous membrane, and then performing polymerization by heating, etc.; thereby obtaining a membrane-like object.

From the point of robustness of the membrane, the porous membrane-supported anion exchange membrane obtained using the method described in (3) or (4) is preferably used. Suitable porous membranes include thermoplastic resin-made porous membranes such as polyolefins including polyethylene, polypropylene, polymethylpentene, etc.; and fluorine-based resin membranes including polytetrafluoroethylene, polytetrafluoroethylene-hexafluoropropylene), polyvinylidene fluoride, etc. Also, a thickness of the anion exchange membrane is preferably within a range between 5 to 200 $\mu$m, and more preferably 8 to 150 $\mu$m, from the point of suppressing a resistance to low level and from the point of giving a necessary mechanical strength as a supporting membrane.

The anion exchange capacity of the anion exchange membrane is, obviously, within the same range as the anion exchange resin. Thus, the anion exchange capacity of the anion exchange membrane is preferably within a range between 0.5 and 4.0 mmol/g, and more preferably within a range between 1.2 and 3.5 mmol/g.

Regarding the preset invention, in the case of the porous membrane-supported anion exchange membrane, it is necessary to fill the voids of the porous membrane with the necessary amount of the ion exchange resin. From the point of increasing the filling amount of the ion exchange resin, it is preferable to use the method (4). In this case, considering the porous membrane-supported anion exchange membrane itself, the anion exchange capacity is preferably within a range between 1.2 to 3.0 mmol/g, and more preferably within a range between 1.5 to 2.8 mmol/g. The anion exchange capacity is an exchange capacity with respect to the entire weight of the anion exchange membrane; and in the case of the porous membrane-supported type, the weight of the porous membrane is calculated in the weight of the anion exchange membrane. Note that, regarding the method (4), there is a method (4-1) in which the porous membrane is filled with the anion exchange group-containing monomer and then polymerizing the monomers. Also, there is a method (4-2) which the porous membrane is filled with the monomer containing the imidazole group, and then performing polymerization to quaternize. These methods will be described later.

<Anion Exchange Group-Containing Monomer>

The anion exchange group-containing monomer used in the method (4-1) is shown by the below formula (2).

[Chemical formula 11]

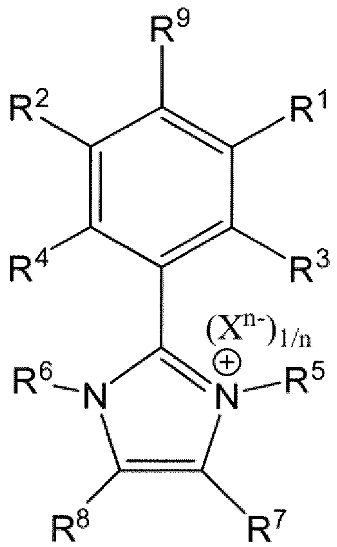

(2)

In the above formula, $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a polymerizable group shown by a below formula (P);

$R^5$ and $R^6$ each represents an alkyl group which may contain a substituent group, a cycloalkyl group which may contain a substituent group, an aryl group which may contain a substituent group, or a polymerizable group shown by a below formula (P);
at least one of $R^1$, $R^2$, $R^3$, $R^4$, $R^5$, $R^6$, $R^7$, $R^8$, and $R^9$ is the polymerizable group shown by the below formula (P); and $X^{n-}$ represents an anion and n represents an anion valance.

[Chemical formula 12]

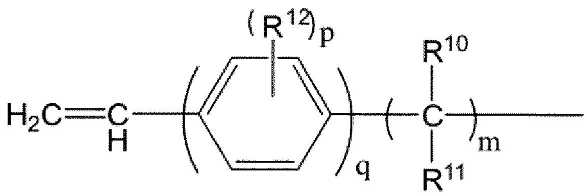

(P)

In the above formula, $R^{10}$ and $R^{11}$ each represents a hydrogen atom, an alkyl group which may contain a substituent group, a cycloalkyl group which may contain a substituent group, or an aryl group which may contain a substituent group;

m is an integer of 0 to 10;
$R^{12}$ represents a hydrogen atom, an alkyl group which may contain a substituent group, a cycloalkyl group which may contain a substituent group, or an aryl group which may contain a substituent group;
p is an integer of 0 to 4; and
q is an integer of 0 to 10.

Preferable embodiments of $R^1$, $R^2$, $R^3$, $R^4$, $R^5$, $R^6$, $R^7$, $R^8$, $R^9$, $R^{10}$, $R^{11}$, $R^{12}$, m, p, and q shown in the above formula are the same as those which are described in regards with the formula (1). Note that, "bonding group (P')" which is described in regarding with the formula (1) is read as the "polymerizable group (P)".

The number of polymerizable groups (P) included in the anion exchange group-containing monomer is preferably within a range between 1 to 3, and particularly preferably one.

A method for producing the anion exchange group-containing monomer is not particularly limited. For example, a compound containing an imidazole structure and a halogen compound containing the polymerizable group (P) are reacted, and then, one of nitrogen in the imidazole structure is quaternized, and thereby the anion exchange group-containing monomer can be obtained.

The compound containing the imidazole structure and the halogen compound containing the polymerizable group (P) may be reacted under the presence of strong base such as sodium hydride (NaH) in a non-aqueous solvent such as tetrahydrofuran, dimethylformamide, etc., at a temperature around room temperature. The reaction time is not particularly limited, and it may be within a range between about 3 and 48 hours. Due to this reaction, hydrogen of the NH group of the imidazole structure and halogen of the halogen compound react with each other and causes to eliminate halogenated hydrogen, and the polymerizable group (P) is introduced to the compound containing the imidazole structure.

Next, the obtained compound is reacted with a quaternizing agent such as ethyl iodide, etc., and the anion exchange group-containing monomer is obtained. This reaction is carried out, for example, in an organic solvent such as acetonitrile, etc., at a temperature around 80°C. The reaction time is not particularly limited, and it may be within a range between about 3 and 96 hours. Due to this reaction, one of nitrogen in the imidazole structure is quaternized, and the anion exchange group-containing monomer is obtained.

Although it is not limited in any way, below shows an example of the reaction.

[Chemical formula 13]

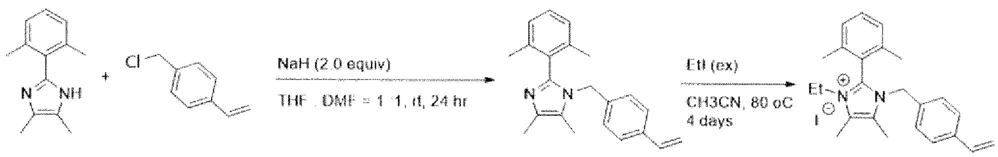


Also, in the case that the polymerizable group is introduced into $R^9$, the below method can be employed. For example, a phenyl compound containing a halogen atom and aldehyde group is reacted with ammonium acetate and 2,3-bitanedion to form an imidazole compound (form an imidazole compound containing a halogen atom and an imidazole group). The quaternizing agent such as ethyl iodide, etc., is reacted with this imidazole compound to form the halogen compound containing the anion exchange group. Then, the halogen atom is replaced with a vinyl group, thereby the anion exchange group-containing monomer can be obtained.

<Production of Anion Exchange Resin and Membrane Using Anion Exchange Group-Containing Monomer>

The anion exchange resin containing the side chain shown by the formula (1) is obtained by polymerizing a polymerization-curable composition (A) which includes the anion exchange group-containing monomer. By polymerizing the anion exchange group-containing monomer, a polymer which includes a structural unit containing the side chain shown by the formula (1) can be obtained. That is, the anion exchange resin can be obtained.

In addition to the anion exchange group-containing monomer, the polymerization-curable composition (A) may include other monomers capable of polymerizing with the polymerization-curable composition (A). Said other monomers may be included within a range in which a content ratio of the side chain shown by the formula (1) in the anion exchange resin after the polymerization is 10 mass% or more.

Note that, the used amount of said other monomers may be determined based on the hydrophilic property and the hydrophobic property of the used anion exchange group-containing monomer. In the case of a homopolymer which is made only of the anion exchange group-containing monomer, the physical properties such as an ion exchange capacity, etc., become the highest. That is, in the case that the polymer obtained by polymerizing the anion exchange group-containing monomer is highly hydrophobic, then said other monomers may not be used. On the other hand, in the case that the polymer obtained by polymerizing the anion exchange group-containing monomer is highly hydrophilic, preferably said other monomers are used to enhance the hydrophobic property. Therefore, depending on the structure of the used anion exchange group-containing monomer (that is, depending on the balance between the hydrophobic property and the hydrophilic property), said other monomers may be used or may not be used. Therefore, as similar to the case of the content ratio of the side chain, preferably the used amount of said other monomers is adjusted depending on the structure of the used anion exchange group-containing monomer. When the total blending amount of the anion exchange group-containing monomer shown by the formula (2) and said other monomers is 100 mass%, the blending amount of said other monomers is preferably as described in below.

For example, in the case of using the anion exchange group-containing monomer to which the side chain shown by the formula (1A) can be inserted, the used amount of said other monomers is preferably within a range between 0.1 mass% or more and 90 mass% or less, more preferably within a range between 5 mass% or more and 75 mass% or less, and even more preferably within a range between 10 mass% or more and 60 mass% or less.

Also, in the case of using the anion exchange group-containing monomer to which the side chain shown by the formula (1B) can be inserted, the used amount of said other monomers is preferably within a range between 0.1 mass% or more and 80 mass% or less, more preferably within a range between 5 mass% or more and 70 mass% or less, and even more preferably within a range between 10 mass% or more and 50 mass% or less.

Also, in the case of using the anion exchange group-containing monomer to which the side chain shown by the formula (1C) can be inserted, the used amount of said other monomers is preferably within a range between 0.1 mass% or more and 70 mass% or less, more preferably within a range between 5 mass% or more and 50 mass% or less, and even more preferably within a range between 10 mass% or more and 40 mass% or less.

Also, the polymerization-curable composition (A) may include a radical polymerization initiator, a solvent, and so on. As the radical polymerization initiator, a widely used initiator such as azo compounds, peroxides, etc., can be used without any particular limitation. Also, other than these, as the radical polymerization initiator, a mixture of ethyl 2-bromoisobutyrate, copper (II) chloride ($CuCl_2$), diethyl tin ($SnEt_2$), and tris[2-(dimethylamino)ethyl]amine (MeeTREN) may be used. As the solvent, the above-mentioned halogen-based solvent, an ether-based solvent, and other polar solvents can be used. Furthermore, known additives such as antioxidants, polymerization inhibitors, etc., may be further blended in the polymerization-curable composition (A).

The polymerization-curable composition (A) is polymerized under inert gas atmosphere such as nitrogen or argon atmosphere, at a temperature range between 40 to 120°C for about 1 to 48 hours.

The polymerization-curable composition (A) may be formed into a membrane and then polymerized. A thickness of the membrane may be set so that the thickness of the obtained anion exchange membrane is about 5 to 200 μm. A method of coating the polymerization-curable composition (A) is not particularly limited, and it may be coated using a spray coat method, a bar coat method, a roll coat method, a gravure printing method, a screen-printing method, etc., on the surface of a releasing paper or so, and then, if needed, drying may be performed.

As mentioned in above, from the point of the robustness of the membrane, the polymerization-curable composition (A) and the porous membrane are contacted to fill at least the voids of the porous membrane with the polymerization-curable composition (A), and then the polymerization-curable composition (A) is polymerized. Thereby, preferably the porous membrane-supported anion exchange membrane is obtained. In order to fill the voids of the porous membrane with the polymerization-curable composition (A) by contacting the porous membrane with the polymerization-curable composition (A), the porous membrane may be impregnated in the polymerization-curable composition (A).

<Imidazole Group-Containing Monomer>

The imidazole group-containing monomer used in the method mentioned in the above (4-2) is shown by the below formula (3).

[Chemical formula 14]

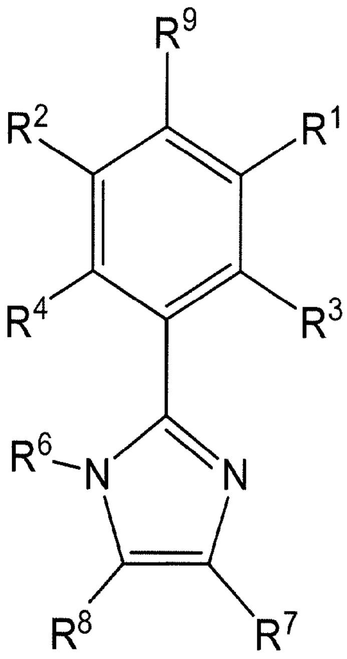

(3)

In the formula (3), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a polymerizable group shown by a below formula (P);

$R^6$ represents an alkyl group which may contain a substituent group, a cycloalkyl group which may contain a substituent group, an aryl group which may contain a substituent group, or a polymerizable group shown by a below formula (P); and
at least one of $R^1$, $R^2$, $R^3$, $R^4$, $R^6$, $R^7$, $R^8$, and $R^9$ is a polymerizable group shown by the below formula (P).

[Chemical formula 15]

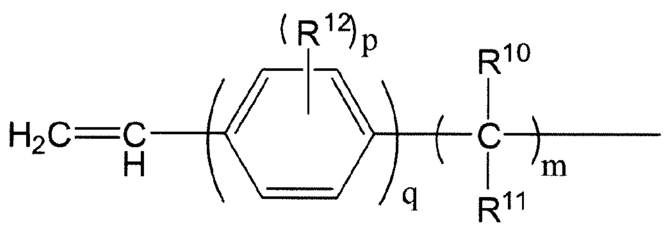

(P)

In the formula (P), $R^{10}$ and $R^{11}$ each represents a hydrogen atom, an alkyl group which may contain a substituent group, a cycloalkyl group which may contain a substituent group, or an aryl group which may contain a substituent group;

m is an integer of 0 to 10;
$R^{12}$ represents a hydrogen atom, an alkyl group which may contain a substituent group, a cycloalkyl group which may contain a substituent group, or an aryl group which may contain a substituent group;
p is an integer of 0 to 4; and
q is an integer of 0 to 10.

Embodiments of preferable $R^1$, $R^2$, $R^3$, $R^4$, $R^6$, $R^7$, $R^8$, $R^9$, $R^{10}$, $R^{11}$, $R^{12}$, m, p, and q shown in above are the same as those which are described in the formula (1) and the formula (2).

The number of polymerizable groups (P) contained in the imidazole group-containing monomer is preferably 1 to

3, and particularly preferably it is 1.

A method of producing the imidazole group-containing monomer is not particularly limited. For example, a compound containing an imidazole structure and a halogen compound containing the polymerizable group (P) may be reacted.

The compound containing the imidazole structure and the halogen compound containing the polymerizable group (P) may be reacted under the presence of strong base such as such as sodium hydride (NaH) in a non-aqueous solvent such as tetrahydrofuran, dimethylformamide, etc., at a temperature around room temperature. The reaction time is not particularly limited, and it may be within a range between about 3 and 48 hours. Due to this reaction, hydrogen of the NH group in the imidazole structure reacts with halogen of the halogen compound and causes to eliminate halogenated hydrogen, thereby the polymerizable group (P) is introduced to the compound containing the imidazole structure.

Although it is not limited in any way, an example of the reaction is shown in below.

[Chemical Formula 16]

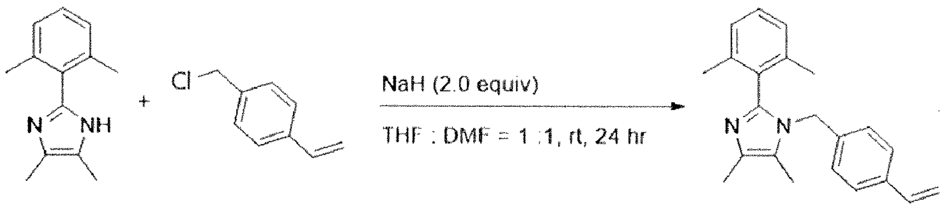


Also, in the case that the polymerizable group is introduced into $R^9$, below method can be employed. For example, a phenyl compound containing a halogen atom and an aldehyde group is reacted with ammonium acetate and 2,3-butanedion to form an imidazole compound (an imidazole compound containing a halogen atom and an imidazole group is formed). By replacing the halogen atom with the vinyl group, the imidazole group-containing monomer can be obtained.

<Production of Anion Exchange Resin and Membrane Using Anion Exchange Group-Containing Monomer>

The anion exchange resin containing the side chain shown by the formula (1) is obtained by polymerizing a polymerization-curable composition (B) which includes the imidazole group-containing monomer shown by the formula (3) to obtain the imidazole group-containing resin, and then quaternizing one of nitrogen of the imidazole structure in the imidazole group-containing resin. By quaternizing the nitrogen of the imidazole group in the polymer, the polymer including the structural unit containing the side chain shown by the formula (1) can be obtained; that is, the anion exchange resin can be obtained.

In addition to the imidazole group-containing monomer shown by the formula (3), the polymerization-curable composition (B) may include other monomers capable of polymerizing with the imidazole group-containing monomer. Note that, said other monomers may be contained within range in which the side chain shown by the formula (1) in the anion exchange resin after the polymerization and quaternization of the polymerization-curable composition (B) is 10 mass% or more. The used amount of said other monomers is the same as in the case of using the anion exchange group-containing monomer, and it may be determined depending on the structure of the side chain containing the anion exchange group formed at the end. That is, the suitable used amount of said other monomers is the same as in the case of using the anion exchange group-containing monomer.

Also, the polymerization-curable composition (B) may include a radical polymerization initiator and a solvent. As the radical polymerization initiator, a widely used initiator such as azo compounds, peroxides, etc., can be used without any particular limitation. Also, other than these, as a radical polymerization initiator, a mixture of ethyl 2-bromoisobutyrate, copper (II) chloride ($CuCl_2$), diethyl tin ($SnEt_2$), and tris[2-(dimethylamino)ethyl]amine (MeeTREN) may be used. As the solvent, toluene, benzene, xylene, etc., can be used. Furthermore, known additives such as antioxidants, polymerization inhibitors, etc., may be blended in the polymerization-curable composition (B).

The polymerization-curable composition (B) is polymerized under inert gas atmosphere such as nitrogen or argon atmosphere, at a temperature within a range between 40 and 130°C for about 1 to 6 hours.

The polymerization-curable composition (B) may be coated and then polymerized. The thickness of the coated polymerization-curable composition may be set so that the thickness of the obtained imidazole group-containing resin membrane is within a range between 5 and 200 μm or so. A method for coating the polymerization-curable composition (B) is not particularly limited, and it may be coated using a spray coat method, a bar coat method, a roll coat method, a gravure printing method, a screen-printing method, etc., on the surface of a releasing paper or so, and then, if needed, drying may

be performed.

As mentioned in above, from the point of the robustness of the membrane, the polymerization-curable composition (B) and the porous membrane are contacted to fill at least the voids of the porous membrane with the polymerization-curable composition (B), and then the polymerization-curable composition (B) is polymerized. Thereby, the porous membrane-supported imidazole group-containing resin membrane is preferably obtained. In order to fill the voids of the porous membrane with the polymerization-curable composition (B) by contacting the polymerization-curable composition (B) with the porous membrane, the porous membrane may be impregnated in the polymerization-curable composition (B).

After obtaining the imidazole group-containing resin membrane as mentioned in above, one of nitrogen of the imidazole group is quaternized, and thereby the anion exchange resin containing the side chain shown by the below formula (1) is obtained. As a quaternizing agent, a halogen-containing compound shown by $R^{5'}$-X1 is used. Here, $R^{5'}$ is an alkyl group which may contain a substituent group, a cycloalkyl group which may contain a substituent group, or an aryl group which may contain a substituent group; and the preferable embodiment is the same as $R^5$ which is explained in the formula (1) and the formula (2). Also, X1 represents halogen, and preferably, it is one or more selected from the group consisting of Cl, Br, and I; and a compound containing two or more halogens may be used. In the case that the imidazole-group containing resin is formed into a membrane, and then reacted with the quaternizing agent, preferably a halogen-containing compound which includes an alkyl group is used as the quaternizing agent, that is, halogenated alkyl is preferably used. The halogenated alkyl is $R^{5'}$-X1 in which $R^{5'}$ is an alkyl group.

The reaction between the imidazole group-containing resin and the quaternizing agent is, for example, performed in an organic solvent such as acetonitrile at a temperature about 80°C. Here, if necessary, reflux may be done. The reaction time is not particularly limited, and it may be about 3 to 96 hours. Due to the reaction between the imidazole group-containing resin and the quaternizing agent, the anion exchange resin containing the side chain shown by the below formula (1') can be obtained.

[Chemical formula 17]

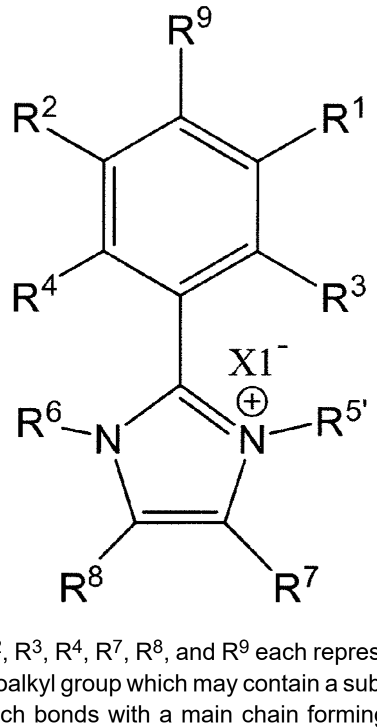

(1')

In the formula (1'), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with a main chain forming the anion exchange resin by polymerizing the polymerizable group shown by the above-formula (P);

$R^{5'}$ represents an alkyl group which may contain a substituent group, a cycloalkyl group which may contain a substituent group, or an aryl group which may contain a substituent group; and

$R^6$ represents an alkyl group which may contain a substituent group, a cycloalkyl group which may contain a substituent group, an aryl group which may contain a substituent group, or the group which bonds with the main chain forming the anion exchange resin by polymerizing the polymerizable group shown by the above formula (P).

Note that, at least one of $R^1$, $R^2$, $R^3$, $R^4$, $R^6$, $R^7$, $R^8$, and $R^9$ is the group which bonds with the main chain forming the anion exchange resin by polymerizing the polymerizable group shown by the above formula (P); and
X1- represents a halogen ion.

Preferable embodiments of $R^1$, $R^2$, $R^3$, $R^4$, $R^6$, $R^7$, $R^8$, $R^9$, $R^{10}$, $R^{11}$, $R^{12}$, m, p, and q shown in the above formula are the same as those described in regards with the formula (1). Also, a preferable embodiment of $R^{5'}$ is the same as $R^5$ which is described in above regarding the formula (1) (note that, $R^{5'}$ is not a polymerizable group). Also, in the case of forming the imidazole group-containing resin into a membrane and then quarternizing, $R^{5'}$ is an alkyl group.

By going through the above-mentioned steps, nitrogen in the imidazole group-containing resin is quaternized and forms imidazolium cation, and thereby an anion exchange resin containing a halogen ion as a counter ion is obtained. This anion exchange resin itself can be used for a water electrolysis device. Preferably, a halogen ion is contacted with a substance containing anion ($X2^{n-}$) which is other than the halogen ion in order to exchange the halogen ion to the anion ($X2^{n-}$) other than the halogen ion, and then it is preferably used for a water electrolysis device or so. Such anion exchange resin is shown by the below formula (1"). As the substance containing anion ($X2^{n-}$) other than the halogen ion, for example, OH-type anion exchange resin or so may be mentioned.

[Chemical formula 18]

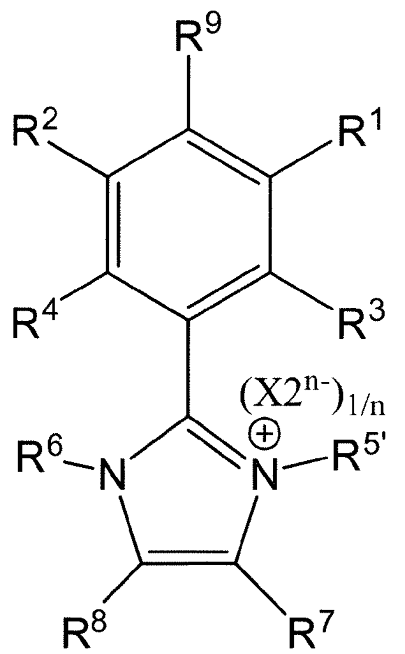

(1")

In the above-formula (1"), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with the main chain forming the anion exchange resin by polymerizing the polymerizable group shown by the above formula (P);

$R^{5'}$ represents an alkyl group which may contain a substituent group, a cycloalkyl group which may contain a substituent group, or an aryl group which may contain a substituent group; and
$R^6$ represents an alkyl group which may contain a substituent group, a cycloalkyl group which may contain a substituent group, an aryl group which may contain a substituent group, or the group which bonds with the main chain forming the anion exchange resin by polymerizing the polymerizable group shown by the above formula (P).

Note that, at least one of $R^1$, $R^2$, $R^3$, $R^4$, $R^6$, $R^7$, $R^8$, and $R^9$ is the group which bonds with the main chain forming the anion exchange resin by polymerizing the polymerizable group shown by the above formula (P); and
$X2^{n-}$ represents anion other than a halogen ion and n represents an anion valence. Here, the anion other than the halogen ion is one or more selected from the group consisting of $OH^-$, $HCO_3^-$, and $CO_3^{2-}$; and two or more anions may be included. In the case of using the anion exchange resin in a water electrolysis device, generally it is used as OH-type, however, $HCO_3^-$ and $CO_3^{2-}$ may be included.

Preferable embodiments of $R^1$, $R^2$, $R^3$, $R^4$, $R^6$, $R^7$, $R^8$, $R^9$, $R^{10}$, $R^{11}$, $R^{12}$, m, p, and q shown in above are the same

as those described in regards with the formula (1). Also, a preferable embodiment of $R^{5'}$ is the same as $R^5$ which is described in above regarding the formula (1) (note that, it is not a polymerizable group). Also, in the case of forming the imidazole group-containing resin into a membrane and then reacting with the quaternizing agent, $R^{5'}$ is an alkyl group.

By going through the above-mentioned steps, nitrogen in the imidazole group-containing resin is quaternized and forms imidazolium cation, and thereby an anion exchange resin containing anion ($X2^{n-}$) which is other than a halogen ion as the counter ion of the imidazolium ion is obtained. This anion exchange resin is suitably used for a water electrolysis device, etc.

<Other Production Methods>

A method for producing the anion exchange resin containing the side chain shown by the above formula (1) is not particularly limited. For example, as shown by the below reaction, an imidazole compound may be reacted with a boromobutylstyrene polymer, and thereby nitrogen in imidazole may be quaternized.

[Chemical formula 19]

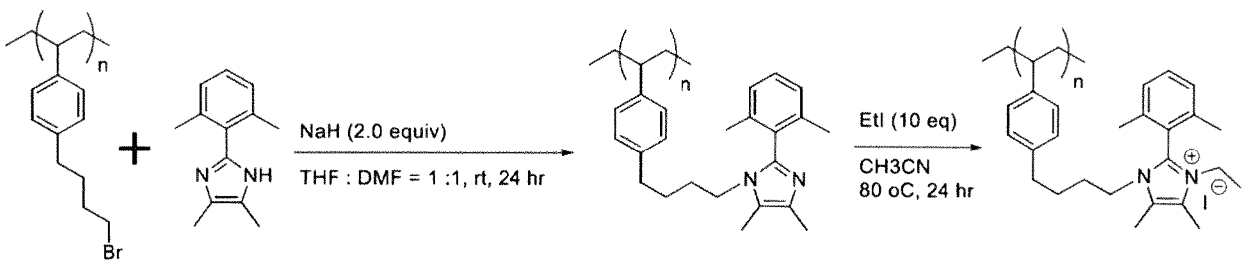


<Water Electrolysis Device>

The anion exchange resin described in above can be used as a separator used in the water electrolysis device, and also can be used as an ionic conductivity-imparting agent (binder resin) for forming a catalytic electrode. In below, a simplified configurational example of the water electrolysis device is described.

The simplified configurational example of the water electrolysis device is shown in FIG.1, however, a use of the anion exchange resin of the present invention is not limited to the structure shown in FIG.1. FIG.1 shows an example of a water electrolysis device 20 using an anion exchange membrane 8. As the anion exchange membrane 8, the anion exchange membrane of the present invention mentioned in above is used. To one surface of the anion exchange membrane 8, a cathode catalytic electrode layer 11 is provided, and to the other surface, an anode catalytic electrode layer 12 is provided. To the front surface of each of the catalytic electrode layers, a gas diffusion layer may be provided, however, it is not shown in the figure. The cathode catalytic electrode layer 11 and the anode catalytic electrode layer 12 may include the anion exchange resin of the present invention as the ionic conductivity-imparting agent (binder).

Any known catalysts can be used as the catalyst of the catalytic electrode layer. In the water electrolysis device using the anion exchange membrane of the present invention, a reaction field is alkaline, thus nickel can be used for the cathode side, and iron, cobalt, nickel, etc., which are inexpensive metals can be used for the anode side.

Note that, a particle size of the metal particle which becomes these catalysts is usually within a range of 0.1 to 100 nm, and more preferably, it is within a range of 0.5 to 10 nm. The smaller the particle size is, the higher the catalyst performance. However, when the particle size is smaller than 0.5 nm, it is difficult to produce, and when the particle size is larger than 100 nm, then it is difficult to achieve sufficient catalyst performance. Also, these catalysts may be supported on a conductive agent in advance, and then it is used. The conductive agent is not particularly limited as long as it is an electroconductive substance, and for example, carbon blacks such as furnace black or acetylene black, activated carbon, graphite, etc., are generally used either alone or in combination. A content of these catalysts is usually within a range between 0.01 to 10 $mg/cm^2$, and more preferably it is within a range between 0.1 to 5.0 $mg/cm^2$ in terms of a metal mass per unit area while the catalytic electrode layer is formed into a sheet form. The catalytic electrode layer may be those using a known binder resin.

Examples of the gas diffusion layer include metal foams such as nickel foam, titanium foam; carbon paper; carbon cross; and porous graphite, etc; and these can be used without particular limitation.

Each catalytic electrode layer is connected to an external power source 30 via conductive wires 31 and 32. Raw water supplying tubes 13 and 14 which supply raw water is provided to the electrolytic cell. As such raw water, water (pure water) may be used, or a diluted alkali aqueous solution may be used. Electrolysis of water begins by supplying raw water

and supplying electricity from the external power source. Reactions of cathode side and anode side are shown in below.

(Cathode side): $2H_2O + 2e^- \rightarrow H_2 + 2OH^-$

(Anode side): $2OH^- \rightarrow 1/2O_2 + H_2O + 2e^-$

At the cathode side, hydrogen and hydroxide ion are generated due to electrolysis of water. The hydroxide ion reaches to the anode side through anion exchange membrane 8; then oxygen, water, and electrons are generated. Hydrogen generated at the cathode is collected through a gas collection tube 15, and oxygen generated at the anode side is collected through a gas collection tube 16.

Regarding the water electrolysis device using the anion exchange resin of the present invention, the anion exchange membrane has high alkali resistance and does not affect the main chain structure even when the anion exchange group is degraded, and can maintain the durability. Thus, the water electrolysis can operate stably for long period of time which contributes to lower the cost of hydrogen production.

EXAMPLES

Hereinbelow, the present invention is discussed in detail using examples, however, the present invention is not limited to these examples.

(Preliminary Test)

In order to evaluate a stability of quaternary nitrogen, the stabilities of three types of compounds shown in FIG.2 were evaluated. Note that, in the figure, anions are not shown, however, halogen ions (such as $I^-$, $Br^-$, $Cl^-$), $OH^-$, $HCO_3^-$, $CO_3^{2-}$, etc., were included as anions.

Each compound shown in FIG.2 was introduced in 1N sodium hydroxide solution of deuterated methanol, so that the concentration was 1 mass%, and then the solution temperature was maintained at 80°C. Immediately after the introduction, samples were taken after every predetermined time period, and sodium 3-(trimethylsilyl)-1- propane sulfonate ($TMSCH_2CH_2CH_2SO_3Na$) was added as an internal standard, and NMR measurement was performed to obtain a remaining ratio of quaternary nitrogen. Results are shown in FIG.2. As shown in FIG.2, it was confirmed that quaternary nitrogen in an imidazolium backbone had an excellent alkali resistance. Therefore, longer lifetime can be expected even under alkaline environment for an anion exchange resin containing such structure.

Properties of the anion exchange resins shown by the examples and the comparative examples exhibited values which were measured using the below described methods.

(1) Measurement of Weight Average Molecular Weight (Mw)

A weight average molecular weight (Mw) of the produced anion exchange resin was obtained using a gel permeation chromatography (GPC) method under the below shown conditions by using HLC-8220-GPC made by TOSOH CORPORATION.

Colum Temperature: 40°C
Column: TSK gel SuperMultipore (2 columns)
Eluent: Tetrahydrofuran
Detector: RI (Refractive Index Detector)

Measurement was performed under the above-described configurations. The weight average molecular weight was obtained by converting from a calibrated curve formed using polystyrene standard (TSK standard POLYSTYRENE made by TOSOH CORPORATION).

Further, Mw was also obtained using GPC in which the eluent was DMF (hereinafter, this may be referred to as GPC(DMF)) under the below-described conditions by using JASCO ChromNAV.

Colum Temperature: 40°C
Column: Shodex K-807L (2 columns)
Eluent: 10 mM LiBr/DMF
Detector: UV (272 nm)

Measurement was performed under the above-described configurations. The weight average molecular weight

was obtained by converting from a calibrated curve formed using polystyrene standard (AMR EasiCal PS-1).

(2) Anion Exchange Capacity of Anion Exchange Resin

The solution in which the anion exchange resin was dissolved (an anion exchange resin concentration of 3.0 mass%, a solution amount of 2.5 g) was coated on a petri dish of Teflon® to produce a cast film. The cast film was thoroughly washed in advance using an ion exchange water. The washed cast film and the ion exchange water were together placed in a visking tube (purchased from AS ONE CORPORATION) vacuum dried for 3 hours at 50°C to measure the weight thereof (Dv (g)); and then both ends of the visking tube were tied. This tube was immersed in 0.5 mol/L-HCL solution (50 mL) for 30 minutes or longer, and this procedure was repeated for three times. Thereby, a counter ion of quaternary nitrogen contained in the cast film was formed into chloride ion type. Next, the tube was immersed in the ion exchange water (50 mL) for washing. This washing was repeated for 10 times. Next, the washed tube was immersed in 0.2 mol/L-NaNOs solution (50 mL) for 30 minutes or longer to convert into a nitrate ion type, thereby the released chloride ions were extracted. This extraction process was repeated for 4 times. Further, the tube after the extraction process was immersed in the ion exchange water (50 mL) for 30 minutes or longer to collect the remaining chloride ions. This collection process was repeated for twice. The chloride ions obtained through the extraction process and through the collection process were all collected for quantification by a potentiometric titrator (COMTITE-900 made by HIRANUMA Co.,Ltd) using silver nitrate solution (the quantified value is referred to as A (mol)).

Next, the tube after the collection process was immersed in 0.5 mol/L-NaCl solution (50 g) for 30 minutes or longer and then the tube was thoroughly washed using the ion exchange water. Then, the tube was taken out, and left in a drier of 50°C for 15 hours to remove water content in the tube, and then the tube was vacuum dried for 3 hours at 50°C to measure the weight (Dt (g)). Based on the measured value, the ion exchange capacity was obtained using the below formula.

$$\text{Anion exchange capacity} = A \times 1000/(Dt - Dv)\ [\text{mmol/g – Dried weight}]$$

(3) Solubility In Water at 20°C

A cast film made of the anion exchange resin having a thickness of about 40 to 60 μm, which was produced by the same method as mentioned in above, was vacuum dried for 3 hours at 50°C, and then a weight of the cast film (Ddry 1 (g)) was measured. Next, this cast film was immersed in water of 20°C and left for 15 hours. Then, the cast film was taken out from the water, and then vacuum dried again for 3 hours at 50°C to again measure a weight of the cast film (Ddry 2 (g)). Based on the measured value, the solubility in water at 20°C was obtained.

$$\text{Solubility in water} = ((\text{Ddry 1} - \text{Ddry 2})/\text{Ddry 1}) \times 100\ [\%]$$

(4) Alkali Resistance Measurement of Anion Exchange Resin

In order to evaluate the alkali resistance of quaternary nitrogen in the anion exchange resin, the change rate of the anion exchange capacity was evaluated. The cast film made of the anion exchange resin having a thickness of about 40 to 60 μm, which was produced by the same method as mentioned in above, was placed in 1N sodium hydroxide solution, and maintained a solution temperature at 80°C. The anion exchange capacity of before the immersion and after 240 hours of immersion were measured using the method (2), and the change rate of the anion exchange capacity was obtained using the below formula.

Change rate of anion exchange capacity = (anion exchange capacity after 240 hours immersion)/(anion exchange capacity before immersion) × 100[%]

(5) Anion Exchange Capacity Measurement of Anion Exchange Membrane

An anion exchange membrane (a membrane of which the anion exchange resin is supported by a porous membrane) was immersed in 0.5 mol/l-HCl solution for 10 hours or longer to convert counter ions of the anion exchange groups in the anion exchange membrane to chloride ions. The counter ions were changed to nitrate ions from chloride ions in 0.2 mol/l-$NaNO_3$ solution, and the released chloride ions were quantified (A (mol)) using a potentiometric titrator (COMTITE-900 made by HIRANUMA Co.,Ltd) using silver nitrate solution.

Next, the same anion exchange membrane was immersed in 1 mol/l-HCl solution for 4 hours or longer, and thorough washing was performed using the ion exchange water, then vacuum drying was performed for 5 hours at 60°C to

measure the dried weight (Dg). Based on the obtained value, the anion exchange capacity of the anion exchange membrane was obtained using the following equation.

$$\text{Anion exchange capacity} = A \times 1000/D \text{ [mmol/g – dried weight]}$$

(6) Alkali Resistance Measurement of Anion Exchange Membrane

A change rate of the anion exchange capacity as an indicator showing alkali resistance of an anion exchange membrane was measured using the same method used for measuring the alkali resistance of the anion exchange resin in the above-described (4), except that a membrane which the anion exchange resin supported by a porous membrane was used instead of the cast film of the anion exchange resin.

Change rate of anion exchange capacity = (anion exchange capacity after 240 hours of immersion)/(anion exchange capacity before immersion) $\times$ 100[%]

(7) Resistance of Anion Exchange Membrane

The ion exchange membrane was placed between two cells containing platinum black electrodes, and both sides of the anion exchange membrane were filled with 0.5 mol/l-sodium chloride solution, and resistance at 25°C between electrodes was measured using AC bridge (frequency of 1000 cycles/sec). Then, the resistance was obtained from the difference in a resistance of said electrodes and a resistance of electrodes without placing the ion exchange membrane.

(8) Evaluation of Water Electrolysis Performance

(8-1) Forming of Water Electrolysis Catalytic Electrode Layer

4.1 g of the anion exchange resin solution (a concentration:3.0 mass%, a counter ion being a bicarbonate ion), 0.5 g of platinum-carrying carbon (a fuel cell catalyst TEC10E50E made by Tanaka Kikinzoku Kogyo), 0.35 g of the ion exchange water, and 0.5 g of 1-propanol were mixed and stirred until a uniform paste was formed, thereby a catalytic layer forming composition was prepared.

This catalytic layer forming composition was screen printed uniformly so that an adhered amount of platinum on the anion exchange resin membrane was 3 mg/cm$^2$. Next, it was dried for 15 hours at room temperature, and then a cathode catalytic layer was formed on the anion exchange membrane. An area of the catalytic layer was 25 cm$^2$.

Also, 1.8 g of the anion exchange resin solution (a concentration of 3.0 mass%, a counter ion being a bicarbonate ion), 2.0 g of iridium oxide catalyst (made by Aldrich), and 2.0 g of 1-propanol were mixed and stirred until a uniform paste was formed, thereby a catalytic layer forming composition was prepared. This catalytic layer forming composition was screen printed uniformly so that 5 mg/cm$^2$ of iridium oxide was adhered on the anion exchange resin membrane having the cathode catalytic layer. Next, it was dried for 15 hours at room temperature, and thereby an anode catalytic layer was formed on the anion exchange membrane. An area of the catalytic layer was 25 cm$^2$.

(8-2) Measurement of Water Electrolysis Performance

By using the anion exchange membrane (the membrane that the cathode catalytic layer and the anode catalytic layer were stacked on the anion exchange membrane) to which the water electrolysis electrodes were formed using the above-mentioned method, a water electrolysis performance was evaluated.

The anion exchange membrane, to which the water electrolysis electrodes were formed, was placed using different gas diffusion layers to each of the cathode electrode side and the anode electrode side, and then assembled into the water electrolysis cell (made by EIWA Corporation) which was compliant with JARI (Japan Automobile Research Institute standard cell. As the gas diffusion layer, a carbon paper (TGP-H-060 made by TORAY INDUSTRIES, INC) was used for the cathode electrode side and a nickel foam (thickness of 200 $\mu$m) was used for the anode electrode side. As a separator of the water electrolysis cell, a carbon block having a serpentine type flow passage was used for the cathode electrode side, and a titanium block having the same flow passage as the cathode electrode side was used for the anode electrode side.

A temperature of the water electrolysis cell was set to 40°C, and 0.5 mol/L-KOH solution heated to 40°C was supplied at a rate of 100 ml/min to the anode cell. Using an electrochemical measuring device (HZ-PROM4 made by MEIDENSHA CORPORATION), voltage was applied to the water electrolysis cell so to achieve desired current density. The current density was increased from zero current to 1,000 mA/cm$^2$ in intervals of 20 mA/cm$^2$, maintaining each current

density value for 5 minutes before increasing it. Then, cell voltages at zero current, 200 mA/cm$^2$, 500 mA/cm$^2$, and 1,000 mA/cm$^2$ were recorded.

(9) Alkali Resistance of Water Electrolysis Cell

The influence on the water electrolysis cell caused by contacting the high temperature alkali solution where water electrolysis reaction was not conducted was evaluated. After measuring the water electrolysis performance curve in (8-2), the water electrolysis cell was removed, and the flow passages of the anode electrode side and cathode electrode side were filled with 1 mol/L-KOH solution. Then, it was maintained for 48 hours in a drier of 80°C. By using the method described in (8-2), the water electrolysis performance was evaluated using the water electrolysis cell taken out of the direr. A simplified evaluation was carried out to evaluate an alkali resistance of the cell based on an increase rate shown by the below equation of the cell voltage at 500 mA/cm$^2$ of before and after drying.

(Increase Rate of Cell Voltage) = (Water electrolysis cell voltage at 500 mA/cm$^2$ after maintained at 80°C)/(Water electrolysis cell voltage at 500 mA/cm$^2$ before maintained at 80°C) $\times$ 100

(Production Example 1)

An imidazole compound shown by the below formula 1 and bromobutylstyrene (1.5 mmol) shown by the below formula 2 were reacted in an organic solvent (THF:DMF = 1:1) using NaH (2.0 equiv) for 24 hours at room temperature to obtain an imidazole group-containing monomer shown by the below formula 3. In FIG.3, $^1$H-NMR of the obtained imidazole group-containing monomer is shown.

[Chemical formula 20]

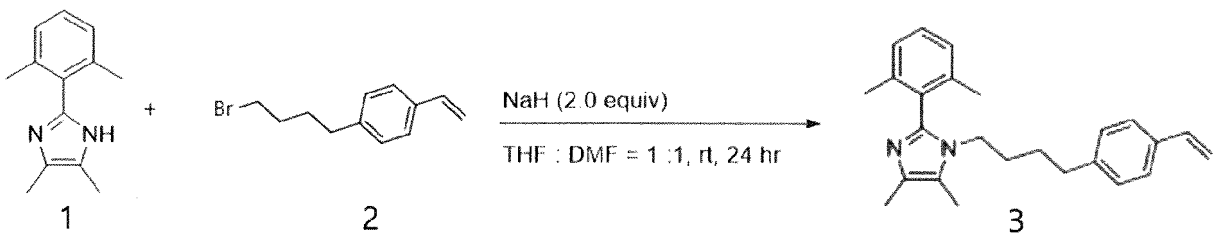


1 2 3

(Production Example 2)

The imidazole group-containing monomer shown by the below formula 3 was reacted with ethyl iodide in acetonitrile at 80°C for four days to quaternize nitrogen in the imidazole backbone to obtain an anion exchange group-containing monomer shown by the below formula 4. In FIG.4, $^1$H-NMR of the obtained anion exchange group-containing monomer is shown.

[Chemical formula 21]

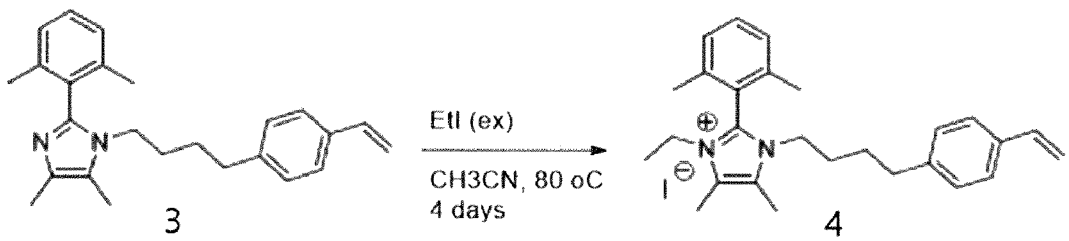


3 4

(Production Example 3)

The imidazole group-containing monomer shown by the above formula 3 was polymerized to produce the anion

exchange resin. Specifically, using a mixture of 2-bromoethyl isobutyrate (0.05 eq), copper (II) chloride ($CuCl_2$) (0.05 eq), diethyltin ($SnEt_2$) (0.025 eq), and tris[2-(dimethylamino)ethyl]amine ($Me_6TREN$) (0.1 eq) as a radical polymerization initiator, a polymerizable composition including the imidazole group-containing monomer (2.5 g) shown by the above formula 3 was polymerized under nitrogen atmosphere for 3 hours at 80°C. The imidazole group-containing resin (the below formula 5) was obtained. In FIG.5, $^1$H-NMR of the obtained imidazole group-containing resin is shown. The molecular weight by GPC measurement was Mw = 78,000.

[Chemical formula 22]

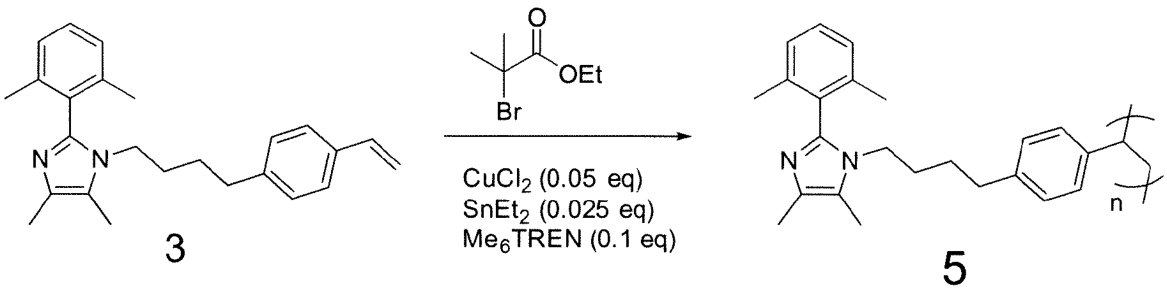


3

5

(Production Example 4)

While refluxing at 80°C in acetonitrile, the imidazole group-containing resin shown by the above formula 5 was reacted with 1-bromohexane for 24 hours to quaternize nitrogen in the imidazole backbone, and thereby the anion exchange resin shown by the formula 6 was obtained. In FIG.6, $^1$H-NMR of the obtained anion exchange resin is shown. Also, the anion exchange resin shown by the below formula 6 was capable of dissolving in 1-propanol, thus it was possible to prepare 3 mass% solution by stirring for 2 days at 25°C. Physical properties of the anion exchange resin shown by the below formula 6 were as shown in Table 3. Also, the molecular weight of the imidazole group-containing resin shown by the formula 6 by GPC measurement was Mw = 85,000.

[Chemical formula 23]

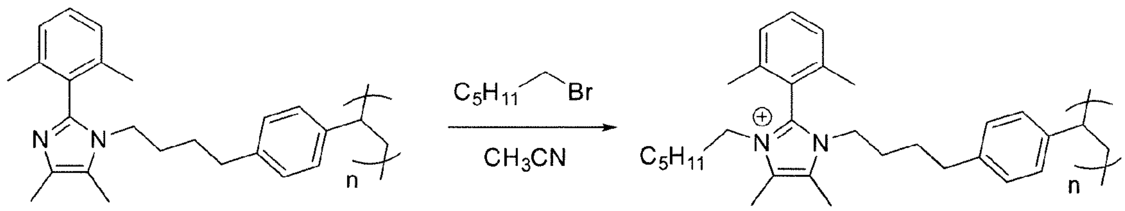


5

6

(Production Example 5)

The production as in the case of the production example 1 was carried out except that chloromethylstyrene was used in the production example 5 instead of bromobutylstyrene shown by the above formula 2. Further, the obtained compound was used instead of the compound shown by the above formula 3 of the production example 2. Thereby, an anion exchange group-containing monomer shown by the below formula 7 was produced. The monomer shown by the below formula 7 is a monomer which the alkali resistance shown in FIG.2 was measured. In FIG.7, $^1$H-NMR of the compound shown by the below formula 7 is shown.

[Chemical formula 24]

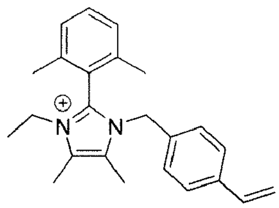

7

(Production Example 6)

The same reaction was performed as in the case of the production example 1 except that an imidazole compound shown by the below formula 8 was used in the production example 6 instead of the imidazole compound shown by the above formula 1, thereby an imidazole group-containing monomer shown by the below formula 9 was made. In FIG.8, $^{1}$H-NMR of the compound shown by the below formula 9 is shown.

[Chemical formula 25]

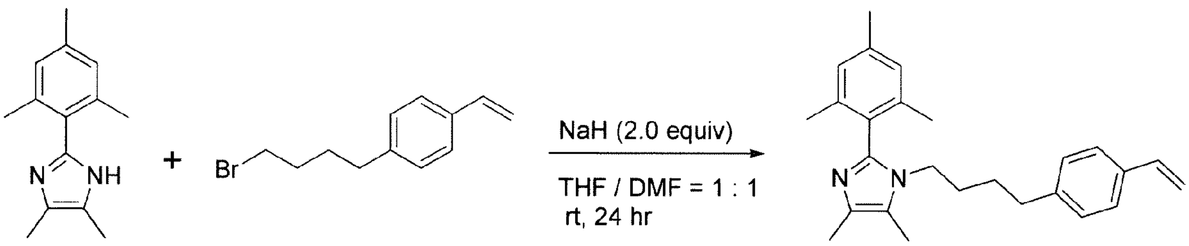


8 9

(Production Example 7)

The polymerization was carried out using a compound shown by the below formula 9 instead of the imidazole group-containing monomer shown by the above formula 3 used in the production example 3, and the resin containing the imidazole group in the side chain shown by the below formula 10 was produced. In FIG.9, $^{1}$H-NMR of the resin shown by the below formula 10 is shown. The molecular weight by GPC measurement was Mw = 110,000.

[Chemical formula 26]

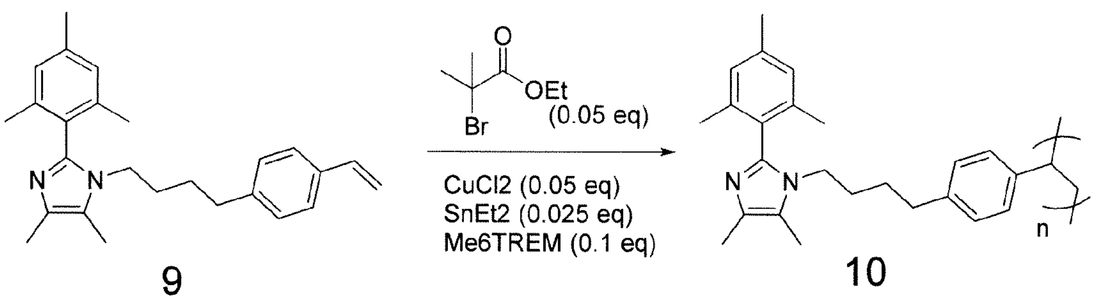


9 10

(Production Example 8)

The same process as in the case of the production example 4 was performed except that the imidazole group-containing resin shown by the formula 10 was used in the production example 8 instead of the imidazole-group containing shown by the above formula 5, thereby the anion exchange resin shown by the below formula 11 was obtained. Also, the

anion exchange resin of the below formula 11 was soluble in 1-propanol, thus it was possible to prepare 3 mass% solution by stirring for 2 days at 25°C. The physical properties of the anion exchange resin shown by the below formula 11 were as shown by Table 3.

[Chemical formula 27]

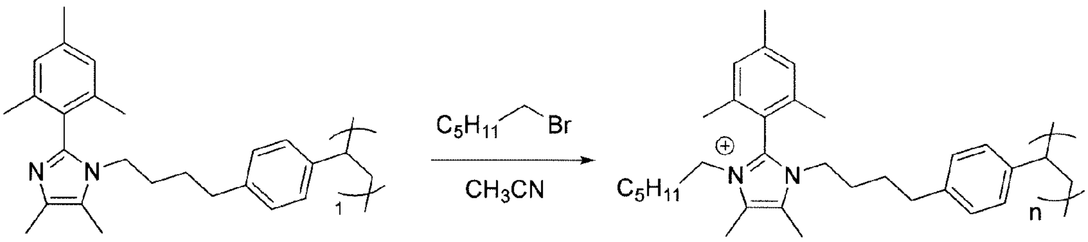


10 11

(Production Example 9)

The same reaction as in the case of the production example 1 was performed except that an imidazole compound shown by the below formula 12a was used in the production example 9 instead of the imidazole compound shown by the above formula 1, thereby an imidazole group-containing monomer shown by the below formula 12 was made. In FIG.10, $^1$H-NMR of the compound shown by the below formula 12 is shown.

[Chemical formula 28]

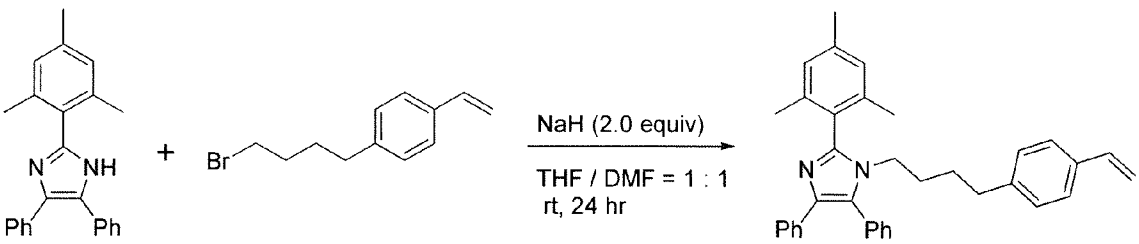


12a 12

(Production Example 10)

The imidazole group-containing monomer shown by the above formula 12 was reacted with ethyl iodide for 4 days at 80°C in acetonitrile to quaternize nitrogen in the imidazole backbone, thereby an anion exchange group-containing monomer shown by the below formula 13 was obtained. In FIG.11, $^1$H-NMR of the anion exchange group-containing monomer is shown.

[Chemical formula 29]

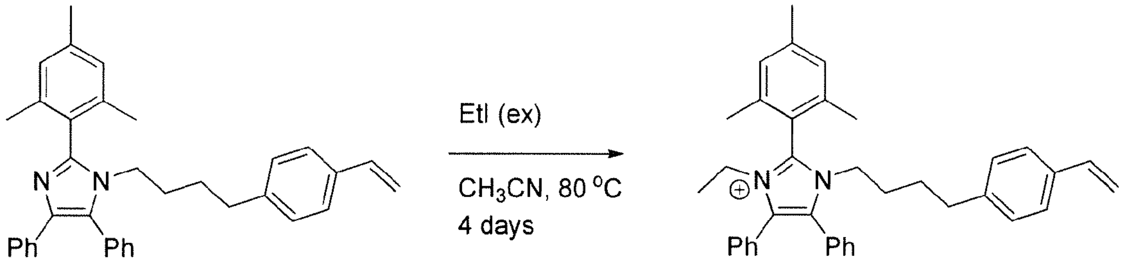


12

13

(Production example 11)

An aldehyde compound shown by the below formula 14 was reacted with ammonium acetate (2.5 eq) and 2,3-butanedion (1 eq) for 5 days at room temperature in dehydrated methanol, thereby an imidazole compound shown by the below formula 15 was obtained. This compound 15 was reacted with NaH (2.0 eq) and iodoethane (1.1 eq), thereby N-ethylimidazole compound shown by the below formula 16 was obtained. Further, this compound 16 was reacted with vinylboronic acid anhydride pyridine complex (1.01 eq) together with a Pd[P(Ph)3]4 (0.024 eq) catalyst and potassium carbonate (1.0 eq) for 24 hours at 100°C in 1,2-dimethoxyethane/water solvent, thereby an imidazole group-containing monomer shown by the below formula 17 was obtained. In FIG.12, $^1$H-NMR of the compound shown by the below formula 17, which is an imidazole group-containing monomer, is shown.

[Chemical formula 30]

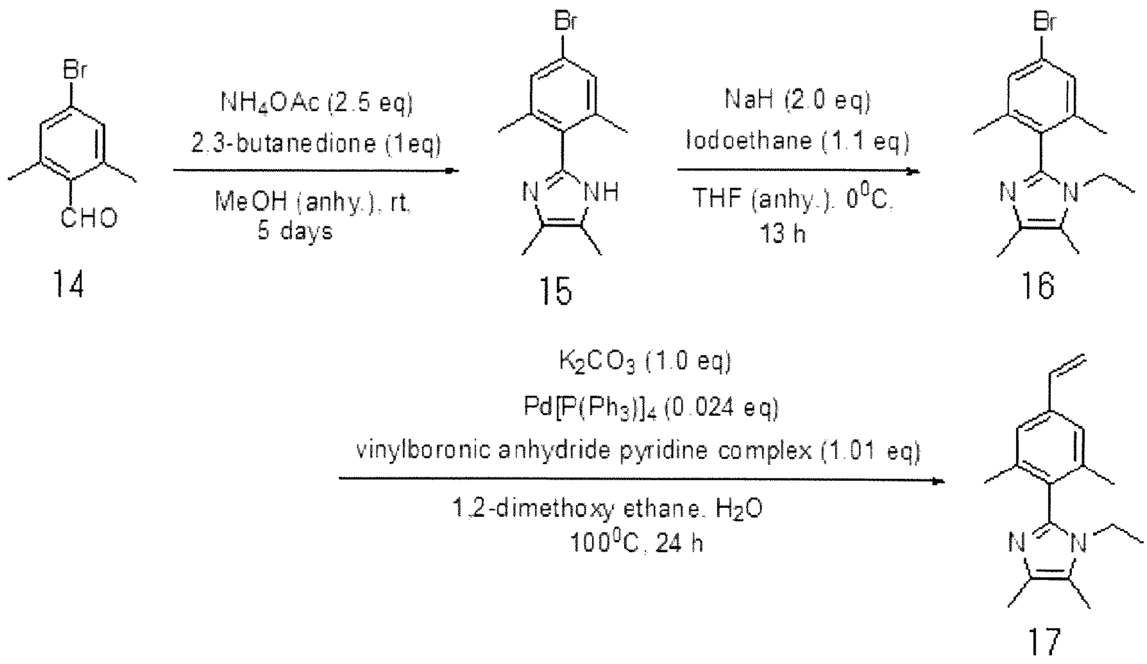


14

15

16

17

(Production example 12)

The imidazole-group containing monomer shown by the above formula 17 was reacted over night with iodoethane (3 eq) at 60°C in THF to quaternize nitrogen in the imidazole backbone, thereby an anion exchange group-containing monomer shown by the below formula 18 was obtained. In FIG.13, $^1$H-NMR of the below formula 18, which is the obtained anion exchange group-containing monomer, is shown.

[Chemical formula 31]

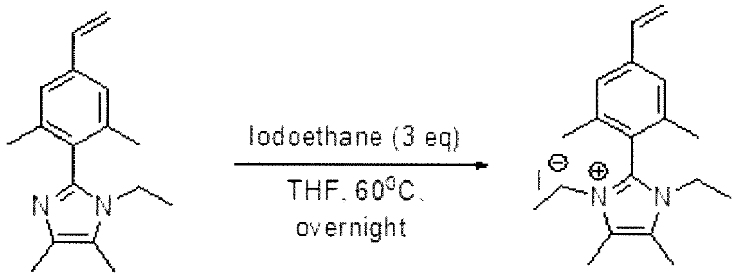


17 18

(Production Example 13)

The imidazole group-containing monomer shown by the above formula 17 was copolymerized with styrene (2.4 eq) and AIBN (0.15 eq) as an initiator, thereby a resin containing an imidazole group in the side chain, which is shown in the below formula 19, was produced. In FIG.14, $^{1}$H-NMR of the resin shown in the below formula 19, which is the resin containing an imidazole group in the side chain, is shown. A mol ratio of n/m was 0.39. Also, the imidazole group-containing resin shown by the below formula 19 had a molecular weight of Mw = 220,000 which was obtained using GPC measurement.

[Chemical formula 32]

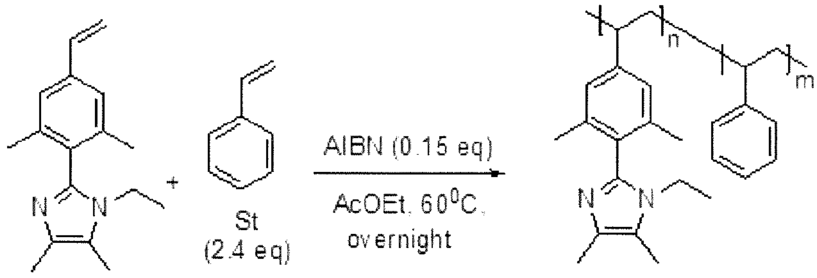


17 19

(Production Example 14)

The imidazole group-containing resin shown by the above formula 19 obtained in the production example 13 was reacted overnight with iodoethane in chlorobenzene at 60°C to quaternize nitrogen in the imidazole backbone, thereby an anion exchange resin shown by the below formula 20 was obtained. In FIG.15, $^{1}$H-NMR of the obtained anion exchange resin is shown. It was possible to quaternize nitrogen atoms in the imidazole backbone mostly quantitatively. Also, the anion exchange resin shown by the below formula 20 was soluble in 1-propanol, thus it was possible to prepare 3 mass% solution by stirring for 2 days at 25°C. Physical properties of the anion exchange resin shown by the below formula 20 were as shown in Table 3.

[Chemical formula 33]

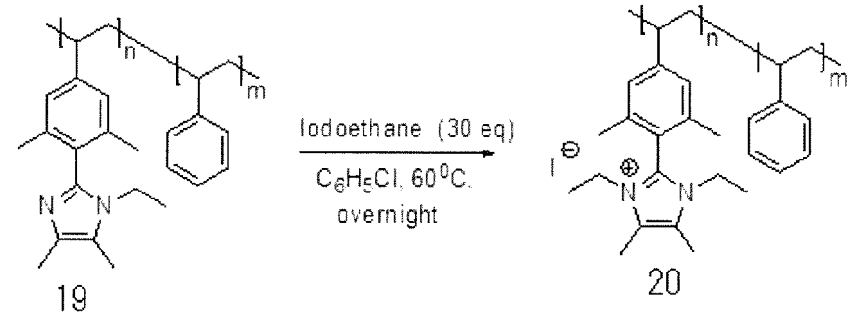


19

20

(Production example 15)

The aldehyde compound shown by the above formula 14 was reacted with ammonium acetate (2.5 eq) and 3,4-hexanedione (1.1 eq) for 2 days in dehydrated methanol at 40°C to obtain an imidazole compound shown by the below formula 21. This compound was reacted with NaH (2.0 eq) and iodomethane (1.1 eq) to obtain N-methylimidazole compound shown by the below formula 22. This compound 22 was reacted with potassium vinyltrifluoroborate (2.0 eq) under the presence of a $PdCl_2$ (dppf) (0.04 eq) catalyst and triethylamine (2.0 eq) for 4 days in isopropanol/water (10/1) solvent at 90°C to obtain an imidazole group-containing monomer shown by the below formula 23. In FIG.16, $^1$H-NMR of the compound shown by the below formula 23, which is the imidazole group-containing monomer, is shown.

[Chemical formula 34]

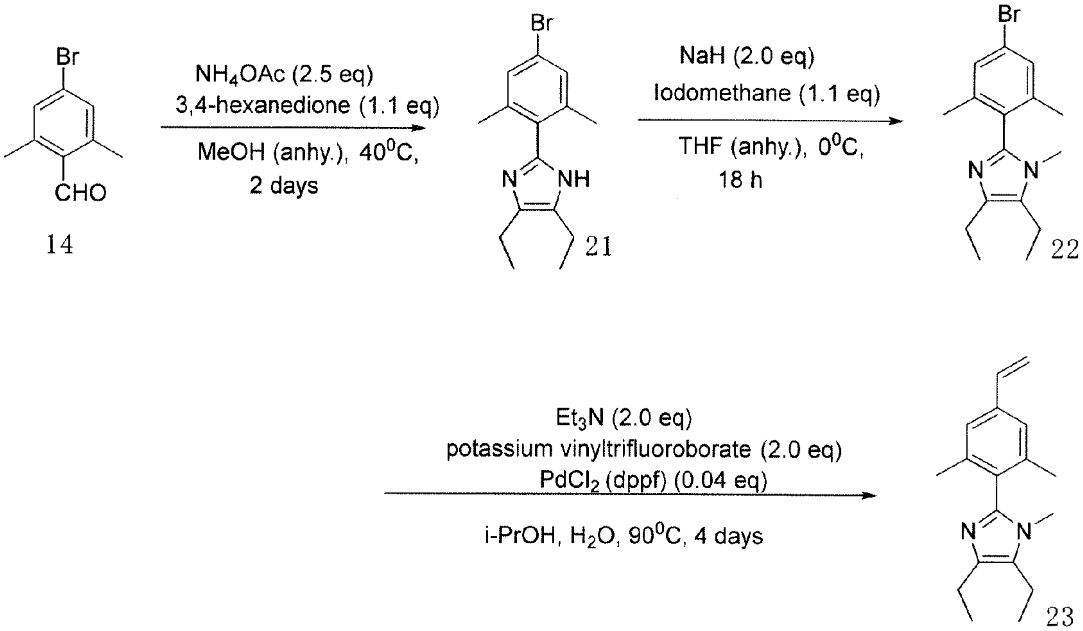


14

21

22

23

(Production Example 16)

The imidazole group-containing monomer shown by the above formula 23 was copolymerized with styrene (2.6 eq) and AIBN (0.16 eq) as an initiator, and thereby a resin containing an imidazole group in the side chain, which is shown in the below formula 24, was produced. In FIG.17, $^1$H-NMR of the resin shown in the below formula 24, which is the resin containing an imidazole group in the side chain, is shown. A mol ratio of n/m was 0.27. Also, the imidazole group-containing resin shown by the below formula 24 had a molecular weight of Mw = 107,000 which was obtained using GPC measurement.

[Chemical formula 35]

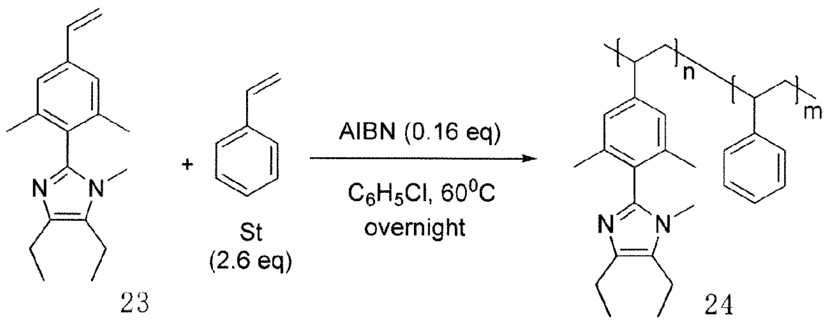


23 24

(Production example 17)

The imidazole group-containing resin shown by the above formula 24 was reacted overnight with iodomethane at 40°C in chlorobenzene to quaternize nitrogen in the imidazole backbone, and thereby an anion exchange resin shown by the below formula 25 was obtained. In FIG.18, $^1$H-NMR of the below formula 25 which is the obtained anion exchange resin is shown. It was possible to quaternize nitrogen atoms in the imidazole backbone mostly quantitatively.

[Chemical formula 36]

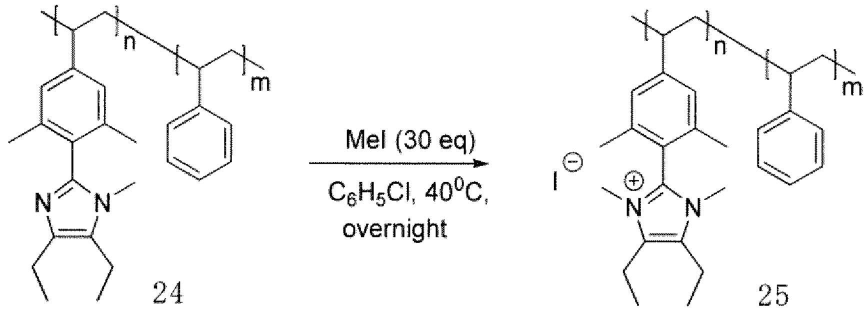


24 25

(Production example 18)

The aldehyde compound shown by the above formula 14 was reacted with ammonium acetate (2.5 eq) and 2,3-heptandione (1.1 eq) for 5 days at 40°C in dehydrated methanol, and thereby an imidazole compound shown by the below formula 26 was obtained. This compound 26 was reacted with NaH (2.0 eq) and iodomethane (1.1 eq), thereby a mixture of N-methylimidazole compounds shown by the below formula 27 and 28 was obtained. Further, the mixture of 27 and 28 was reacted with potassium vinyltrifluoroborate (2.0 eq) under the presence of a $PdCl_2$ (dppf) (0.04 eq) catalyst and triethylamine (2.0 eq) for 3 days at 80°C in ethanol solution, and thereby a mixture of imidazole group-containing monomers shown by the below formulae 29 and 30 was obtained. In FIG.19, $^1$H-NMR of the mixture of the imidazole group-containing monomers shown by the below formulae 29 and 30 is shown.

[Chemical formula 37]

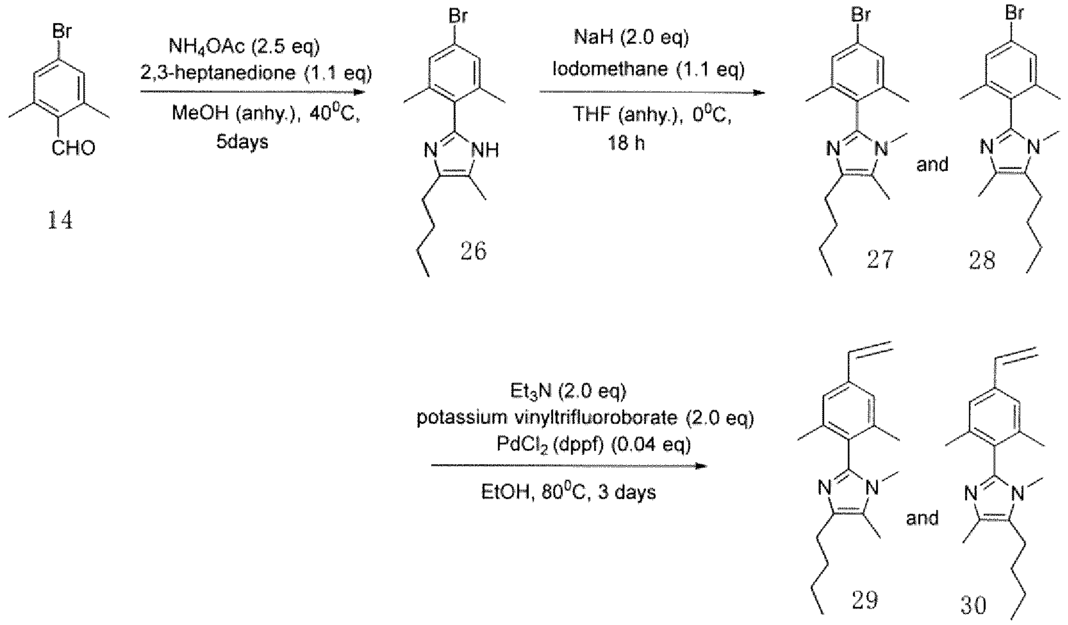


14 26 27 28 29 30

(Production example 19)

The mixture of the imidazole group-containing monomers shown by the formulae 29 and 30 was copolymerized using styrene (2.7 eq) and AIBN (0.17 eq) as an initiator, and thereby a resin containing an imidazole group in the side chain shown by the below formula 31 was produced. In FIG.20, $^1$H-NMR of the resin shown by the below formula 31, which is the resin containing an imidazole group in the side chain, is shown. A mol ratio of n/m was 0.38. Also, the imidazole group-containing resin shown by the below formula 31 had a molecular weight of Mw = 167,000 which was obtained using GPC measurement (DMF).

[Chemical formula 38]

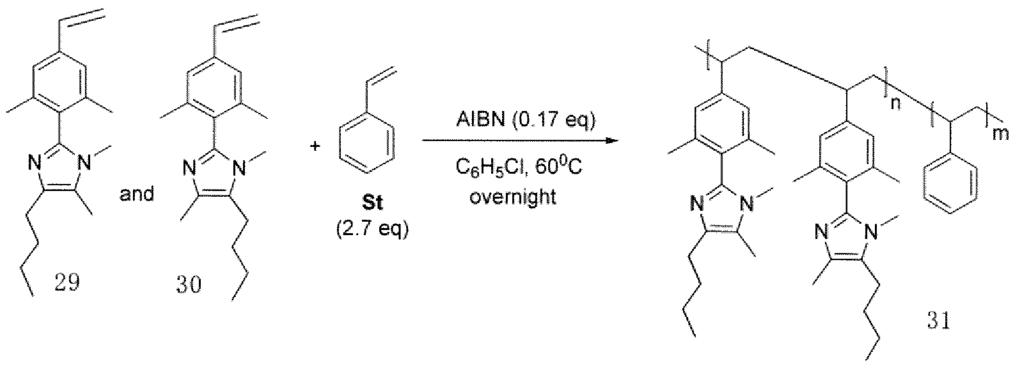


29 30 31

(Production example 20)

The imidazole group-containing resin shown by the above formula 31 was reacted overnight with iodomethane at 40°C in chlorobenzene to quaternize nitrogen in the imidazole backbone, and thereby an anion exchange resin shown by the below formula 32 was obtained. In FIG.21, $^1$H-NMR of the below formula 32 which is the obtained anion exchange resin is shown. It was possible to quaternize nitrogen atoms in the imidazole backbone mostly quantitatively.

[Chemical formula 39]

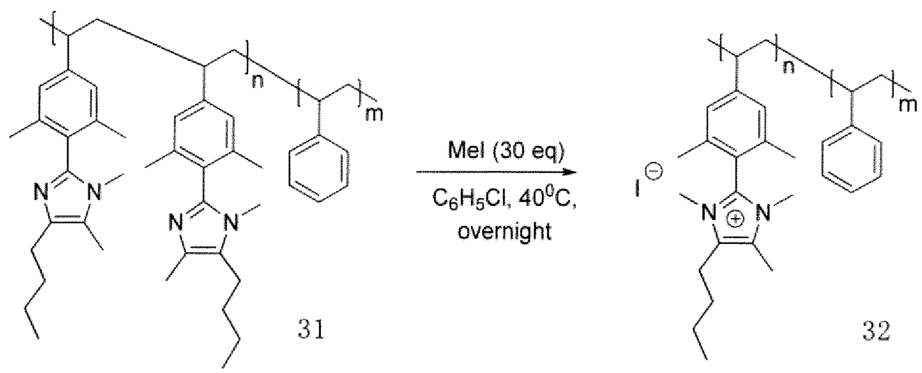


31 32

(Reference Example 1)

40 g of commercially available polystyrene-poly(ethylene-butylene)-polystyrene triblock copolymer (a weight average molecular weight of 70,000, a styrene content of 42 weight%) (containing 161 mmol of benzene rings) was dissolved in a mixed solvent of 520 g of chloroform and 760 g (9.4 mol) of chloromethyl methyl ether. Further, a solution containing 35 g (134 mmol) of tin (IV) chloride dissolved in 83 g of chloroform was added, and reacted under nitrogen atmosphere at a temperature between 35 to 40°C for 2 hours. Thereby, chloromethyl groups were introduced into the resin. 200 ml of a mixed solution of 1,4-dioxane and water in 1:1 ratio was introduced in the reaction solution in order to stop the reaction, and then 6000 ml of a methanol solution was introduced to precipitate the resin. After washing for several times using methanol, it was dried for 15 hours or longer at 25°C, thereby 45.5 g of a polystyrene-poly(ethylene-propylene)-polystyrene triblock copolymer, in which chloromethyl groups were introduced into the benzene rings, was obtained as a chloromethyl group-containing resin.

40 g of the chloromethyl group-containing resin obtained by the above method was immersed in a mixed solution which contained 823 g of 30 mass% trimethylamine solution, 594 g of acetone, and 2560 g of water, and these were stirred for 15 hours or longer at 25°C. This resin solution was introduced into 2000 ml of 1 mol/L hydrochloric acid, and thereby, an anion exchange resin obtained by filtration having halogen ions as counter ions was obtained. The anion exchange resin was washed twice using hydrochloric acid, and then washed several times using the ion exchange water. Further, the anion exchange resin was immersed in 4 L of 0.5 mol/L sodium bicarbonate solution for 4 times or more, and then washed several times using the ion exchange water. Next, the obtained anion exchange resin was washed for five times using 2000 ml of tetrahydrofuran, then dried for 15 hours at 25°C. Thereby, 51.9 g of polystyrenepoly(ethylene-propylene)-polystyrene triblock copolymer introduced with a quaternary ammonium salt group which the counter ion was a bicarbonate type, was obtained. 3.0 g was taken from the obtained anion exchange resin, and it was stirred and dissolved in 1-propanol at 25°C, and thereby 100 g of 3 mass% solution was prepared. The physical properties of the obtained anion exchange resin were as shown in Table 3. The obtained anion exchange resin had a low solubility in water, had a low remaining ratio of the anion exchange resin, and had a poor alkali resistance.

(Reference Example 2)

The commercially available polystyrene-poly(ethylene-butylene)-polystyrene triblock copolymer used in the production of the anion exchange resin of the reference example 1 was changed to that having a weight average molecular weight of 50000 and a styrene content of 67 wt%. Further, the same process as the reference example 1 was performed while changing the amounts of chloroform to 450 g, chloromethylmethylether to 1093 g, and chloride tin (IV) to 41 g which were used for a chloromethylation reaction. Thereby, the anion exchange resin was prepared. 3.0 g was taken from the obtained anion exchange resin, and it was stirred and dissolved in 1-propanol at 25°C to prepare 100 g of 3 mass% solution. The physical properties of the obtained anion exchange resin were as shown in Table 3. The obtained anion exchange resin was highly soluble in water.

[Table 3]

| | Ion exchange capacity (mmol/g) | Solubility (%) to water at 20°C | Change rate (%) of anion exchange capacity |
|---|---|---|---|
| Production example 4 | 2.5 | 0.3 | 96 |

(continued)

| | Ion exchange capacity (mmol/g) | Solubility (%) to water at 20°C | Change rate (%) of anion exchange capacity |
|---|---|---|---|
| Production example 8 | 2.5 | 0.3 | 95 |
| Production example 14 | 3.1 | 0.1 | 98 |
| Reference example 1 | 2.6 | 0.5 | 8 |
| Reference example 2 | 3.6 | 100 | 20 |

(Production Example 1 of Anion Exchange Membrane)

An anion exchange membrane was produced using the imidazole group-containing monomer shown by the above formula 3 which was produced in above (production example 1). 9.5 g of the imidazole group-containing monomer shown by the above formula 3, 0.5 g of divinylbenzene, and 5 g of t-butylperoxyethylhexanoate were stirred and mixed at room temperature, and thereby a monomer composition was prepared. A porous membrane (a thickness of 25 μm, a porosity of 37%, an average opening size of 0.03 μm) made of the polyethylene (PE, a weight average molecular weight of 250000) was immersed in the obtained monomer composition for 10 minutes at 25°C in the air.

Next, the porous membrane was taken out of the monomer composition, and 100 μm of polyester film was coated as a releasing material to both sides of the porous membrane. The coated porous membrane was heated at 80°C for 5 hours while applying nitrogen at 0.3 MPa, thereby the monomer impregnated in the porous membrane was polymerized.

Next, the polyester film was released. The porous membrane, in which the impregnated monomer composition had been polymerized, was immersed in iodomethyl/tetrahydrofuran solution of 10 mass% concentration for 2 days at room temperature. Thereby, the anion exchange membrane, in which imidazole was quaternized, was obtained. The anion exchange capacity, the membrane resistance, the remaining ratio of the anion exchange groups of the obtained anion exchange membrane were measured and the results are show in Table 4. Compared to the anion exchange membrane shown in below (the production example 2 of the anion exchange membrane), the obtained anion exchange membrane had a higher change rate of the anion exchange capacity, and had a better alkali resistance.

(Production Example 2 of Anion Exchange Membrane)

9 g of chloromethylstyrene, 1 g of divinylbenzene, 0.5 g of polyethylene glycol epoxide, and 0.5 g of t-butyl peroxyethylhexanoate were mixed and stirred at room temperature to prepare a monomer composition.

Next, the same polyethylene porous membrane used in the production example 1 of the anion exchange membrane was used, and under the same condition, it was immersed in the monomer composition obtained using the above method. Then, heat polymerization was carried out under the same condition as used in the production example 1 of the anion exchange membrane, and a porous membrane, in which the impregnated monomer composition had been polymerized, was obtained.

Next, the porous membrane, in which the monomer composition had been polymerized, was immersed in 20 mass% trimethylamine/acetone solution for 24 hours at room temperature to obtain a quaternary ammonium salt type-anion exchange membrane (anion exchange membrane). The anion exchange capacity, the membrane resistance, the remaining ratio of the anion exchange groups of the obtained anion exchange membrane were measured, and the results are show in Table 4. The change rate of the anion exchange capacity of the obtained anion exchange membrane was lower than that of the anion exchange membrane obtained by the production example 1. The anion exchange group of the obtained anion exchange membrane had a known benzyltrimethylammonium salt structure, and exhibited a poor alkali resistance.

[Table 4]

| | Ion exchange capacity (mmol/g) | Resistance of anion exchange membrane (Ω · cm2) | Change rate (%) of anion exchange capacity |
|---|---|---|---|
| Production example 1 of anion exchange membrane | 1.8 | 0.4 | 97 |
| Production example 2 of anion exchange membrane | 2.5 | 0.4 | 9 |

(Examples 1 to 3)

The anion exchange membrane obtained using the production example 1 of the anion exchange membrane was used as an anion exchange membrane for Examples 1 to 3. As anion exchange resins for forming the anode electrode (catalytic electrode) and the cathode electrode (catalytic electrode), the anion exchange resin shown by the formula 6 produced in the production example 4, the anion exchange resin shown by the formula 11 produced in the production example 8, and the anion exchange resin shown by the formula 20 produced in the production example 14 were respectively used. A water electrolysis performance which is an initial performance and an increase rate of cell voltage which indicates a simple alkali resistance of the water electrolysis cell were evaluated, and the results are shown in Table 5. Table 6 shows configurations thereof.

(Examples 4 to 6)

The anion exchange membrane obtained using the production example 2 of the anion exchange membrane was used as an anion exchange membrane for Examples 4 to 6. As anion exchange resins for forming the anode electrodes (catalytic electrode) and the cathode electrode (catalytic electrode), the anion exchange resin shown by the formula 6 produced in the production example 4, the anion exchange resin shown by the formula 11 produced in the production example 8, and the anion exchange resin shown by the formula 20 produced in the production example 14 were respectively used. A water electrolysis performance which is an initial performance and an increase rate of cell voltage which indicates a simple alkali resistance of the water electrolysis cell were evaluated, and the results are shown in Table 5. Table 6 shows configurations thereof.

(Example 7)

The anion exchange membrane obtained using the production example 1 of the anion exchange membrane was used as an anion exchange membrane. As anion exchange resins for forming the anode electrode (catalytic electrode) and the cathode electrode (catalytic electrode), the anion exchange resin produced in the reference example 1 was used. A water electrolysis performance which is an initial performance and an increase rate of cell voltage which indicates a simple alkali resistance of the water electrolysis cell were evaluated, and the results are shown in Table 5. Table 6 shows configurations thereof.

(Comparative Examples 1 and 2)

The anion exchange membrane obtained using the production example 2 of the anion exchange membrane was used as an anion exchange membrane. As anion exchange resins for forming the anode electrode (catalytic electrode) and the cathode electrode (catalytic electrode), the anion exchange resin produced in the reference example 1 and the reference example 2 were respectively used. The water electrolysis performance which is an initial performance and water were evaluated. The results are shown in Table 5. In the case of using the anion exchange resin produced by the reference example 2, it was speculated that the anion exchange resin had eluted in water at a current density of 500 $mA/cm^2$ or higher which caused the electrodes to break, therefore it was not possible to operate. Table 6 shows configurations thereof.

[Table 5]

| | Water electrolysis performance Cell voltage(V)at each current density | | | Increase rate (%) of cell voltage |
|---|---|---|---|---|
| | 200 $mA/cm^2$ | 500 $mA/cm^2$ | 1,000 $mA/cm^2$ | |
| Example 1 | 1.7 | 2.1 | 2.6 | 105 |
| Example 2 | 1.7 | 2.2 | 2.7 | 105 |
| Example 3 | 1.6 | 2.0 | 2.5 | 105 |
| Example 4 | 1.7 | 2.3 | 2.7 | 109 |
| Example 5 | 1.7 | 2.4 | 2.8 | 108 |
| Example 6 | 1.6 | 2.2 | 2.9 | 109 |
| Example 7 | 1.7 | 2.4 | 2.7 | 179 |
| Comparative example 1 | 1.8 | 2.3 | 2.5 | 205 |

(continued)

| | Water electrolysis performance Cell voltage(V)at each current density | | | Increase rate (%) of cell voltage |
|---|---|---|---|---|
| | 200 mA/cm$^2$ | 500 mA/cm$^2$ | 1,000 mA/cm$^2$ | |
| Comparative example 2 | 3.5 | Unable to measure | Unable to measure | - |

[Table 6]

| | Anion exchange membrane | | | Anion exchange resin used for anode electrode and cathode electrode |
|---|---|---|---|---|
| | Production example | Type | Used anion exchange resin | |
| Example 1 | Production example 1 of anion exchange membrane | Porous membrane-supported type | · Polymer of composition including monomer shown by formula 3<br>· Resin obtained by quaternizing the polymer using iodomethyl | Anion exchange resin shown by formula 6 |
| Example 2 | | | | Anion exchange resin shown by formula 11 |
| Example 3 | | | | Anion exchange resin shown by formula 20 |
| Example 4 | Production example 2 of anion exchange membrane | Porous membrane-supported type | · Polymer of composition including chloromethylstyrene<br>· Resin obtained by quaternizing chloromethyl group of the polymer using trimethylamine | Anion exchange resin shown by formula 6 |
| Example 5 | | | | Anion exchange resin shown by formula 11 |
| Example 6 | | | | Anion exchange resin shown by formula 20 |
| Example 7 | Production example 1 of anion exchange membrane | Porous membrane-supported type | · Polymer of composition including monomer shown by formula 3<br>· Resin obtained by quaternizing the polymer using iodomethyl | (Reference example 1)<br>· Polystyrene-poly(ethylene-propylene)-polystyrene triblock copolymer introduced with chloromethyl group<br>· Resin obtained by quaternizing chloromethyl group of the polymer using trimethylamine |
| Comparative example 1 | Production example 2 of anion exchange membrane | Porous membrane-supported type | · Polymer of composition including chloromethylstyrene<br>· Resin obtained by quaternizing chloromethyl; group of the polymer using trimethylamine | (Reference example 1)<br>· Polystyrene-poly(ethylene-propylene)-polystyrene triblock copolymer introduced with chloromethyl group<br>· Resin obtained by quaternizing chloromethyl group of the copolymer using trimethylamine |
| Comparative example 2 | Production example 2 of anion exchange membrane | Porous membrane-supported type | · Polymer of composition including chloromethylstyrene<br>· Resin obtained by quaternizing chloromethyl group of the polymer using trimethylamine | (Reference example 2)<br>· Copolymer of chloromethylstylene, divinyl benzene, and polyethylene glycol epoxide<br>· Resin obtained by quaternizing chloromethyl group of the copolymer using trimethylamine |

## REFERENCE SIGNS LIST

8; Anion exchange resin
11; Cathode side catalytic electrode layer
12; Anode side catalytic electrode layer
13, 14; Raw water supply tube
15, 16; Gas collection tube
20; Water electrolysis device
30; External power source
31, 32; Conductive wires

## Claims

1. An anion exchange resin comprising a side chain shown by a below formula (1).

[Chemical formula 40]

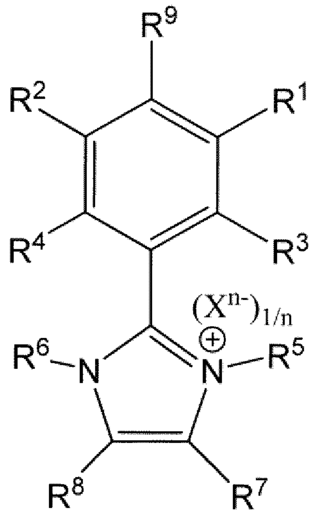

(1)

In the formula (1), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with a main chain forming the anion exchange resin;
$R^5$ and $R^6$ each represents an alkyl group which may contain a substituent group, a cycloalkyl group which may contain a substituent group, an aryl group which may contain a substituent group, or a group which bonds with the main chain forming the anion exchange resin;
at least one of $R^1$, $R^2$, $R^3$, $R^4$, $R^5$, $R^6$, $R^7$, $R^8$, and $R^9$ is a group which bonds with the main chain of the anion exchange resin; and
$x^{n-}$ represents an anion and n represents an anion valance.

2. An anion exchange membrane comprising the anion exchange resin according to claim 1.

3. An anion exchange membrane comprising a porous membrane as a base material, and voids of the porous membrane are filled with the anion exchange resin according to claim 1.

4. A catalytic electrode forming composition comprising the anion exchange resin according to claim 1 and an electrocatalyst metal.

5. An anion exchange group-containing monomer shown by a below formula (2)

[Chemical formula 41]

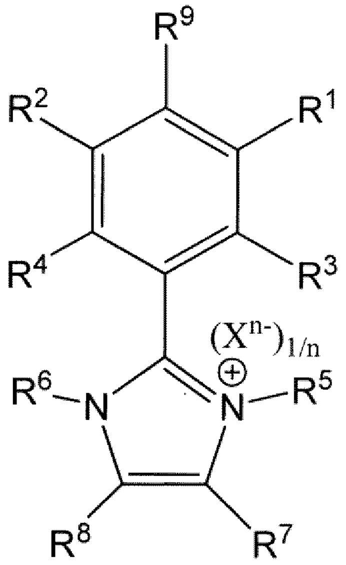

(2)

In the formula (2), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a polymerizable group shown by a below formula (P);

$R^5$ and $R^6$ each represents an alkyl group which may contain a substituent group, a cycloalkyl group which may contain a substituent group, an aryl group which may contain a substituent group, or a polymerizable group shown by a below formula (P);

at least one of $R^1$, $R^2$, $R^3$, $R^4$, $R^5$, $R^6$, $R^7$, $R^8$, and $R^9$ is the polymerizable group shown by the below formula (P); and

$x^{n-}$ represents an anion and n represents an anion valance,

[Chemical formula 42]

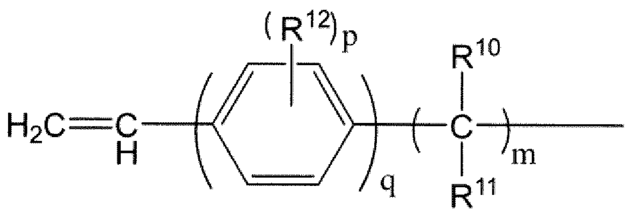

(P)

In the formula (P), $R^{10}$ and $R^{11}$ each represents a hydrogen atom, an alkyl group which may contain a substituent group, a cycloalkyl group which may contain a substituent group, or an aryl group which may contain a substituent group;

m is an integer of 0 to 10;

$R^{12}$ represents a hydrogen atom, an alkyl group which may contain a substituent group, a cycloalkyl group which may contain a substituent group, or an aryl group which may contain a substituent group;

p is an integer of 0 to 4; and

q is an integer of 0 to 10.

6. A polymerization-curable composition comprising the anion exchange group-containing monomer according to claim 5.

7. An anion exchange resin obtained by polymerizing the polymerization-curable composition according to claim 6.

**8.** An anion exchange membrane obtained by coating and polymerizing the polymerization-curable composition according to claim 6.

**9.** A method for producing an anion exchange membrane, comprising:

filling at least voids of a porous membrane with the polymerization-curable composition according to claim 6 by contacting the porous membrane and the polymerization-curable composition; and
polymerizing the polymerization-curable composition to thereby produce an anion exchange membrane in which an anion exchange resin is filled in the voids.

**10.** A monomer containing an imidazole group shown by a below formula (3).

[Chemical formula 43]

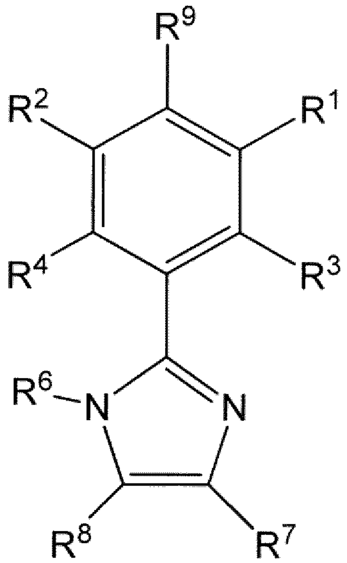

(3)

In the formula (3), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a polymerizable group shown by a below formula (P);
$R^6$ represents an alkyl group which may contain a substituent group, a cycloalkyl group which may contain a substituent group, an aryl group which may contain a substituent group, or a polymerizable group shown by a below formula (P); and
at least one of $R^1$, $R^2$, $R^3$, $R^4$, $R^6$, $R^7$, $R^8$, and $R^9$ is a polymerizable group shown by below formula (P),

[Chemical formula 44]

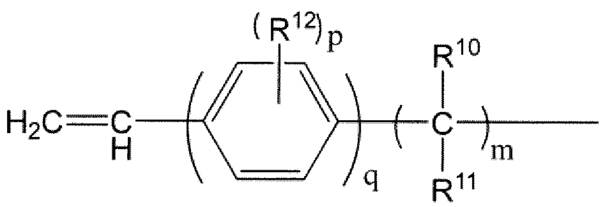

(P)

In the formula (P), $R^{10}$ and $R^{11}$ each represents a hydrogen atom, an alkyl group which may contain a substituent group, a cycloalkyl group which may contain a substituent group, or an aryl group which may contain a substituent group;
m is an integer of 0 to 10;
$R^{12}$ represents a hydrogen atom, an alkyl group which may contain a substituent group, a cycloalkyl group which

may contain a substituent group, or an aryl group which may contain a substituent group;
p is an integer of 0 to 4; and
q is an integer of 0 to 10.

**11.** A polymerization-curable composition comprising a monomer containing the imidazole group according to claim 10.

**12.** A method for producing an anion exchange resin comprising a side chain shown by a below formula (1'), comprising:

polymerizing the polymerization-curable composition according to claim 11 to obtain a resin containing an imidazole group; and
contacting the obtained resin containing the imidazole group with $R^{5'}$-X1 which is a halogen-containing compound containing at least one selected from an alkyl group which may containing a substituent group, a cycloalkyl group which may contain a substituent group, or an aryl group which may contain a substituent group.

[Chemical formula 45]

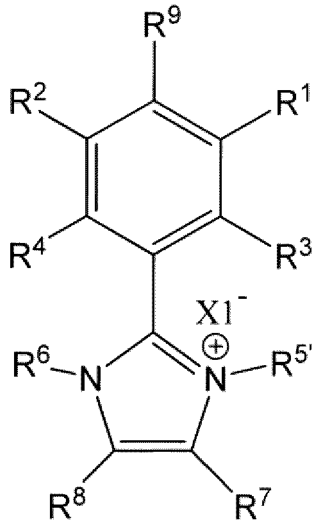

(1')

In the formula (1'), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with a main chain forming the anion exchange resin by polymerizing the polymerizable group shown by the above-formula (P);
$R^{5'}$ represents an alkyl group which may contain a substituent group, a cycloalkyl group which may contain a substituent group, or an aryl group which may contain a substituent group;
$R^6$ represents an alkyl group which may contain a substituent group, a cycloalkyl group which may contain a substituent group, an aryl group which may contain a substituent group, or the group which bonds with the main chain forming the anion exchange resin by polymerizing the polymerizable group shown by the above-formula (P);
at least one of $R^1$, $R^2$, $R^3$, $R^4$, $R^6$, $R^7$, $R^8$, and $R^9$ is the group which bonds with the main chain forming the anion exchange resin by polymerizing the polymerizable group shown by the above-formula (P); and
X1- represents a halogen ion.

**13.** A method for producing an anion exchange membrane including a quaternary imidazole group which contains a halogen ion as a counter ion, comprising:

coating a polymerization-curable composition according to claim 11 to form a membrane;
contacting a resin membrane containing an imidazole group obtained by polymerizing the polymerization-curable composition and halogenated alkyl which contains an alkyl group.

**14.** A method for producing an anion exchange membrane including a quaternary imidazole group which contains a halogen ion as a counter ion, comprising:

filling at least voids of a porous membrane with the polymerization-curable composition according to claim 11 by contacting the porous membrane and the polymerization-curable composition;
polymerizing the polymerization-curable composition to thereby produce an anion exchange membrane precursor in which a resin containing an imidazole group is filled in the voids; and
contacting the obtained anion exchange membrane precursor and halogenated alkyl which contains an alkyl group.

**15.** A method for producing an anion exchange resin shown by a below formula (1"), comprising:

forming the anion exchange resin containing a halogen ion as a counter ion using the method according to claim 12; and
contacting the anion exchange resin and substances containing an anion other than the halogen ion.

[Chemical formula 46]

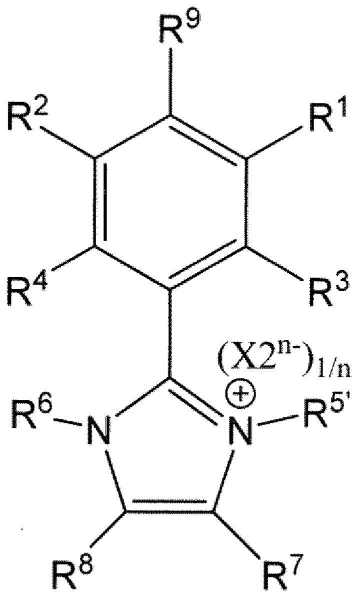

(1")

In the above-formula (1"), $R^1$, $R^2$, $R^3$, $R^4$, $R^7$, $R^8$, and $R^9$ each represents a hydrogen atom, an alkyl group which may contain a substituent group, a cycloalkyl group which may contain a substituent group, an aryl group which may contain a substituent group, or a group which bonds with the main chain forming the anion exchange resin by polymerizing the polymerizable group shown by the above-formula (P);
$R^{5'}$ represents an alkyl group which may contain a substituent group, a cycloalkyl group which may contain a substituent group, or an aryl group which may contain a substituent group;
$R^6$ represents an alkyl group which may contain a substituent group, a cycloalkyl group which may contain a substituent group, an aryl group which may contain a substituent group, or the group which bonds with the main chain forming the anion exchange resin by polymerizing the polymerizable group shown by the above-formula (P);
at least one of $R^1$, $R^2$, $R^3$, $R^4$, $R^6$, $R^7$, $R^8$, and $R^9$ is the group which bonds with the main chain forming the anion exchange resin by polymerizing the polymerizable group shown by the above-formula (P); and
$X2^{n-}$ represents an anion other than a halogen ion and n represents an anion valence.

**16.** A method for producing an anion exchange membrane, comprising:

forming an anion exchange membrane containing a quaternary imidazole group including a halogen ion as a counter ion using the method according to claim 13; and
contacting the obtained anion exchange membrane and a substance containing an anion other than the halogen ion.

**17.** A method for producing an anion exchange membrane, comprising:

forming an anion exchange membrane containing a quaternary imidazole group including a halogen ion as a counter ion using the method according to claim 14; and
contacting the obtained anion exchange membrane and a substance containing an anion other than the halogen ion.

FIG.1

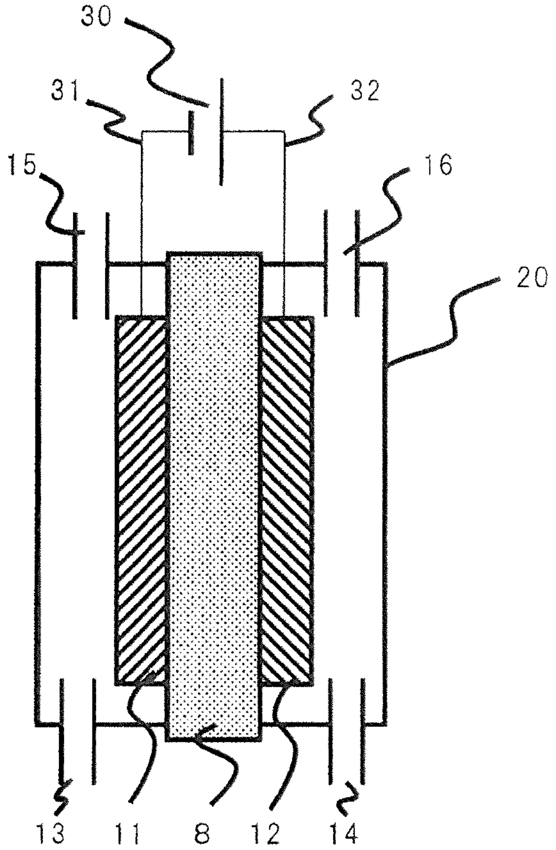
30
31
32
15
16
20
13
11
8
12
14

FIG.2

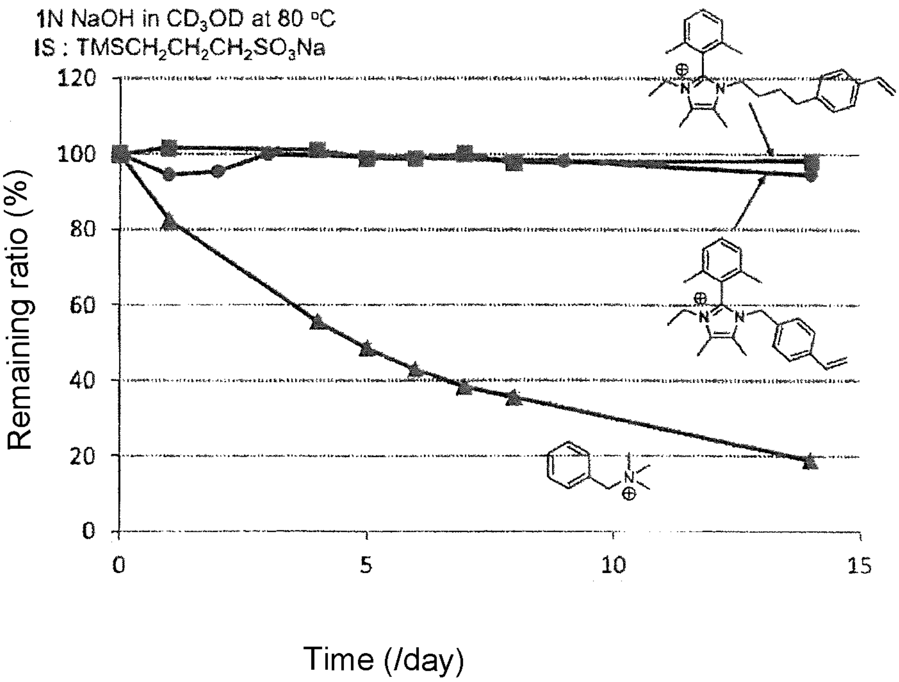
1N NaOH in $CD_3OD$ at 80 °C
IS : $TMSCH_2CH_2CH_2SO_3Na$
Remaining ratio (%)
0
20
40
60
80
100
120
0
5
10
15
Time (/day)

FIG.3

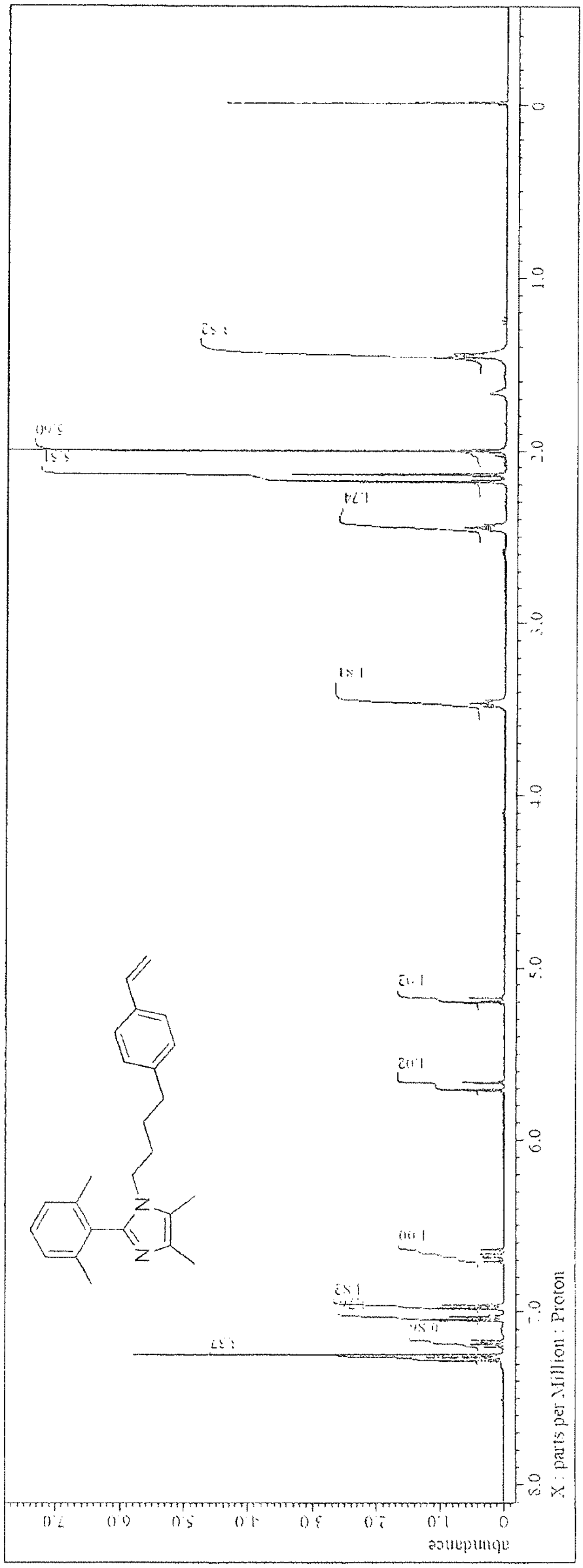

FIG.4

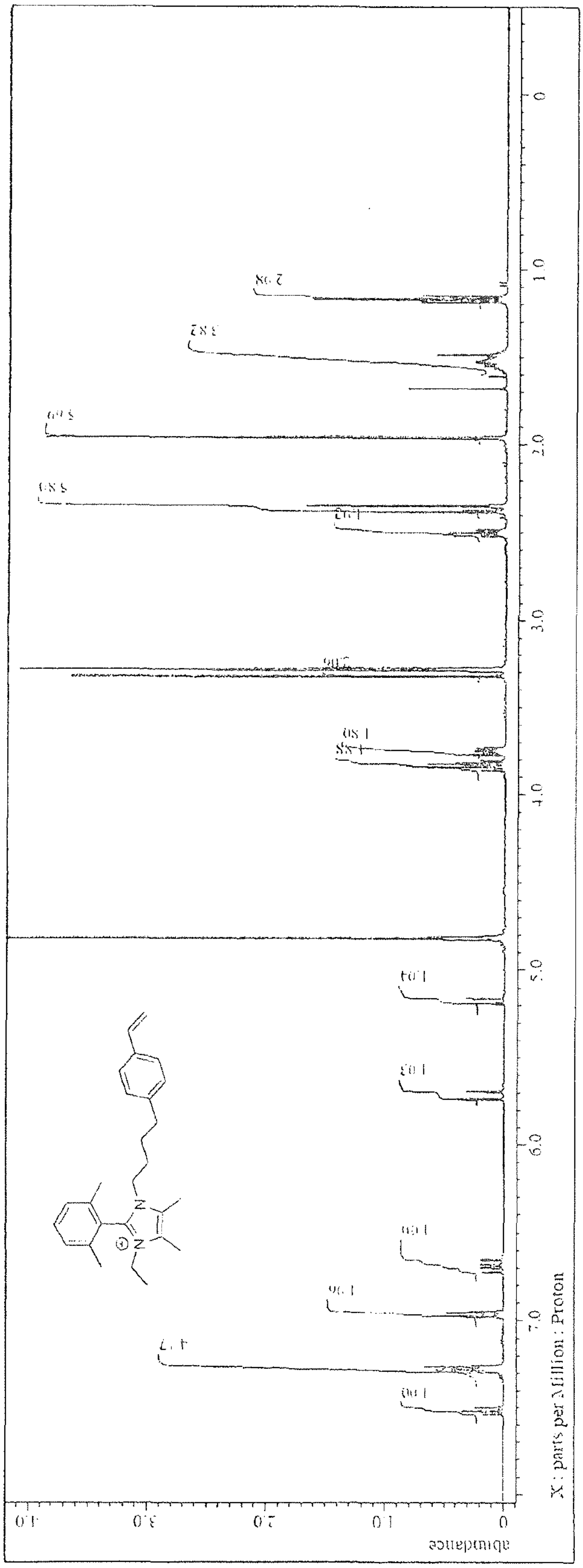

FIG.5

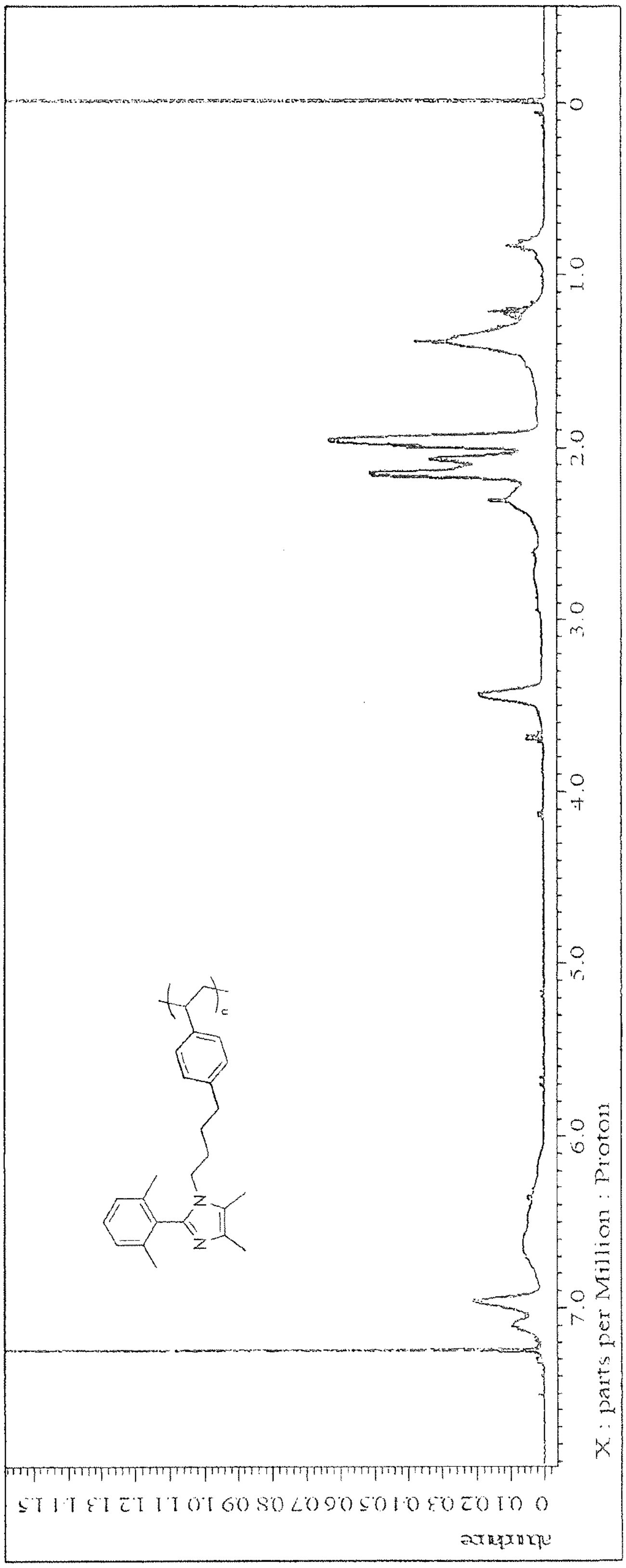

FIG.6

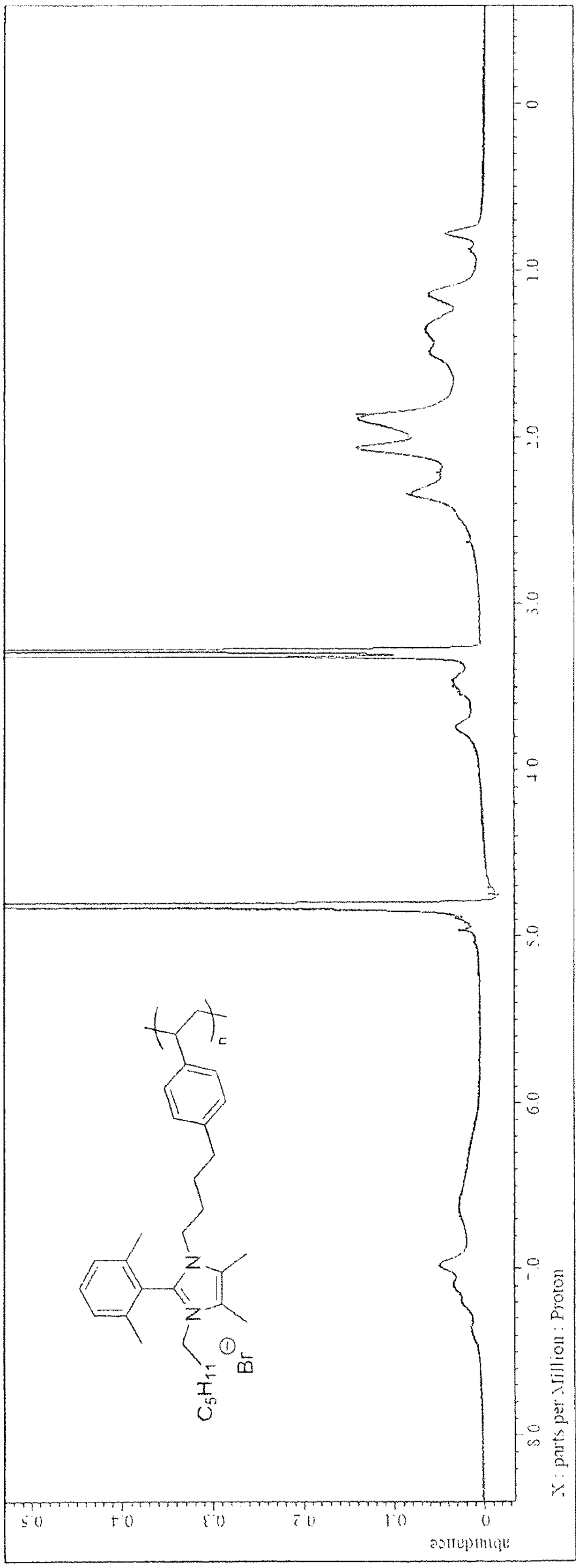

FIG.7

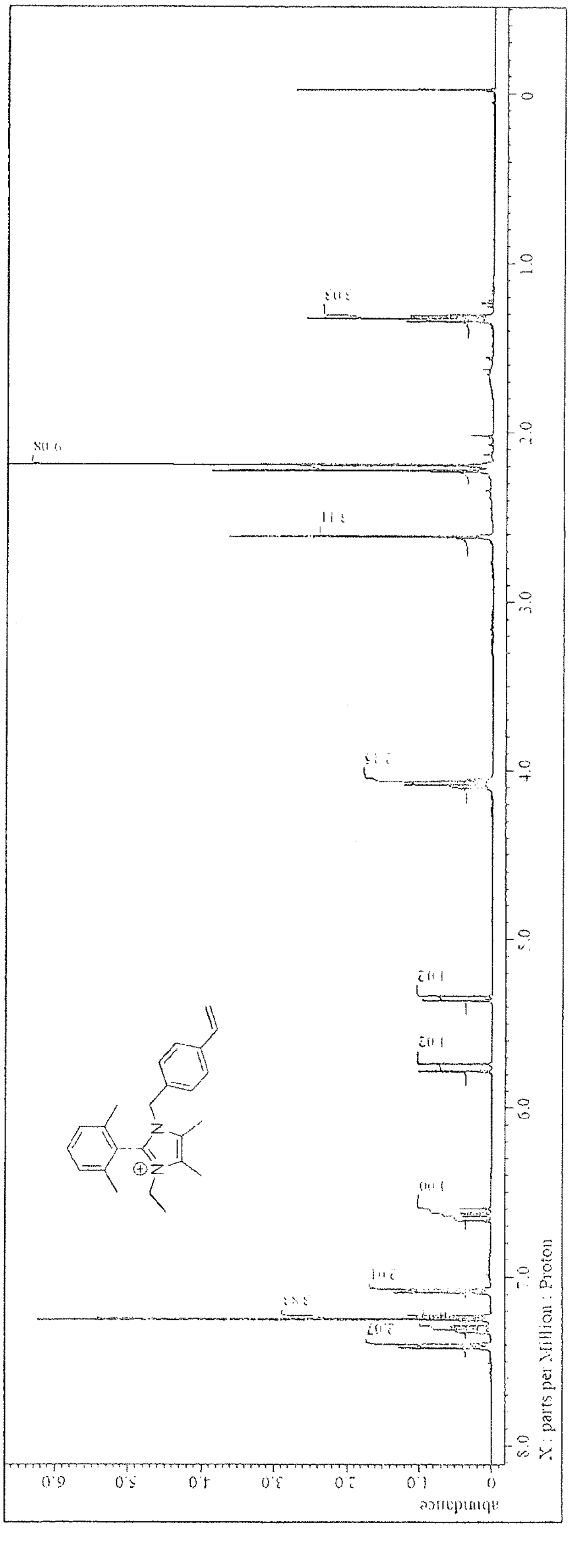

FIG.8

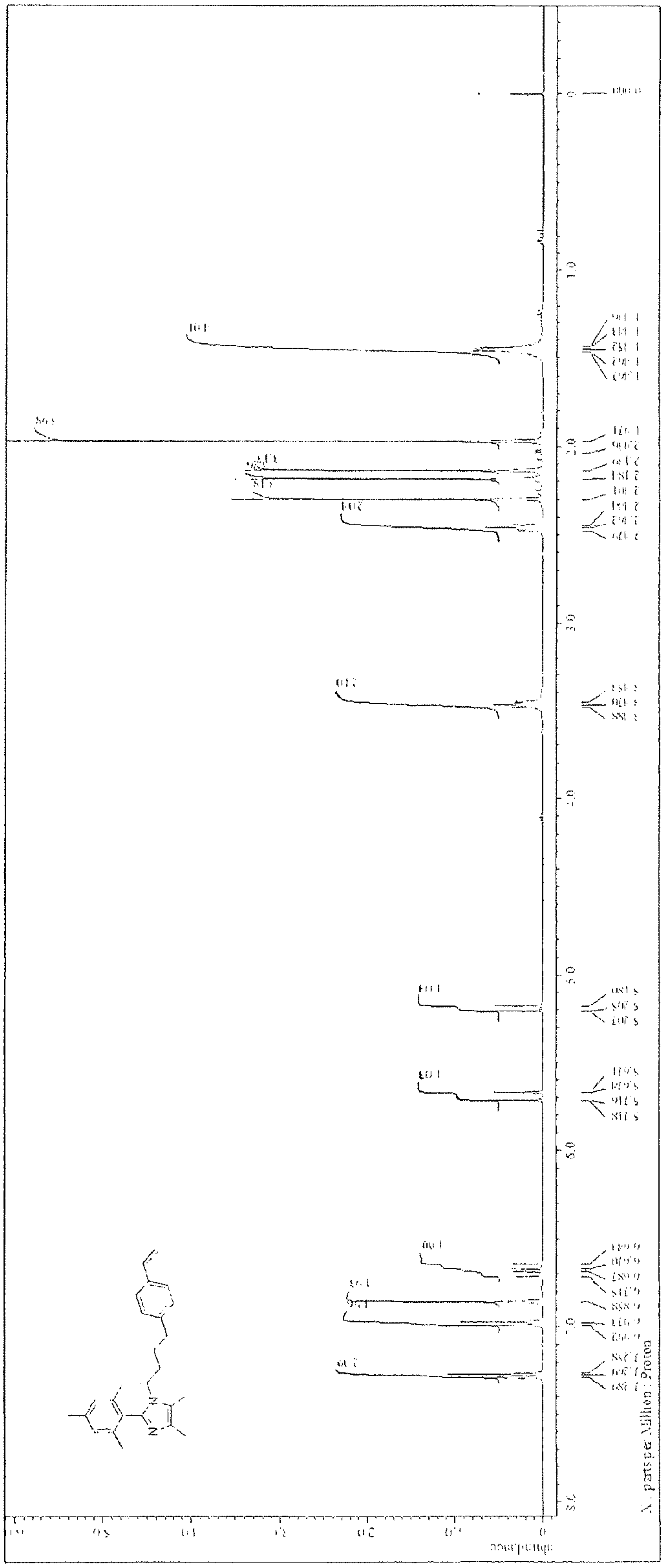

FIG.9

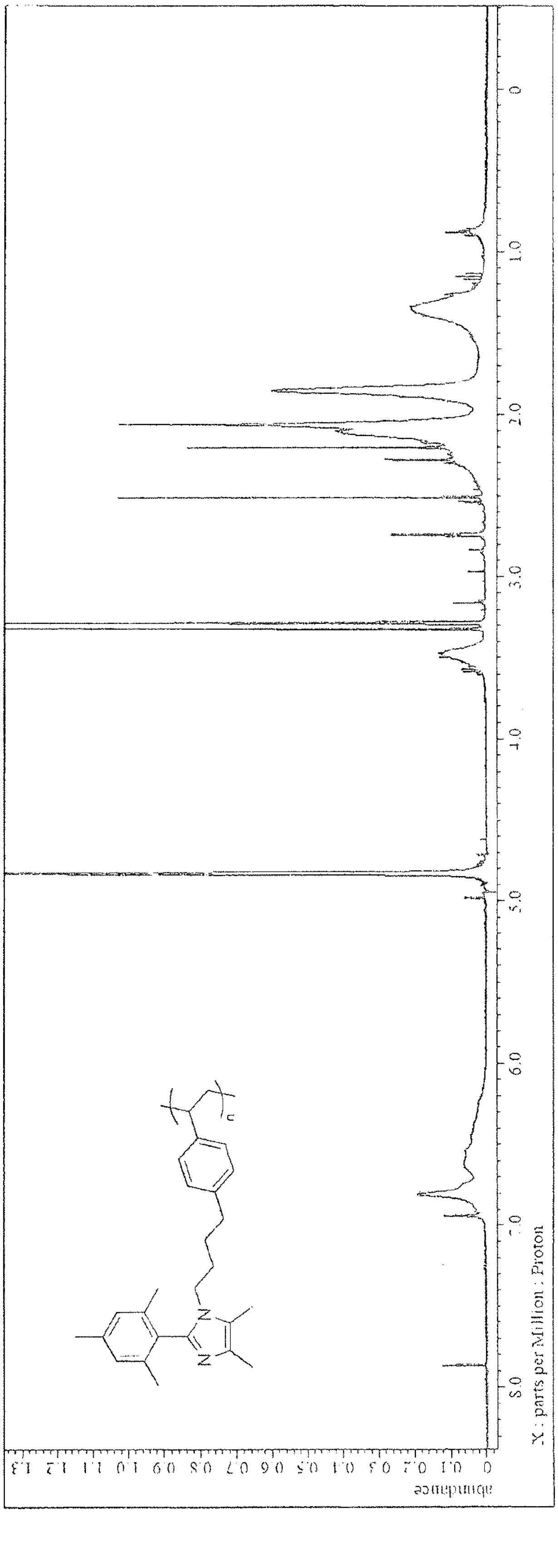

FIG.10

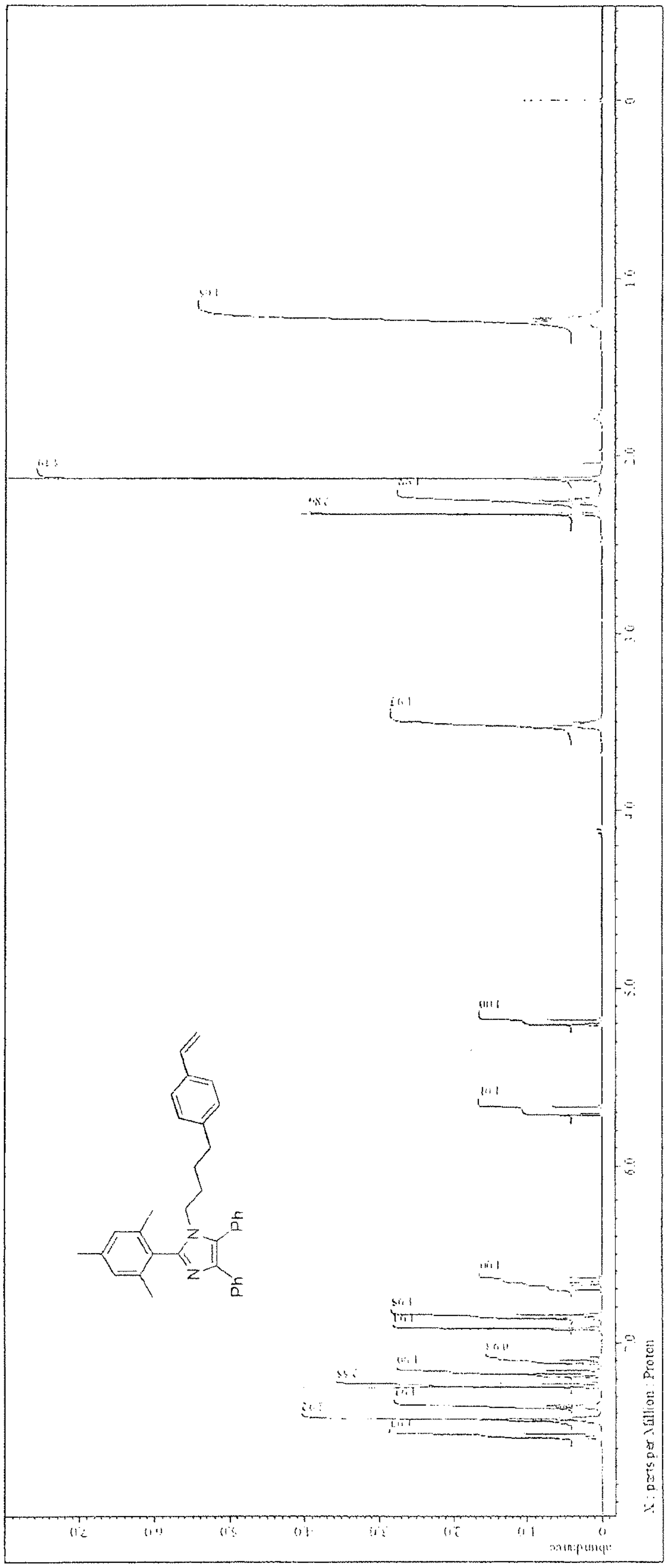

FIG.11

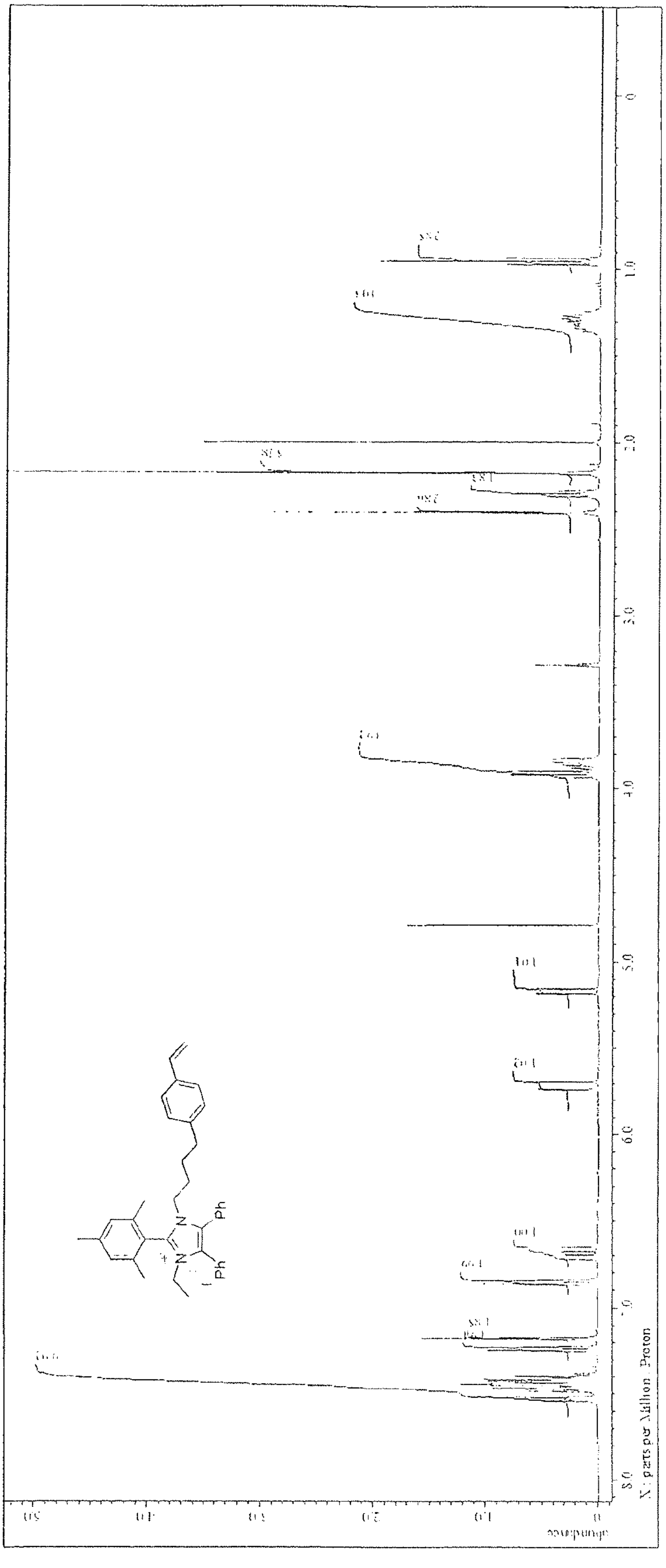

FIG.12

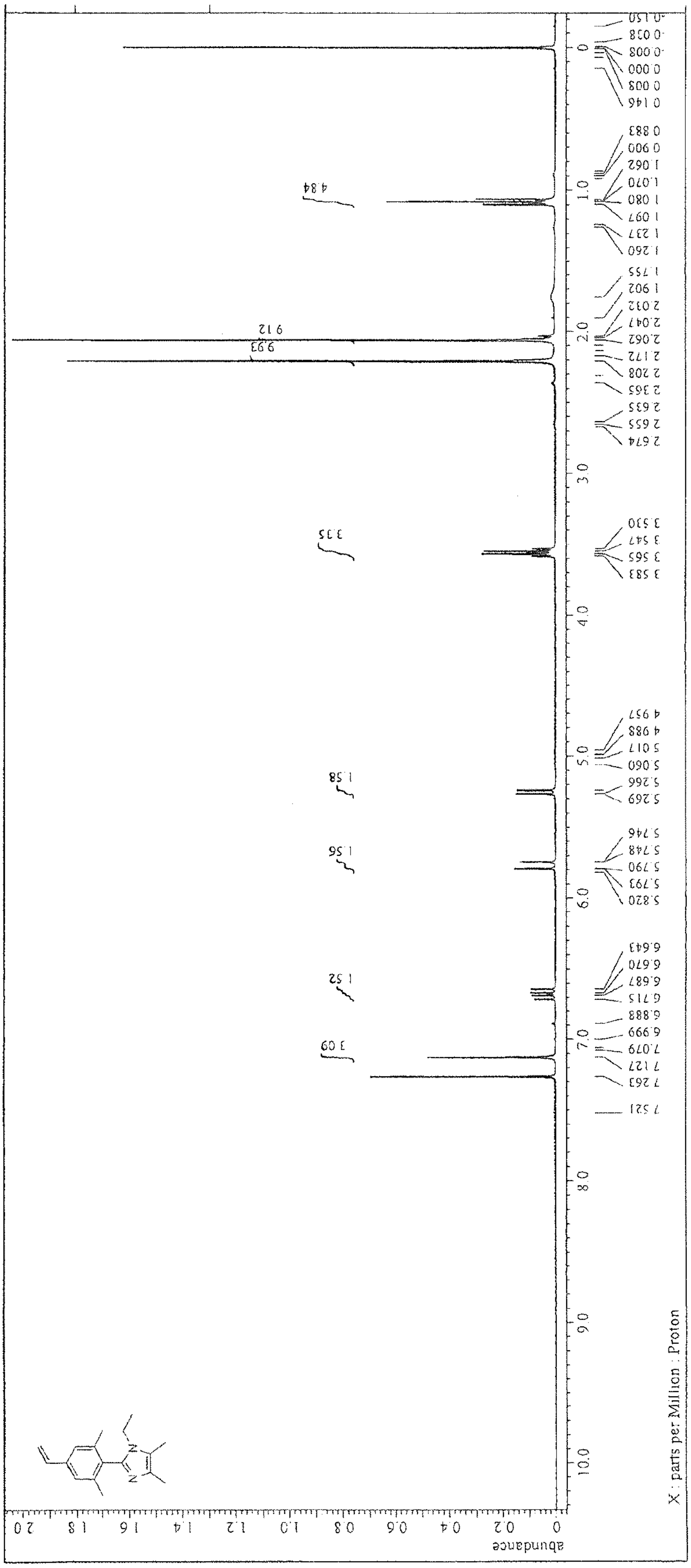

FIG.13

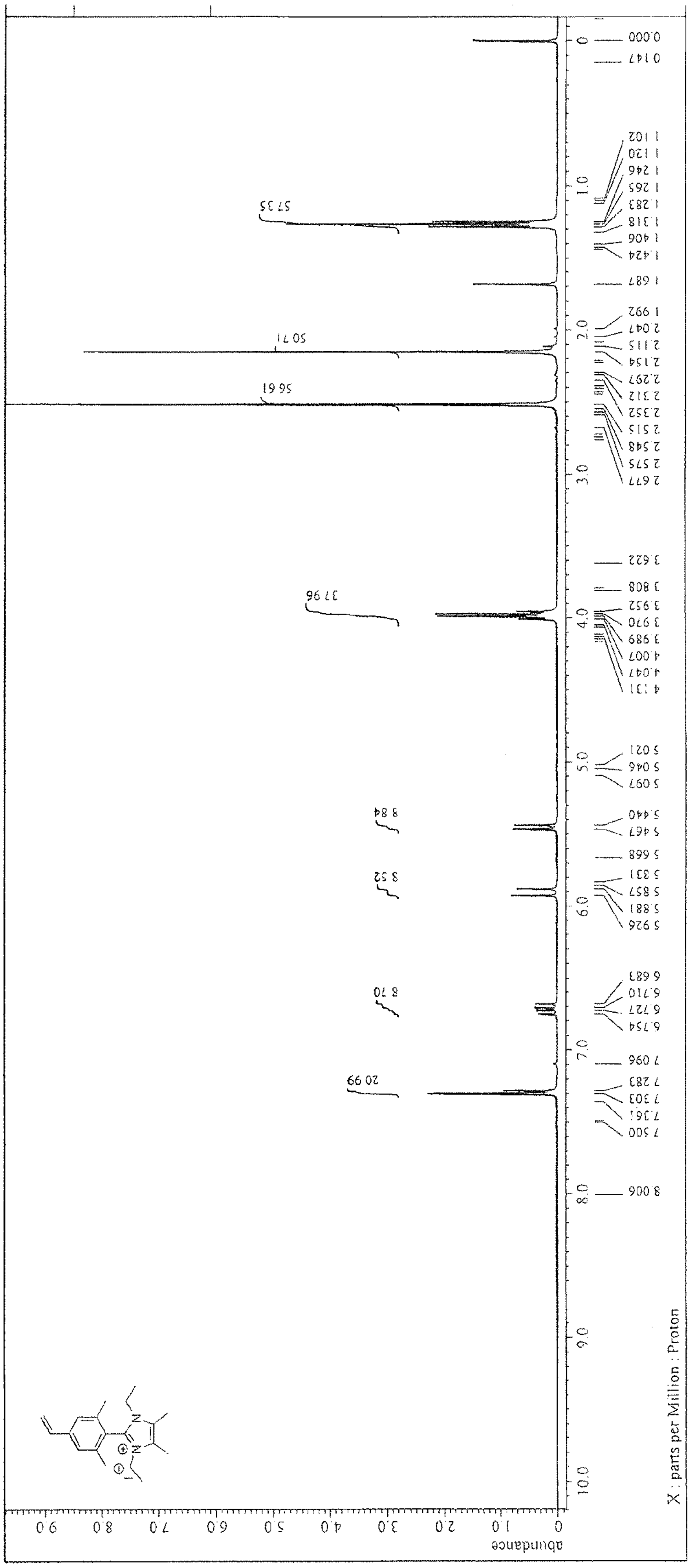

FIG.14

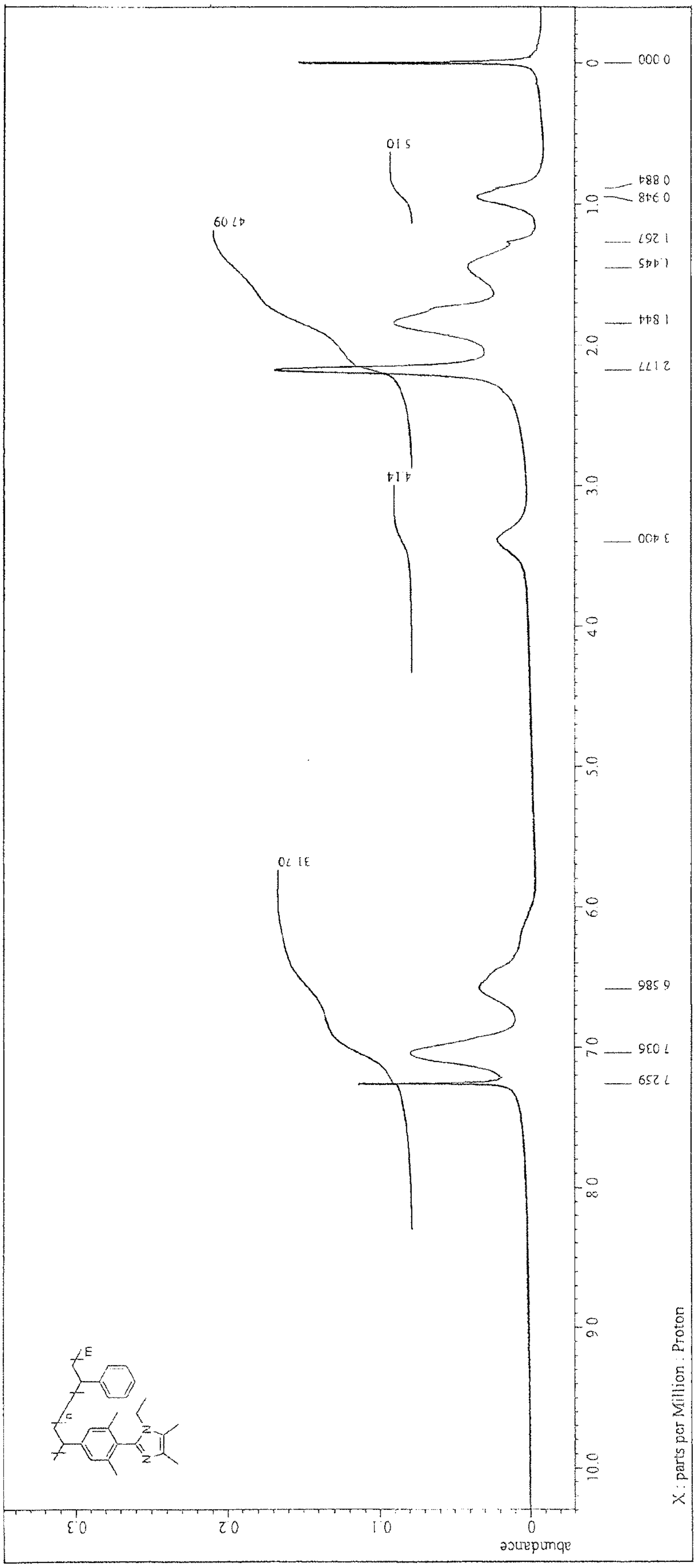
X : parts per Million : Proton
abundance
0.000
0.884
0.948
1.257
1.445
1.844
2.177
3.400
6.586
7.036
7.259
5.10
47.09
4.14
31.70

FIG.15

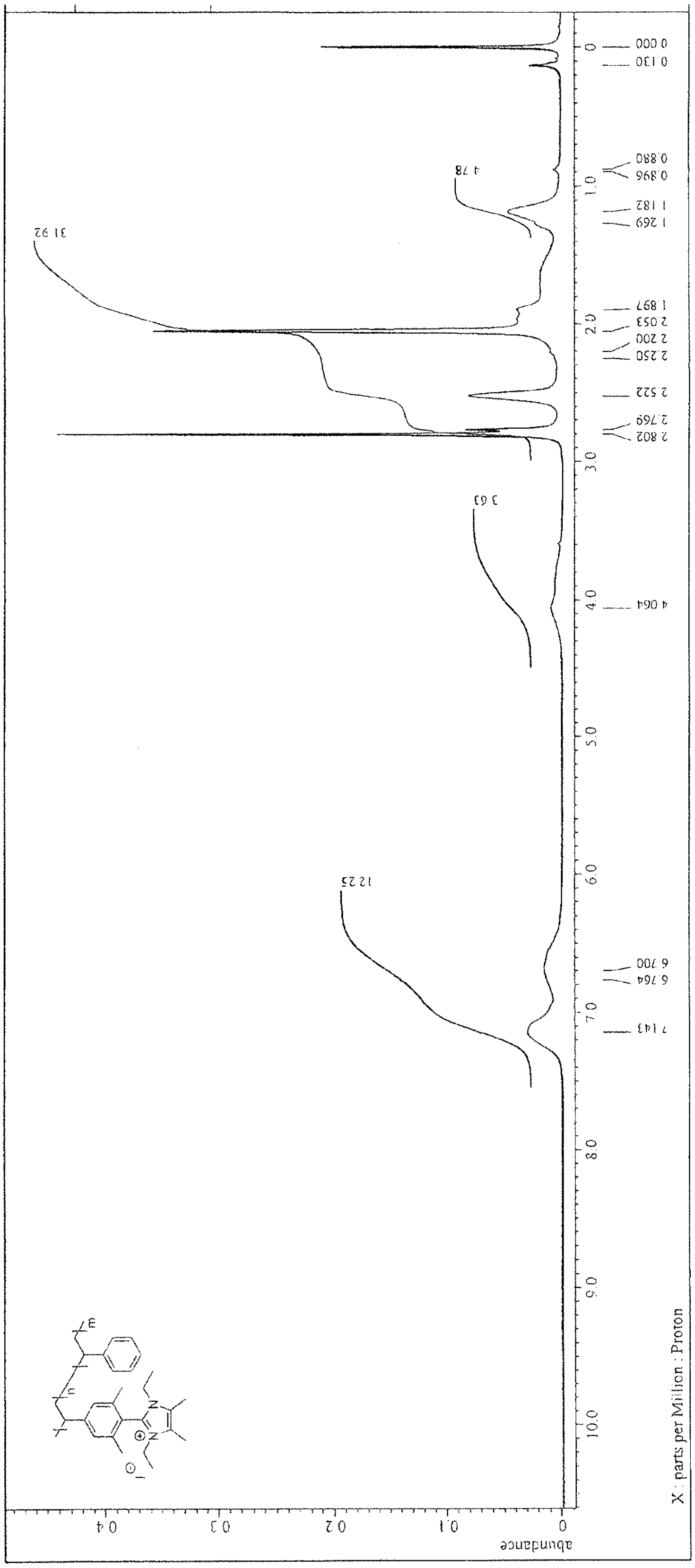
0.000
0.130
0.880
0.896
1.182
1.269
1.897
2.053
2.200
2.250
2.522
2.769
2.802
4.064
6.700
6.764
7.143
4.78
31.92
3.63
12.25
X : parts per Million : Proton
abundance

FIG.16

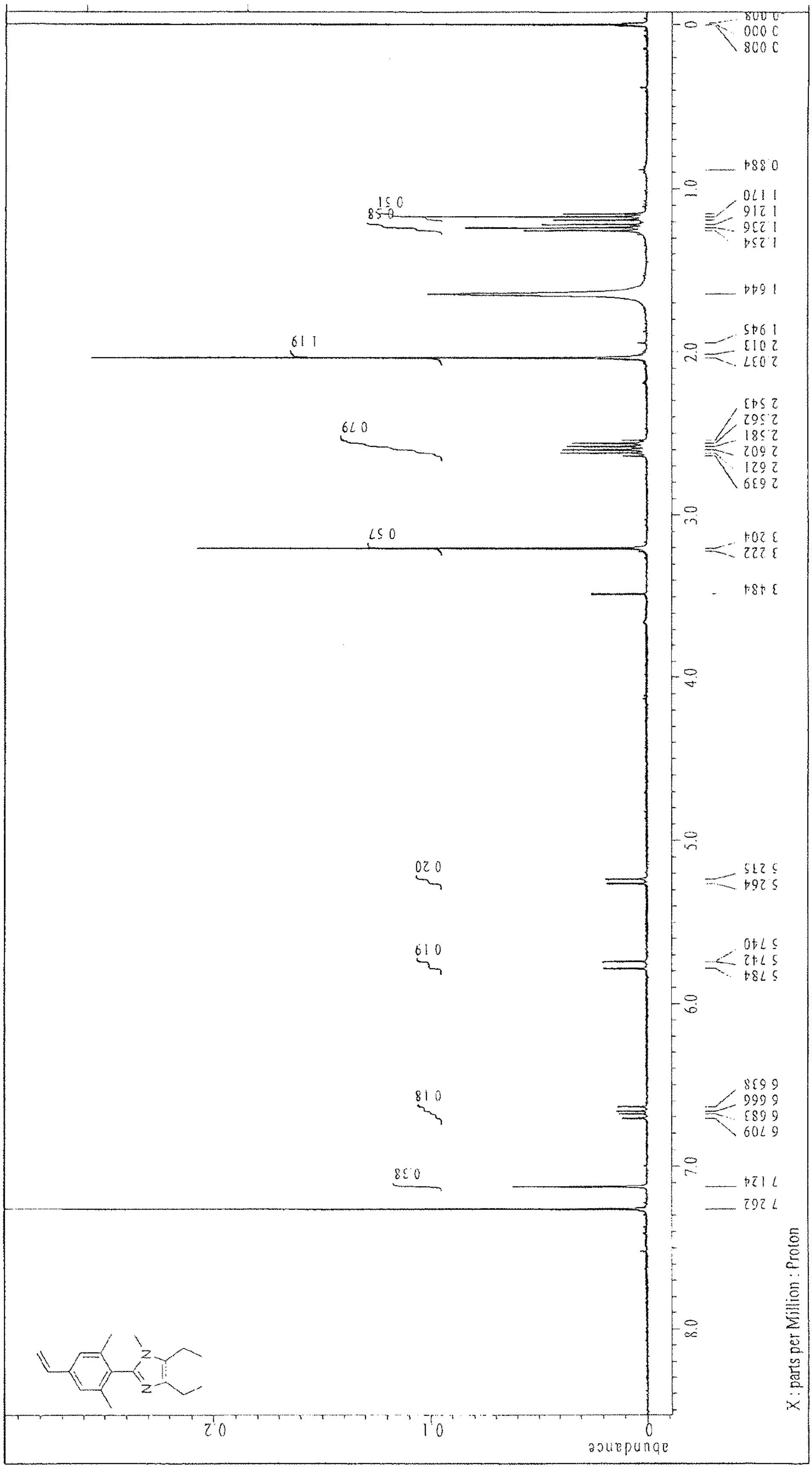

FIG.17

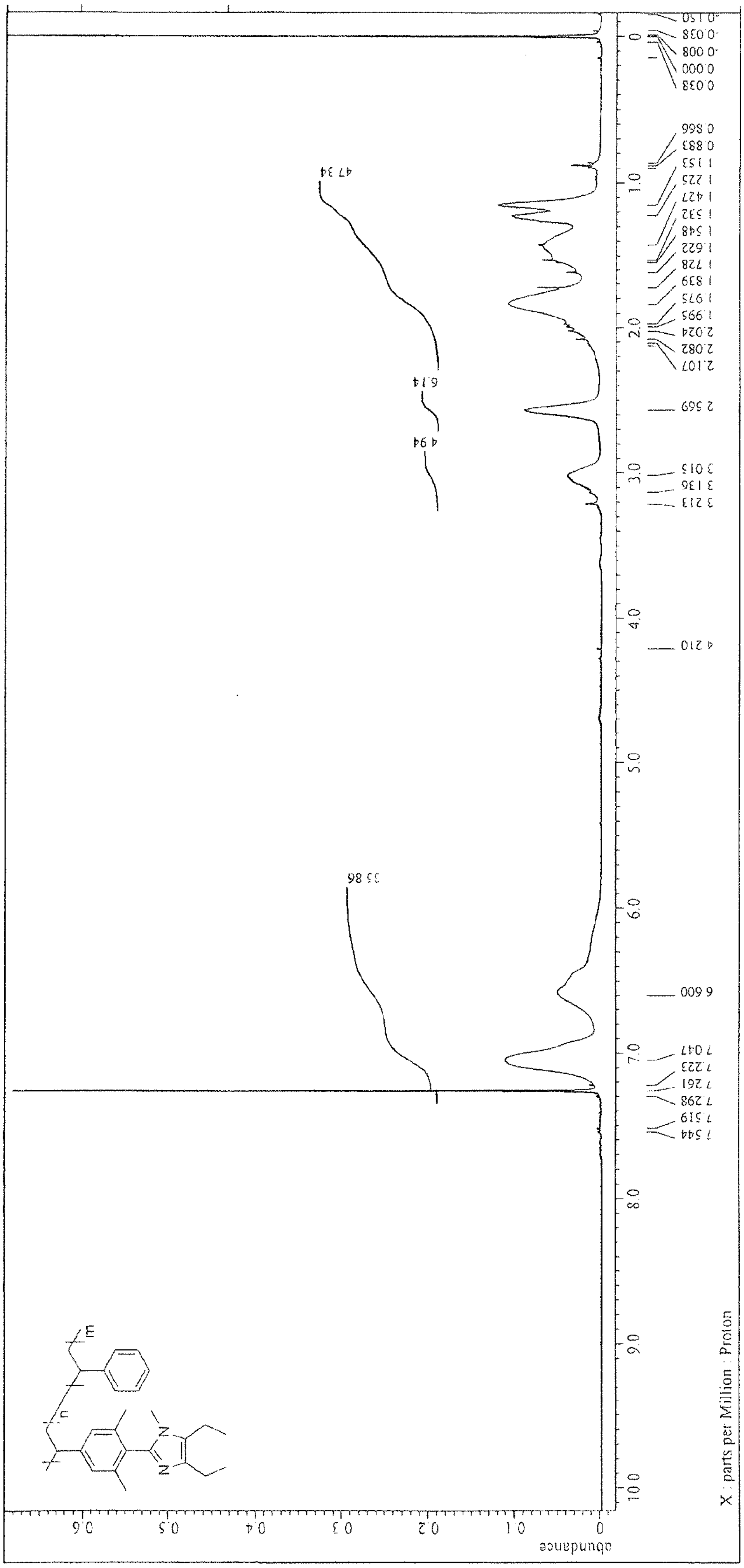

FIG.18

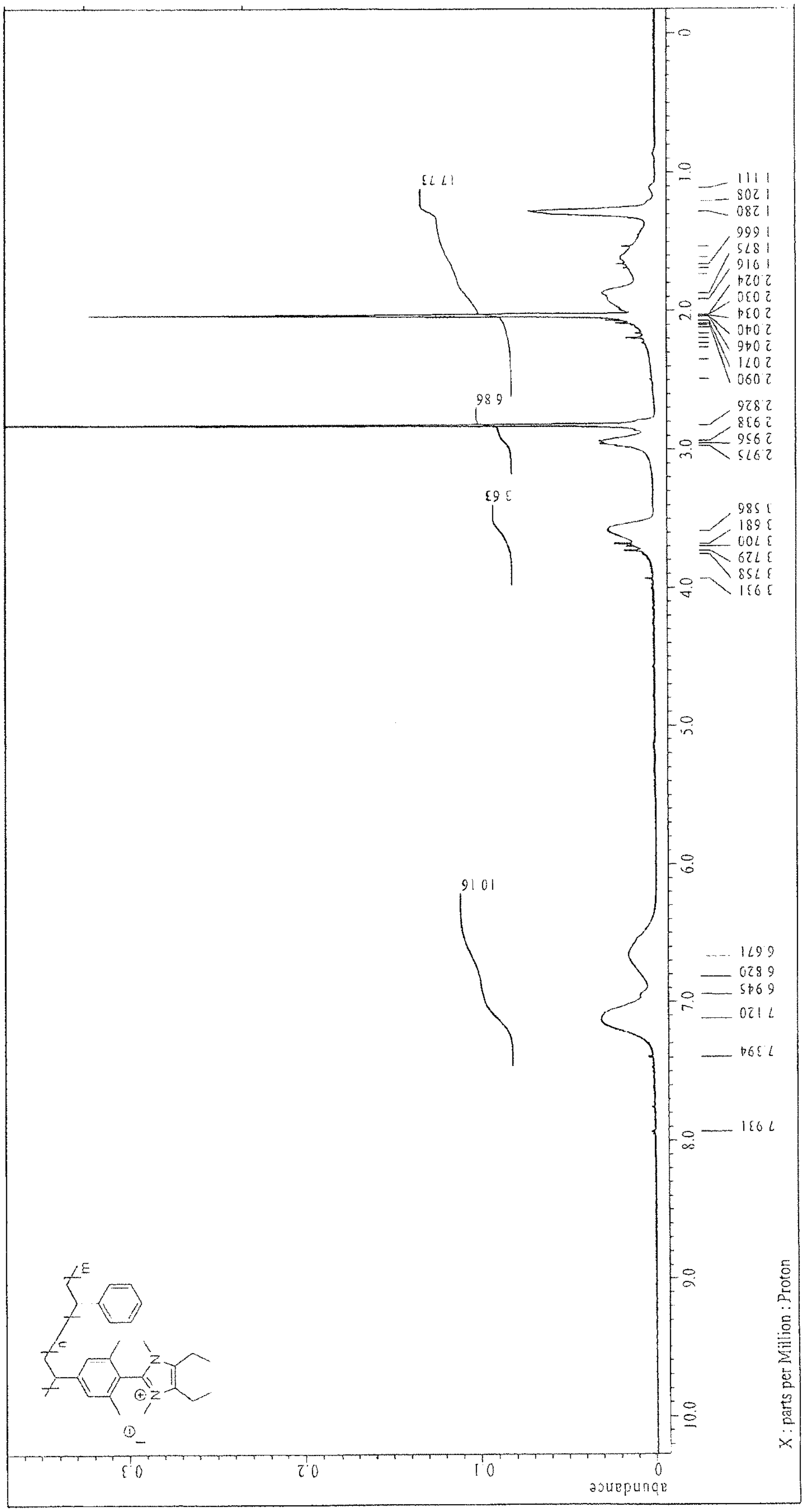

FIG.19

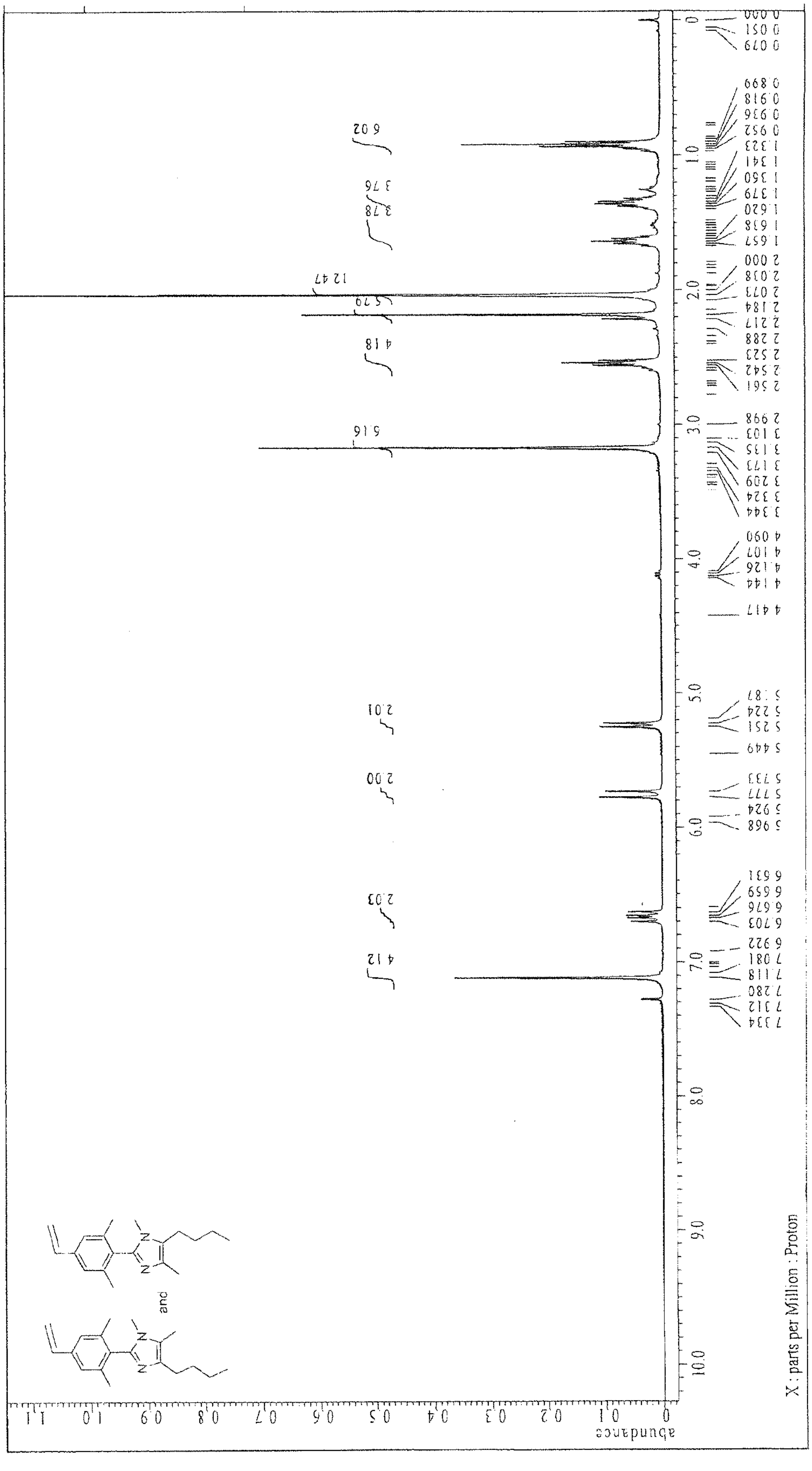

FIG.20

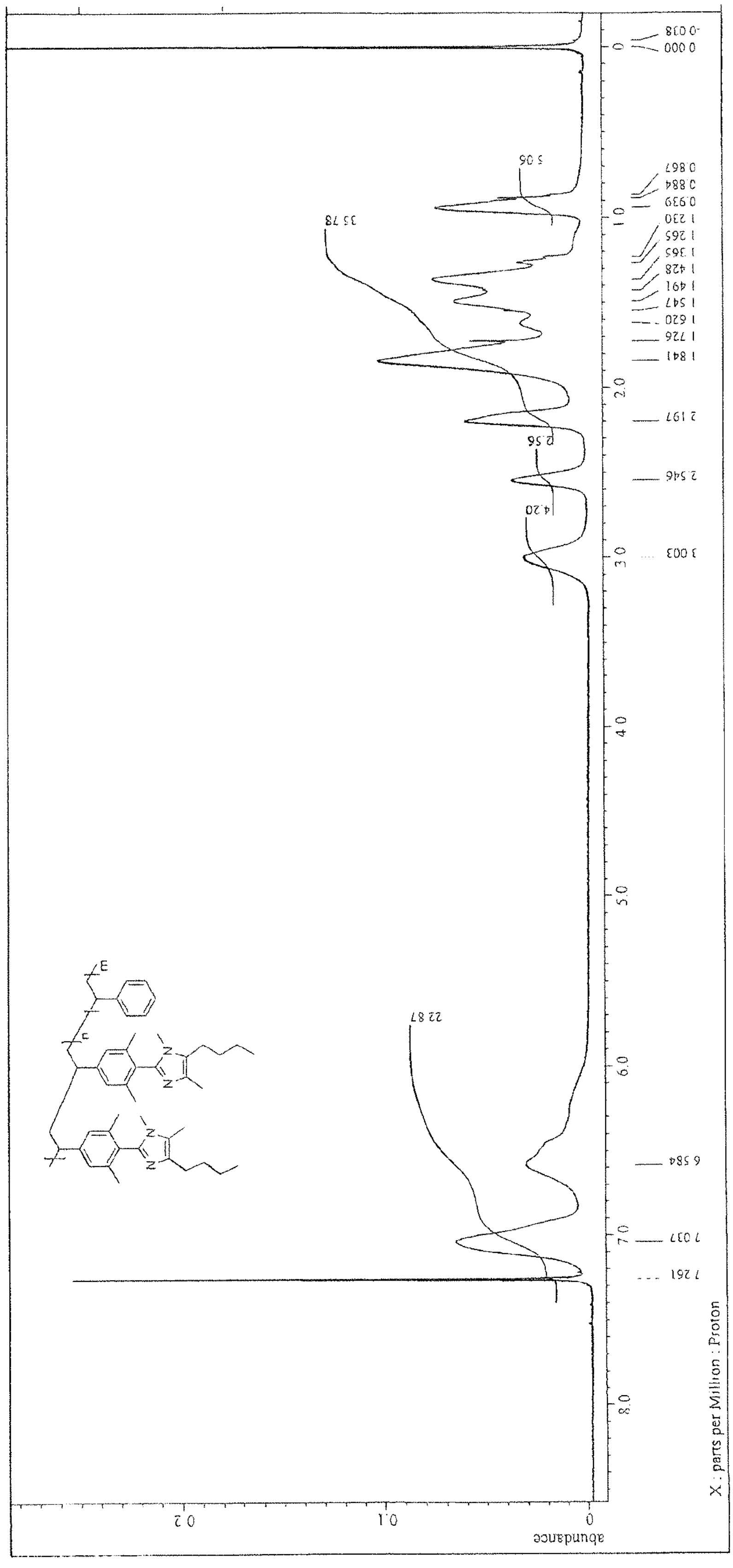

FIG.21

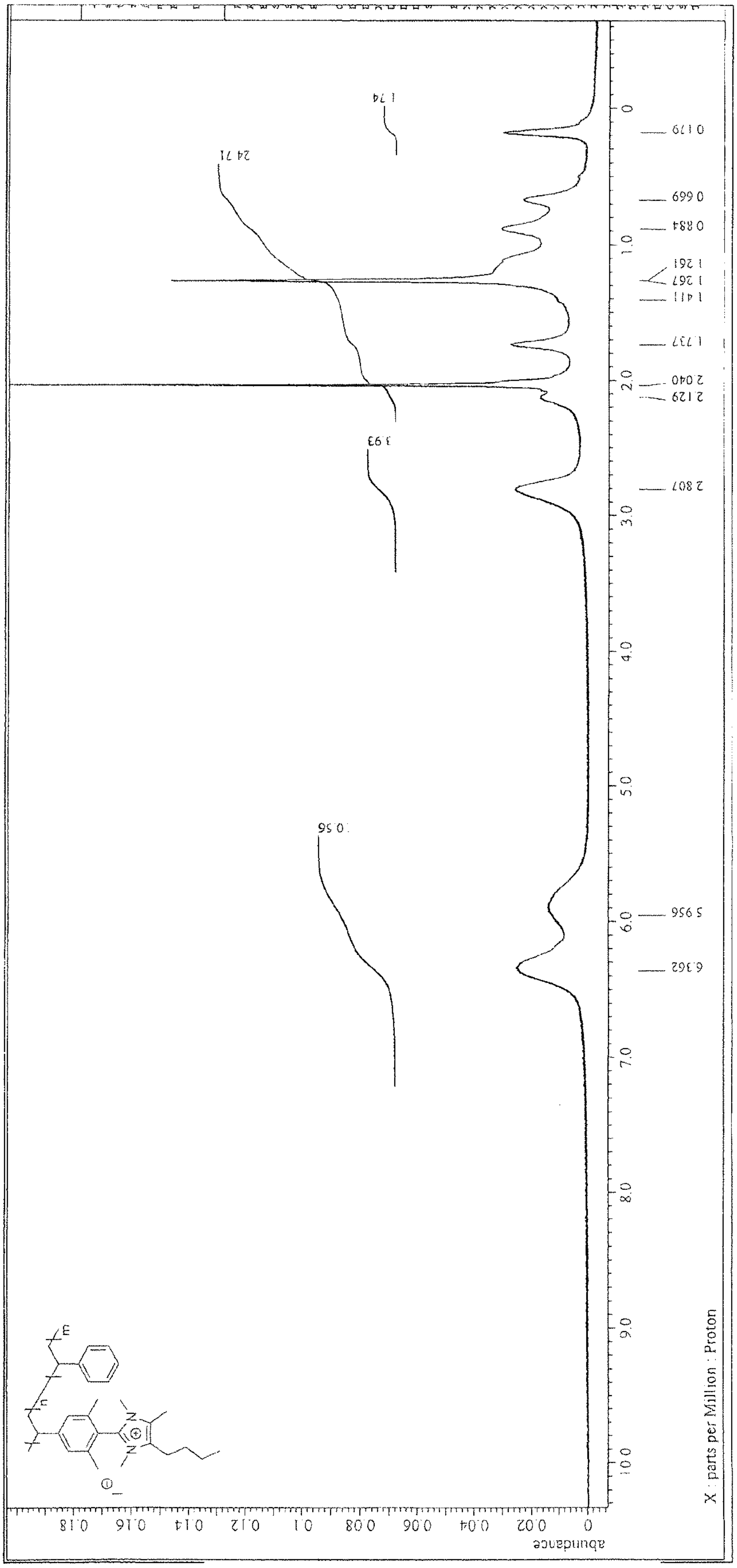

**INTERNATIONAL SEARCH REPORT**

International application No. **PCT/JP2023/012655**

**A. CLASSIFICATION OF SUBJECT MATTER**

***C08F 12/26***(2006.01)i; ***C08L 25/18***(2006.01)i; ***C08J 9/40***(2006.01)i; ***C08J 5/22***(2006.01)i; ***B01J 41/05***(2017.01)i; ***B01J 41/14***(2006.01)i; ***B01J 47/12***(2017.01)i; ***C08K 3/01***(2018.01)i
FI: C08F12/26; C08J5/22 104; C08L25/18; C08K3/01; B01J41/05; B01J41/14; B01J47/12; C08J9/40 CET

According to International Patent Classification (IPC) or to both national classification and IPC

**B. FIELDS SEARCHED**

Minimum documentation searched (classification system followed by classification symbols)

C08F12/26; C08L25/18; C08J9/40; C08J5/22; B01J41/05; B01J41/14; B01J47/12; C08K3/01

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2019-525995 A (DIOXIDE MATERIALS, INC.) 12 September 2019 (2019-09-12) claims, paragraphs [0012], [0017] | 1-2, 5-8, 10-13, 15-17 |
| Y | | 3-4, 9, 14 |
| Y | WO 2011/125717 A1 (TOKUYAMA CORP.) 13 October 2011 (2011-10-13) claims, examples | 3, 9, 14 |
| Y | WO 2017/022775 A1 (TOKUYAMA CORP.) 09 February 2017 (2017-02-09) claims, examples | 4 |
| A | JP 2020-518964 A (DIOXIDE MATERIALS, INC.) 25 June 2020 (2020-06-25) claims, examples | 1-17 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2023** | **23 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/012655**

| Patent document cited in search report | Publication date (day/month/year) | Patent family member(s) | Publication date (day/month/year) |
|---|---|---|---|
| JP 2019-525995 A | 12 September 2019 | US 2019/0226098 A1<br>claims, paragraphs [0012], [0017]<br>WO 2018/022530 A1 | |
| WO 2011/125717 A1 | 13 October 2011 | (Family: none) | |
| WO 2017/022775 A1 | 09 February 2017 | TW 201716232 A | |
| JP 2020-518964 A | 25 June 2020 | US 2018/0316063 A1<br>claims, examples<br>WO 2018/204242 A1<br>CN 110582869 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017022775 A
- WO 2009081931 A
- WO 2011125717 A


### Non-patent literature cited in the description


- *J. American Chemical Society*, 2012, vol. 134 (26), 10753-10756
- *Energy & Environmental Science*, 2016, vol. 9, 2130-2142
- *ACS Macro Letters*, 2017, vol. 6 (10), 1089-1093